# EUROPEAN PATENT APPLICATION

(11) **EP 2 316 951 A1**
(43) Date of publication of application: **04.05.2011**
(21) Application number: 10182383.9
(22) Date of filing: 17.01.2002
(51) Int. Cl.: C12N 15/62, A61K 39/395, C07H 21/04, C07K 16/44, C12N 15/13, C12N 5/10, C12N 15/00

(54) **Binding domain-immunoglobulin fusion proteins**

(30) Priority: 17.01.2001 US 765208
(62) Divisional of application: 02707519.1
(71) Applicant: Trubion Pharmaceuticals, Inc., Seattle, WA 98121 (US)
(72) Inventor: Ledbetter, Jeffrey A., Shoreline, WA 98177 (US); Hayden-Ledbetter, Martha, Shoreline, WA 98177 (US)
(74) Representative: Walker, Ross Thomson

(57) **Abstract**

The invention relates to novel binding domain-immunoglobulin fusion proteins that feature a binding domain for a cognate structure such as an antigen, a counterreceptor or the like, a hinge region polypeptide having either zero or one cysteine residue, and immunoglobulin CH2 and CH3 domains, and that are capable of ADCC and/or CDC while occurring predominantly as monomeric polypeptides. The fusion proteins can be recombinantly produced at high expression levels. Also provided are related compositions and methods, including immunotherapeutic applications.

SEQUENCE LISTING

<110> Genecraft, Inc.
      Ledbetter, Jeffrey
      Hayden-Ledbetter, Martha

<120> Binding Domain-Immunoglobulin Fusion Proteins

<130> 390069.401PC

<140> PCT
<141> 2002-01-17

<160> 38

<170> PatentIn version 3.0

<210> 1
<211> 812
<212> DNA
<213> Artificial Sequence

<220>
<223> SYNTHETIC MOUSE SCFV FUSION GENE

<220>
<221> sig_peptide
<222> (13)..(78)

<220>
<221> V_region
<222> (79)..(396)
<223> light chain variable region for anti-CD20 scFv

<220>
<221> misc_feature
<222> (397)..(444)
<223> asp-gly3ser(gly4ser)2-ser peptide linker

<220>
<221> V_region
<222> (445)..(808)
<223> heavy chain variable region for anti-CD20 scFv

<400> 1
aagcttgccg ccatggattt tcaagtgcag attttcagct tcctgctaat cagtgcttca 60
gtcataattg ccagaggaca aattgttctc tcccagtctc cagcaatcct gtctgcatct 120
ccaggggaga aggtcacaat gacttgcagg gccagctcaa gtgtaagtta catgcactgg 180
taccagcaga agccaggatc ctcccccaaa ccctggattt atgccccatc caacctggct 240
tctggagtcc ctgctcgctt cagtggcagt gggtctggga cctcttactc tctcacaatc 300
agcagagtgg aggctgaaga tgctgccact tattactgcc agcagtggag ttttaaccca 360
cccacgttcg gtgctgggac caagctggag ctgaaaggtg gcggtggctc gggcggtggt 420
ggatctggag gaggtgggag ctctcaggct tatctacagc agtctggggc tgagctggtg 480
aggcctgggg cctcagtgaa gatgtcctgc aaggcttctg gctacacatt taccagttac 540
aatatgcact gggtaaagca gacacctaga cagggcctgg aatggattgg agctatttat 600
ccaggaaatg gtgatacttc ctacaatcag aagttcaagg gcaaggccac actgactgta 660
gacaaatcct ccagcacagc ctacatgcag ctcagcagcc tgacatctga agactctgcg 720
gtctatttct gtgcaagagt ggtgtactat agtaactctt actggtactt cgatgtctgg 780
ggcacaggga ccacggtcac cgtctctgat ca 812

<210>  2
<211>  1518
<212>  DNA
<213>  Artificial Sequence

<220>
<223>  SYNTHETIC MOUSE HUMAN CHIMERIC FUSION GENE

<220>
<221>  misc_feature
<222>  (13)..(807)
<223>  MURINE ANTI-HUMAN CD20 scFv

<220>
<221>  C_region
<222>  (808)..(1513)
<223>  HUMAN IgG1 Fc TAIL, WILD TYPE HINGE, CH2 AND CH3

<400>  2
aagcttgccg ccatggattt tcaagtgcag attttcagct tcctgctaat cagtgcttca      60
gtcataattg ccagaggaca aattgttctc tcccagtctc cagcaatcct gtctgcatct     120
ccaggggaga aggtcacaat gacttgcagg gccagctcaa gtgtaagtta catgcactgg     180
taccagcaga agccaggatc ctcccccaaa ccctggattt atgccccatc caacctggct     240
tctggagtcc ctgctcgctt cagtggcagt gggtctggga cctcttactc tctcacaatc     300
agcagagtgg aggctgaaga tgctgccact tattactgcc agcagtggag ttttaaccca     360
cccacgttcg gtgctgggac caagctggag ctgaaagatg gcggtggctc gggcggtggt     420
ggatctggag gaggtgggag ctctcaggct tatctacagc agtctggggc tgagctggtg     480
aggcctgggg cctcagtgaa gatgtcctgc aaggcttctg gctacacatt taccagttac     540
aatatgcact gggtaaagca gacacctaga cagggcctgg aatggattgg agctatttat     600
ccaggaaatg gtgatacttc ctacaatcag aagttcaagg gcaaggccac actgactgta     660
gacaaatcct ccagcacagc ctacatgcag ctcagcagcc tgacatctga agactctgcg     720
gtctatttct gtgcaagagt ggtgtactat agtaactctt actggtactt cgatgtctgg     780
ggcacaggga ccacggtcac cgtctctgat caggagccca aatcttgtga caaaactcac     840
acatgcccac cgtgcccagc acctgaactc ctggggggac cgtcagtctt cctcttcccc     900
ccaaaaccca aggacaccct catgatctcc cggacccctg aggtcacatg cgtggtggtg     960
gacgtgagcc acgaagaccc tgaggtcaag ttcaactggt acgtggacgg cgtggaggtg    1020
cataatgcca agacaaagcc gcgggaggag cagtacaaca gcacgtaccg tgtggtcagc    1080
gtcctcaccg tcctgcacca ggactggctg aatggcaagg agtacaagtg caaggtctcc    1140
aacaaagccc tcccagcccc catcgagaaa acaatctcca aagccaaagg gcagccccga    1200
gaaccacagg tgtacaccct gcccccatcc cgggatgagc tgaccaagaa ccaggtcagc    1260
ctgacctgcc tggtcaaagg cttctatccc agcgacatcg ccgtggagtg ggagagcaat    1320
gggcagccgg agaacaacta caagaccacg cctcccgtgc tggactccga cggctccttc    1380
ttcctctaca gcaagctcac cgtggacaag agcaggtggc agcaggggaa cgtcttctca    1440
tgctccgtga tgcatgaggc tctgcacaac cactacacgc agaagagcct ctccctgtct    1500
ccgggtaaat gatctaga                                                  1518

<210>  3
<211>  1518
<212>  DNA
<213>  Artificial Sequence

<220>
<223>  SYNTHETIC MOUSE-HUMAN CHIMERIC FUSION GENE

<220>
<221>  misc_feature
<222>  (13)..(807)<223>  MOUSE ANTI-HUMAN CD20 SCFV

<220>
<221> C_region
<222> (808)..(1513)
<223> HINGE CYSTEINES (826-829; 844-847; 853-856) MUTATED TO SERINES
      PROLINE TO SERINE MUTATION (880-883) IN CH2 DISRUPTS EFFECTOR FUN
      CTION

<400> 3
aagcttgccg ccatggattt tcaagtgcag attttcagct tcctgctaat cagtgcttca      60
gtcataattg ccagaggaca aattgttctc tcccagtctc cagcaatcct gtctgcatct     120
ccaggggaga aggtcacaat gacttgcagg gccagctcaa gtgtaagtta catgcactgg     180
taccagcaga agccaggatc ctcccccaaa ccctggattt atgccccatc caacctggct     240
tctggagtcc ctgctcgctt cagtggcagt gggtctggga cctcttactc tctcacaatc     300
agcagagtgg aggctgaaga tgctgccact tattactgcc agcagtggag ttttaaccca     360
cccacgttcg gtgctgggac caagctggag ctgaaagatg gcggtggctc gggcggtggt     420
ggatctggag gaggtgggag ctctcaggct tatctacagc agtctggggc tgagctggtg     480
aggcctgggg cctcagtgaa gatgtcctgc aaggcttctg gctacacatt taccagttac     540
aatatgcact gggtaaagca gacacctaga cagggcctgg aatggattgg agctatttat     600
ccaggaaatg gtgatacttc ctacaatcag aagttcaagg gcaaggccac actgactgta     660
gacaaatcct ccagcacagc ctacatgcag ctcagcagcc tgacatctga agactctgcg     720
gtctatttct gtgcaagagt ggtgtactat agtaactctt actggtactt cgatgtctgg     780
ggcacaggga ccacggtcac cgtctctgat caggagccca aatcttctga caaaactcac     840
acatccccac cgtccccagc acctgaactc ctggggggat cgtcagtctt cctcttcccc     900
ccaaaaccca aggacaccct catgatctcc cggacccctg aggtcacatg cgtggtggtg     960
gacgtgagcc acgaagaccc tgaggtcaag ttcaactggt acgtggacgg cgtggaggtg    1020
cataatgcca agacaaagcc gcgggaggag cagtacaaca gcacgtaccg tgtggtcagc    1080
gtcctcaccg tcctgcacca ggactggctg aatggcaagg agtacaagtg caaggtctcc    1140
aacaaagccc tcccagcccc catcgagaaa acaatctcca aagccaaagg gcagccccga    1200
gaaccacagg tgtacaccct gcccccatcc cgggatgagc tgaccaagaa ccaggtcagc    1260
ctgacctgcc tggtcaaagg cttctatccc agcgacatcg ccgtggagtg ggagagcaat    1320
gggcagccgg agaacaacta caagaccacg cctcccgtgc tggactccga cggctccttc    1380
ttcctctaca gcaagctcac cgtggacaag agcaggtggc agcaggggaa cgtcttctca    1440
tgctccgtga tgcatgaggc tctgcacaac cactacacgc agaagagcct ctccctgtct    1500
ccgggtaaat gatctaga                                                  1518

<210> 4
<211> 1518
<212> DNA
<213> Artificial Sequence

<220>
<223> SYNTHETIC MOUSE-HUMAN CHIMERIC FUSION GENE

<220>
<221> misc_feature
<222> (13)..(807)
<223> MOUSE ANTI-HUMAN CD20 SCFV

<220>
<221> C_region
<222> (808)..(1513)
<223> HINGE CYSTEINES MUTATED TO SERINES (826-829; 844-847; 853-856)
      WILD TYPE CH2 AND CH3 DOMAINS MEDIATE EFFECTOR FUNCTIONS

<400> 4
aagcttgccg ccatggattt tcaagtgcag attttcagct tcctgctaat cagtgcttca      60
gtcataattg ccagaggaca aattgttctc tcccagtctc cagcaatcct gtctgcatct     120
ccaggggaga aggtcacaat gacttgcagg gccagctcaa gtgtaagtta catgcactgg     180
taccagcaga agccaggatc ctcccccaaa ccctggattt atgccccatc caacctggct     240
tctggagtcc ctgctcgctt cagtggcagt gggtctggga cctcttactc tctcacaatc     300
agcagagtgg aggctgaaga tgctgccact tattactgcc agcagtggag ttttaaccca     360

cccacgttcg gtgctgggac caagctggag ctgaaagatg gcggtggctc gggcggtggt 420
ggatctggag gaggtgggag ctctcaggct tatctacagc agtctggggc tgagctggtg 480
aggcctgggg cctcagtgaa gatgtcctgc aaggcttctg gctacacatt taccagttac 540
aatatgcact gggtaaagca gacacctaga cagggcctgg aatggattgg agctatttat 600
ccaggaaatg gtgatacttc ctacaatcag aagttcaagg gcaaggccac actgactgta 660
gacaaatcct ccagcacagc ctacatgcag ctcagcagcc tgacatctga agactctgcg 720
gtctatttct gtgcaagagt ggtgtactat agtaactctt actggtactt cgatgtctgg 780
ggcacaggga ccacggtcac cgtctctgat caggagccca aatcttctga caaaactcac 840
acatccccac cgtccccagc acctgaactc ctggggggac cgtcagtctt cctcttcccc 900
ccaaaaccca aggacaccct catgatctcc cggacccctg aggtcacatg cgtggtggtg 960
gacgtgagcc acgaagaccc tgaggtcaag ttcaactggt acgtggacgg cgtggaggtg 1020
cataatgcca agacaaagcc gcgggaggag cagtacaaca gcacgtaccg tgtggtcagc 1080
gtcctcaccg tcctgcacca ggactggctg aatggcaagg agtacaagtg caaggtctcc 1140
aacaaagccc tcccagcccc catcgagaaa acaatctcca aagccaaagg gcagccccga 1200
gaaccacagg tgtacaccct gcccccatcc cgggatgagc tgaccaagaa ccaggtcagc 1260
ctgacctgcc tggtcaaagg cttctatccc agcgacatcg ccgtggagtg ggagagcaat 1320
gggcagccgg agaacaacta caagaccacg cctcccgtgc tggactccga cggctccttc 1380
ttcctctaca gcaagctcac cgtggacaag agcaggtggc agcaggggaa cgtcttctca 1440
tgctccgtga tgcatgaggc tctgcacaac cactacacgc agaagagcct ctccctgtct 1500
ccgggtaaat gatctaga 1518

<210> 5
<211> 1524
<212> DNA
<213> Artificial Sequence

<220>
<223> SYNTHETIC MOUSE HUMAN CHIMERIC FUSION GENE

<220>
<221> misc_feature
<222> (1)..(796)
<223> MOUSE ANTI HUMAN CD20 SCFV

<220>
<221> N_region
<222> (797)..(864)
<223> HUMAN IGA HINGE REGION

<220>
<221> C_region
<222> (865)..(1518)
<223> HUMAN IGG1 CH2 AND CH3 WILD TYPE FC DOMAIN

<400> 5
atggattttc aagtgcagat tttcagcttc ctgctaatca gtgcttcagt cataattgcc 60
agaggacaaa ttgttctctc ccagtctcca gcaatcctgt ctgcatctcc aggggagaag 120
gtcacaatga cttgcagggc cagctcaagt gtaagttaca tgcactggta ccagcagaag 180
ccaggatcct cccccaaacc ctggatttat gccccatcca acctggcttc tggagtccct 240
gctcgcttca gtggcagtgg gtctgggacc tcttactctc tcacaatcag cagagtggag 300
gctgaagatg ctgccactta ttactgccag cagtggagtt ttaacccacc cacgttcggt 360
gctgggacca agctggagct gaaagatggc ggtggctcgg gcggtggtgg atctggagga 420
ggtgggagct ctcaggctta tctacagcag tctggggctg agctggtgag gcctggggcc 480
tcagtgaaga tgtcctgcaa ggcttctggc tacacattta ccagttacaa tatgcactgg 540
gtaaagcaga cacctagaca gggcctggaa tggattggag ctatttatcc aggaaatggt 600
gatacttcct acaatcagaa gttcaagggc aaggccacac tgactgtaga caaatcctcc 660
agcacagcct acatgcagct cagcagcctg acatctgaag actctgcggt ctatttctgt 720
gcaagagtgg tgtactatag taactcttac tggtacttcg atgtctgggg cacagggacc 780
acggtcaccg tctctgatca gccagttccc tcaactccac ctaccccatc tccctcaact 840
ccacctaccc catctccctc atgcgcacct gaactcctgg ggggaccgtc agtcttcctc 900
ttccccccaa aacccaagga caccctcatg atctcccgga cccctgaggt cacatgcgtg 960

gtggtggacg tgagccacga agaccctgag gtcaagttca actggtacgt ggacggcgtg 1020
gaggtgcata atgccaagac aaagccgcgg gaggagcagt acaacagcac gtaccgtgtg 1080
gtcagcgtcc tcaccgtcct gcaccaggac tggctgaatg gcaaggagta caagtgcaag 1140
gtctccaaca aagccctccc agcccccatc gagaaaacaa tctccaaagc caaagggcag 1200
ccccgagaac cacaggtgta caccctgccc ccatcccggg atgagctgac caagaaccag 1260
gtcagcctga cctgcctggt caaaggcttc tatcccagcg acatcgccgt ggagtgggag 1320
agcaatgggc agccggagaa caactacaag accacgcctc ccgtgctgga ctccgacggc 1380
tccttcttcc tctacagcaa gctcaccgtg gacaagagca ggtggcagca ggggaacgtc 1440
ttctcatgct ccgtgatgca tgaggctctg cacaaccact acacgcagaa gagcctctcc 1500
ctgtctccgg gtaaatgatc taga 1524

<210> 6
<211> 711
<212> DNA
<213> Artificial Sequence

<220>
<223> SYNTHETIC HUMAN PARTIAL FUSION GENE

<220>
<221> misc_feature
<222> (1)..(705)
<223> HINGE CYSTEINES MUTATED TO SERINES (19-21; 37-39; 46-48)

<400> 6
gatcaggagc ccaaatcttc tgacaaaact cacacatccc caccgtcccc agcacctgaa 60
ctcctggggg gaccgtcagt cttcctcttc cccccaaaac ccaaggacac cctcatgatc 120
tcccggaccc ctgaggtcac atgcgtggtg gtggacgtga gccacgaaga ccctgaggtc 180
aagttcaact ggtacgtgga cggcgtggag gtgcataatg ccaagacaaa gccgcgggag 240
gagcagtaca acagcacgta ccgtgtggtc agcgtcctca ccgtcctgca ccaggactgg 300
ctgaatggca aggagtacaa gtgcaaggtc tccaacaaag ccctcccagc ccccatcgag 360
aaaacaatct ccaaagccaa agggcagccc cgagaaccac aggtgtacac cctgccccca 420
tcccgggatg agctgaccaa gaaccaggtc agcctgacct gcctggtcaa aggcttctat 480
cccagcgaca tcgccgtgga gtgggagagc aatgggcagc cggagaacaa ctacaagacc 540
acgcctcccg tgctggactc cgacggctcc ttcttcctct acagcaagct caccgtggac 600
aagagcaggt ggcagcaggg gaacgtcttc tcatgctccg tgatgcatga ggctctgcac 660
aaccactaca cgcagaagag cctctccctg tctccgggta aatgatctag a 711


<210> 7
<211> 729
<212> DNA
<213> Artificial Sequence

<220>
<223> SYNTHETIC HUMAN PARTIAL FUSION GENE

<220>
<221> N_region
<222> (1)..(69)
<223> HUMAN IGA HINGE

<220>
<221> C_region
<222> (70)..(723)
<223> HUMAN WILD TYPE IGG1 CH2 AND CH3, FC

<400> 7
gatcagccag ttccctcaac tccacctacc ccatctccct caactccacc taccccatct 60
ccctcatgcg cacctgaact cctgggggga ccgtcagtct tcctcttccc cccaaaaccc 120
aaggacaccc tcatgatctc ccggacccct gaggtcacat gcgtggtggt ggacgtgagc 180
cacgaagacc ctgaggtcaa gttcaactgg tacgtggacg gcgtggaggt gcataatgcc 240

aagacaaagc cgcgggagga gcagtacaac agcacgtacc gtgtggtcag cgtcctcacc 300
gtcctgcacc aggactggct gaatggcaag gagtacaagt gcaaggtctc caacaaagcc 360
ctcccagccc ccatcgagaa aacaatctcc aaagccaaag ggcagccccg agaaccacag 420
gtgtacaccc tgcccccatc ccgggatgag ctgaccaaga accaggtcag cctgacctgc 480
ctggtcaaag gcttctatcc cagcgacatc gccgtggagt gggagagcaa tgggcagccg 540
gagaacaact acaagaccac gcctcccgtg ctggactccg acggctcctt cttcctctac 600
agcaagctca ccgtggacaa gagcaggtgg cagcagggga acgtcttctc atgctccgtg 660
atgcatgagg ctctgcacaa ccactacacg cagaagagcc tctccctgtc tccgggtaaa 720
tgatctaga 729

<210> 8
<211> 825
<212> DNA
<213> Artificial Sequence

<220>
<223> SYNTHETIC MOUSE SCFV FUSION GENE

<220>
<221> misc_feature
<222> (13)..(72)
<223> LIGHT CHAIN LEADER PEPTIDE

<220>
<221> V_region
<222> (73)..(405)
<223> LIGHT CHAIN VARIABLE DOMAIN FOR MOUSE ANTI-HUMAN CD19: HD37

<220>
<221> misc_feature
<222> (406)..(450)
<223> SYNTHETIC (GLY4SER)3 LINKER PEPTIDE

<220>
<221> V_region
<222> (454)..(825)
<223> HEAVY CHAIN VARIABLE DOMAIN FOR MOUSE ANTI-HUMAN CD19: HD37

<400> 8
aagcttgccg ccatggagac agacacactc ctgctatggg tgctgctgct ctgggttcca 60
ggctccactg gtgacattgt gctgacccaa tctccagctt ctttggctgt gtctctaggg 120
cagagggcca ccatctcctg caaggccagc caaagtgttg attatgatgg tgatagttat 180
ttgaactggt accaacagat tccaggacag ccacccaaac tcctcatcta tgatgcatcc 240
aatctagttt ctgggatccc acccaggttt agtggcagtg ggtctgggac agacttcacc 300
ctcaacatcc atcctgtgga gaaggtggat gctgcaacct atcactgtca gcaaagtact 360
gaggatccgt ggacgttcgg tggaggcacc aagctggaaa tcaaaggtgg cggtggctcg 420
ggcggtggtg ggtcgggtgg cggcggatcg tcacaggttc agctgcagca gtctggggct 480
gagctggtga ggcctgggtc ctcagtgaag atttcctgca aggcttctgg ctatgcattc 540
agtagctact ggatgaactg ggtgaagcag aggcctggac agggtcttga gtggattgga 600
cagatttggc ctggagatgg tgatactaac tacaatggaa agttcaaggg taaagccact 660
ctgactgcag acgaatcctc cagcacagcc tacatgcaac tcagcagcct agcatctgag 720
gactctgcgg tctatttctg tgcaagacgg gagactacga cggtaggccg ttattactat 780
gctatggact actggggtca aggaacctca gtcaccgtct cctca 825

<210> 9
<211> 795
<212> DNA
<213> Artificial Sequence

<220>

<223>  SYNTHETIC MOUSE SCFV FUSION GENE

<220>
<221>  misc_feature
<222>  (13)..(72)
<223>  LIGHT CHAIN LEADER PEPTIDE SEQUENCE

<220>
<221>  V_region
<222>  (73)..(393)
<223>  LIGHT CHAIN VARIABLE DOMAIN FOR MOUSE ANTI-HUMAN CD37:  G28-1

<220>
<221>  misc_feature
<222>  (394)..(441)
<223>  SYNTHETIC LINKER PEPTIDE ENCODED (GLY4SER)3

<220>
<221>  V_region
<222>  (442)..(795)
<223>  HEAVY CHAIN VARIABLE DOMAIN FOR MOUSE ANTI-HUMAN CD37:  G28-1

<400>  9
aagcttgccg ccatggtatc cacagctcag ttccttgggt tgctgctgct gtggcttaca      60
ggtggcagat gtgacatcca gatgactcag tctccagcct ccctatctgc atctgtggga     120
gagactgtca ccatcacatg tcgaacaagt gaaaatgttt acagttattt ggcttggtat     180
cagcagaaac agggaaaatc tcctcagctc ctggtctctt ttgcaaaaac cttagcagaa     240
ggtgtgccat caaggttcag tggcagtgga tcaggcacac agttttctct gaagatcagc     300
agcctgcagc ctgaagattc tggaagttat ttctgtcaac atcattccga taatccgtgg     360
acgttcggtg gaggcaccga actggagatc aaaggtggcg gtggctcggg cggtggtggg     420
tcgggtggcg gcggatcgtc agcggtccag ctgcagcagt ctggacctga gctggaaaag     480
cctggcgctt cagtgaagat ttcctgcaag gcttctggtt actcattcac tggctacaat     540
atgaactggg tgaagcagaa taatggaaag agccttgagt ggattggaaa tattgatcct     600
tattatggtg gtactaccta caaccggaag ttcaagggca aggccacatt gactgtagac     660
aaatcctcca gcacagccta catgcagctc aagagtctga catctgagga ctctgcagtc     720
tattactgtg caagatcggt cggccctatg gactactggg gtcaaggaac ctcagtcacc     780
gtctcttctg atcag                                                      795


<210>  10
<211>  824
<212>  DNA
<213>  Artificial Sequence

<220>
<223>  SYNTHETIC MOUSE FUSION GENE

<220>
<221>  sig_peptide
<222>  (1)..(61)
<223>  native light chain leader peptide

<220>
<221>  V_region
<222>  (62)..(397)
<223>  LIGHT CHAIN VARIABLE DOMAIN FOR MOUSE ANTI-HUMAN CD22:  G28-7

<220>
<221>  misc_feature
<222>  (398)..(445)
<223>  (gly4ser)3 linker peptide

<220>
<221> V_region
<222> (445)..(818)
<223> HEAVY CHAIN VARIABLE DOMAIN FOR MOUSE ANTI-HUMAN CD22: G28-7

<220>
<221> misc_feature
<222> (819)..(824)
<223> BclI restriction site

<400> 10
atggagtcac attcccaggt ctttctctcc ctgctgctct gggtatctgg tacctgtggg 60
aacattatga tgacacagtc gccatcatct ctggctgtgt cagcaggaga aaaggtcact 120
atgaactgta agtccagtca aagtgttttc tacagttcaa atcagaggaa ttatttggcc 180
tggtatcagc agaaaccagg gcagtctccc aaattgctga tctactgggc atctactagg 240
gaatctggtg tccctgatcg cttcacaggc agtggatccg ggacagactt tactcttacc 300
atcagcagtg tacatactga agacctggca gtttattact gtcatcaatt cctctcttcg 360
tggacgttcg gtggaggcac caagctggaa atcaaaggcg gtggtggttc gggtggtggt 420
ggttcgggtg gcggcggatc ttctcaggtc caactgcagc agcctggggc tgaactggtg 480
aagcctggga cttcagtgaa gctgtcctgc aaggcctctg gctacacctt caccaactac 540
tggatggtct gggtgaagca gacgcctgga gaaggccttg agtggattgg agaaattatt 600
cctagcaacg gtcgtactaa atacaatgag aagttcaaga gcaaggccac actgactgca 660
gacaaatcct cccgcacagc ctacatgcaa ctcagcagcc tggcatctga ggactctgcg 720
gtctattatt gtgcaagaga gatgtccatt attactacgg tactgactcc cggtttgctt 780
actggggcca agggactctg gtcactgtct ctgcagcctg atca 824

<210> 11
<211> 266
<212> PRT
<213> Mus musculus

<220>
<221> INIT_MET
<222> (1)..(1)

<220>
<221> SIGNAL
<222> (1)..(22)

<220>
<221> DOMAIN
<222> (23)..(128)
<223> LIGHT CHAIN VARIABLE DOMAIN FOR MOUSE ANTI-HUMAN CD20

<220>
<221> SITE
<222> (129)..(144)
<223> ASP-(GLY3SER)-(GLY4SER)2-SER LINKER PEPTIDE

<220>
<221> DOMAIN
<222> (145)..(266)
<223> HEAVY CHAIN VARIABLE DOMAIN FOR MOUSE ANTI-HUMAN CD20

<400> 11
Met Asp Phe Gln Val Gln Ile Phe Ser Phe Leu Leu Ile Ser Ala Ser
1               5                   10                  15
Val Ile Ile Ala Arg Gly Gln Ile Val Leu Ser Gln Ser Pro Ala Ile
            20                  25                  30
Leu Ser Ala Ser Pro Gly Glu Lys Val Thr Met Thr Cys Arg Ala Ser
        35                  40                  45

Ser Ser Val Ser Tyr Met His Trp Tyr Gln Gln Lys Pro Gly Ser Ser
    50                  55                  60
Pro Lys Pro Trp Ile Tyr Ala Pro Ser Asn Leu Ala Ser Gly Val Pro
65                  70                  75                  80
Ala Arg Phe Ser Gly Ser Gly Ser Gly Thr Ser Tyr Ser Leu Thr Ile
                85                  90                  95
Ser Arg Val Glu Ala Glu Asp Ala Ala Thr Tyr Tyr Cys Gln Gln Trp
            100                 105                 110
Ser Phe Asn Pro Pro Thr Phe Gly Ala Gly Thr Lys Leu Glu Leu Lys
        115                 120                 125
Asp Gly Gly Gly Ser Gly Gly Gly Ser Gly Gly Gly Gly Ser Ser
    130                 135                 140
Gln Ala Tyr Leu Gln Gln Ser Gly Ala Glu Leu Val Arg Pro Gly Ala
145                 150                 155                 160
Ser Val Lys Met Ser Cys Lys Ala Ser Gly Tyr Thr Phe Thr Ser Tyr
                165                 170                 175
Asn Met His Trp Val Lys Gln Thr Pro Arg Gln Gly Leu Glu Trp Ile
            180                 185                 190
Gly Ala Ile Tyr Pro Gly Asn Gly Asp Thr Ser Tyr Asn Gln Lys Phe
        195                 200                 205
Lys Gly Lys Ala Thr Leu Thr Val Asp Lys Ser Ser Ser Thr Ala Tyr
    210                 215                 220
Met Gln Leu Ser Ser Leu Thr Ser Glu Asp Ser Ala Val Tyr Phe Cys
225                 230                 235                 240
Ala Arg Val Val Tyr Tyr Ser Asn Ser Tyr Trp Tyr Phe Asp Val Trp
                245                 250                 255
Gly Thr Gly Thr Thr Val Thr Val Ser Asp
            260                 265

<210>  12
<211>  271
<212>  PRT
<213>  Mus musculus

<220>
<221>  SITE
<222>  (1)..(271)
<223>  MOUSE ANTI-HUMAN CD19 SCFV

<400>  12
Met Glu Thr Asp Thr Leu Leu Leu Trp Val Leu Leu Leu Trp Val Pro
1               5                   10                  15
Gly Ser Thr Gly Asp Ile Val Leu Thr Gln Ser Pro Ala Ser Leu Ala
            20                  25                  30
Val Ser Leu Gly Gln Arg Ala Thr Ile Ser Cys Lys Ala Ser Gln Ser
        35                  40                  45
Val Asp Tyr Asp Gly Asp Ser Tyr Leu Asn Trp Tyr Gln Gln Ile Pro
    50                  55                  60
Gly Gln Pro Pro Lys Leu Leu Ile Tyr Asp Ala Ser Asn Leu Val Ser
65                  70                  75                  80
Gly Ile Pro Pro Arg Phe Ser Gly Ser Gly Ser Gly Thr Asp Phe Thr
                85                  90                  95
Leu Asn Ile His Pro Val Glu Lys Val Asp Ala Ala Thr Tyr His Cys
            100                 105                 110
Gln Gln Ser Thr Glu Asp Pro Trp Thr Phe Gly Gly Gly Thr Lys Leu
        115                 120                 125
Glu Ile Lys Gly Gly Gly Gly Ser Gly Gly Gly Gly Ser Gly Gly Gly
    130                 135                 140
Gly Ser Ser Gln Val Gln Leu Gln Gln Ser Gly Ala Glu Leu Val Arg
145                 150                 155                 160
Pro Gly Ser Ser Val Lys Ile Ser Cys Lys Ala Ser Gly Tyr Ala Phe
                165                 170                 175

Ser Ser Tyr Trp Met Asn Trp Val Lys Gln Arg Pro Gly Gln Gly Leu
            180                 185                 190
Glu Trp Ile Gly Gln Ile Trp Pro Gly Asp Gly Asp Thr Asn Tyr Asn
        195                 200                 205
Gly Lys Phe Lys Gly Lys Ala Thr Leu Thr Ala Asp Glu Ser Ser Ser
    210                 215                 220
Thr Ala Tyr Met Gln Leu Ser Ser Leu Ala Ser Glu Asp Ser Ala Val
225                 230                 235                 240
Tyr Phe Cys Ala Arg Arg Glu Thr Thr Thr Val Gly Arg Tyr Tyr Tyr
                245                 250                 255
Ala Met Asp Tyr Trp Gly Gln Gly Thr Ser Val Thr Val Ser Ser
            260                 265                 270

<210>  13
<211>  259
<212>  PRT
<213>  Mus musculus

<220>
<221>  SITE
<222>  (1)..(259)
<223>  MOUSE ANTI-HUMAN CD37 SCFV

<400>  13
Met Val Ser Thr Ala Gln Phe Leu Gly Leu Leu Leu Leu Trp Leu Thr
1               5                   10                  15
Gly Gly Arg Cys Asp Ile Gln Met Thr Gln Ser Pro Ala Ser Leu Ser
            20                  25                  30
Ala Ser Val Gly Glu Thr Val Thr Ile Thr Cys Arg Thr Ser Glu Asn
        35                  40                  45
Val Tyr Ser Tyr Leu Ala Trp Tyr Gln Gln Lys Gln Gly Lys Ser Pro
    50                  55                  60
Gln Leu Leu Val Ser Phe Ala Lys Thr Leu Ala Glu Gly Val Pro Ser
65                  70                  75                  80
Arg Phe Ser Gly Ser Gly Ser Gly Thr Gln Phe Ser Leu Lys Ile Ser
                85                  90                  95
Ser Leu Gln Pro Glu Asp Ser Gly Ser Tyr Phe Cys Gln His His Ser
            100                 105                 110
Asp Asn Pro Trp Thr Phe Gly Gly Gly Thr Glu Leu Glu Ile Lys Gly
        115                 120                 125
Gly Gly Gly Ser Gly Gly Gly Gly Ser Gly Gly Gly Gly Ser Ser Ala
    130                 135                 140
Val Gln Leu Gln Gln Ser Gly Pro Glu Leu Glu Lys Pro Gly Ala Ser
145                 150                 155                 160
Val Lys Ile Ser Cys Lys Ala Ser Gly Tyr Ser Phe Thr Gly Tyr Asn
                165                 170                 175
Met Asn Trp Val Lys Gln Asn Asn Gly Lys Ser Leu Glu Trp Ile Gly
            180                 185                 190
Asn Ile Asp Pro Tyr Tyr Gly Gly Thr Thr Tyr Asn Arg Lys Phe Lys
        195                 200                 205
Gly Lys Ala Thr Leu Thr Val Asp Lys Ser Ser Ser Thr Ala Tyr Met
    210                 215                 220
Gln Leu Lys Ser Leu Thr Ser Glu Asp Ser Ala Val Tyr Tyr Cys Ala
225                 230                 235                 240
Arg Ser Val Gly Pro Met Asp Tyr Trp Gly Gln Gly Thr Ser Val Thr
                245                 250                 255
Val Ser Ser

<210>  14
<211>  272
<212>  PRT

<213>  Mus musculus

<220>
<221>  SITE
<222>  (1)..(272)
<223>  MOUSE ANTI-HUMAN CD22 SCFV

<400>  14
Met Glu Ser His Ser Gln Val Phe Leu Ser Leu Leu Leu Trp Val Ser
1               5                   10                  15
Gly Thr Cys Gly Asn Ile Met Met Thr Gln Ser Pro Ser Ser Leu Ala
            20                  25                  30
Val Ser Ala Gly Glu Lys Val Thr Met Asn Cys Lys Ser Ser Gln Ser
        35                  40                  45
Val Phe Tyr Ser Ser Asn Gln Arg Asn Tyr Leu Ala Trp Tyr Gln Gln
    50                  55                  60
Lys Pro Gly Gln Ser Pro Lys Leu Leu Ile Tyr Trp Ala Ser Thr Arg
65                  70                  75                  80
Glu Ser Gly Val Pro Asp Arg Phe Thr Gly Ser Gly Ser Gly Thr Asp
                85                  90                  95
Phe Thr Leu Thr Ile Ser Ser Val His Thr Glu Asp Leu Ala Val Tyr
            100                 105                 110
Tyr Cys His Gln Phe Leu Ser Ser Trp Thr Phe Gly Gly Gly Thr Lys
        115                 120                 125
Leu Glu Ile Lys Gly Gly Gly Gly Ser Gly Gly Gly Gly Ser Gly Gly
    130                 135                 140
Gly Gly Ser Ser Gln Val Gln Leu Gln Gln Pro Gly Ala Glu Leu Val
145                 150                 155                 160
Lys Pro Gly Thr Ser Val Lys Leu Ser Cys Lys Ala Ser Gly Tyr Thr
                165                 170                 175
Phe Thr Asn Tyr Trp Met Val Trp Val Lys Gln Thr Pro Gly Glu Gly
            180                 185                 190
Leu Glu Trp Ile Gly Glu Ile Ile Pro Ser Asn Gly Arg Thr Lys Tyr
        195                 200                 205
Asn Glu Lys Phe Lys Ser Lys Ala Thr Leu Thr Ala Asp Lys Ser Ser
    210                 215                 220
Arg Thr Ala Tyr Met Gln Leu Ser Ser Leu Ala Ser Glu Asp Ser Ala
225                 230                 235                 240
Val Tyr Tyr Cys Ala Arg Glu Met Ser Ile Ile Thr Thr Val Leu Thr
                245                 250                 255
Pro Gly Leu Leu Thr Gly Ala Lys Gly Leu Trp Ser Leu Ser Leu Gln
            260                 265                 270

<210>  15
<211>  499
<212>  PRT
<213>  Artificial Sequence

<220>
<223>  MOUSE-HUMAN HYBRID FUSION PROTEIN

<220>
<221>  SITE
<222>  (1)..(265)
<223>  MOUSE ANTI-HUMAN CD20 SCFV:  2H7

<220>
<221>  DOMAIN
<222>  (266)..(499)
<223>  HUMAN IGG1 WILD TYPE HINGE, CH2, CH3 FC

<400>  15

Met Asp Phe Gln Val Gln Ile Phe Ser Phe Leu Leu Ile Ser Ala Ser
1               5                   10                  15
Val Ile Ile Ala Arg Gly Gln Ile Val Leu Ser Gln Ser Pro Ala Ile
            20                  25                  30
Leu Ser Ala Ser Pro Gly Glu Lys Val Thr Met Thr Cys Arg Ala Ser
        35                  40                  45
Ser Ser Val Ser Tyr Met His Trp Tyr Gln Gln Lys Pro Gly Ser Ser
    50                  55                  60
Pro Lys Pro Trp Ile Tyr Ala Pro Ser Asn Leu Ala Ser Gly Val Pro
65                  70                  75                  80
Ala Arg Phe Ser Gly Ser Gly Ser Gly Thr Ser Tyr Ser Leu Thr Ile
                85                  90                  95
Ser Arg Val Glu Ala Glu Asp Ala Ala Thr Tyr Tyr Cys Gln Gln Trp
            100                 105                 110
Ser Phe Asn Pro Pro Thr Phe Gly Ala Gly Thr Lys Leu Glu Leu Lys
        115                 120                 125
Asp Gly Gly Gly Ser Gly Gly Gly Gly Ser Gly Gly Gly Gly Ser Ser
    130                 135                 140
Gln Ala Tyr Leu Gln Gln Ser Gly Ala Glu Leu Val Arg Pro Gly Ala
145                 150                 155                 160
Ser Val Lys Met Ser Cys Lys Ala Ser Gly Tyr Thr Phe Thr Ser Tyr
                165                 170                 175
Asn Met His Trp Val Lys Gln Thr Pro Arg Gln Gly Leu Glu Trp Ile
            180                 185                 190
Gly Ala Ile Tyr Pro Gly Asn Gly Asp Thr Ser Tyr Asn Gln Lys Phe
        195                 200                 205
Lys Gly Lys Ala Thr Leu Thr Val Asp Lys Ser Ser Ser Thr Ala Tyr
    210                 215                 220
Met Gln Leu Ser Ser Leu Thr Ser Glu Asp Ser Ala Val Tyr Phe Cys
225                 230                 235                 240
Ala Arg Val Val Tyr Tyr Ser Asn Ser Tyr Trp Tyr Phe Asp Val Trp
                245                 250                 255
Gly Thr Gly Thr Thr Val Thr Val Ser Asp Gln Glu Pro Lys Ser Cys
            260                 265                 270
Asp Lys Thr His Thr Cys Pro Pro Cys Pro Ala Pro Glu Leu Leu Gly
        275                 280                 285
Gly Pro Ser Val Phe Leu Phe Pro Pro Lys Pro Lys Asp Thr Leu Met
    290                 295                 300
Ile Ser Arg Thr Pro Glu Val Thr Cys Val Val Val Asp Val Ser His
305                 310                 315                 320
Glu Asp Pro Glu Val Lys Phe Asn Trp Tyr Val Asp Gly Val Glu Val
                325                 330                 335
His Asn Ala Lys Thr Lys Pro Arg Glu Glu Gln Tyr Asn Ser Thr Tyr
            340                 345                 350
Arg Val Val Ser Val Leu Thr Val Leu His Gln Asp Trp Leu Asn Gly
        355                 360                 365
Lys Glu Tyr Lys Cys Lys Val Ser Asn Lys Ala Leu Pro Ala Pro Ile
    370                 375                 380
Glu Lys Thr Ile Ser Lys Ala Lys Gly Gln Pro Arg Glu Pro Gln Val
385                 390                 395                 400
Tyr Thr Leu Pro Pro Ser Arg Asp Glu Leu Thr Lys Asn Gln Val Ser
                405                 410                 415
Leu Thr Cys Leu Val Lys Gly Phe Tyr Pro Ser Asp Ile Ala Val Glu
            420                 425                 430
Trp Glu Ser Asn Gly Gln Pro Glu Asn Asn Tyr Lys Thr Thr Pro Pro
        435                 440                 445
Val Leu Asp Ser Asp Gly Ser Phe Phe Leu Tyr Ser Lys Leu Thr Val
    450                 455                 460
Asp Lys Ser Arg Trp Gln Gln Gly Asn Val Phe Ser Cys Ser Val Met
465                 470                 475                 480
His Glu Ala Leu His Asn His Tyr Thr Gln Lys Ser Leu Ser Leu Ser
                485                 490                 495

Pro Gly Lys

<210> 16
<211> 499
<212> PRT
<213> Artificial Sequence

<220>
<223> MOUSE-HUMAN HYBRID FUSION PROTEIN

<220>
<221> SITE
<222> (1)..(265)
<223> 2H7 SCFV TARGETED TO HUMAN CD20

<220>
<221> DOMAIN
<222> (265)..(499)
<223> HINGE CYSTEINES MUTATED TO SERINES (AMINO ACIDS 272, 278, 281)
      PROLINE IN CH2 MUTATED TO SERINE (AMINO ACID 290)

<400> 16
Met Asp Phe Gln Val Gln Ile Phe Ser Phe Leu Leu Ile Ser Ala Ser
1               5                   10                  15
Val Ile Ile Ala Arg Gly Gln Ile Val Leu Ser Gln Ser Pro Ala Ile
            20                  25                  30
Leu Ser Ala Ser Pro Gly Glu Lys Val Thr Met Thr Cys Arg Ala Ser
        35                  40                  45
Ser Ser Val Ser Tyr Met His Trp Tyr Gln Gln Lys Pro Gly Ser Ser
    50                  55                  60
Pro Lys Pro Trp Ile Tyr Ala Pro Ser Asn Leu Ala Ser Gly Val Pro
65                  70                  75                  80
Ala Arg Phe Ser Gly Ser Gly Ser Gly Thr Ser Tyr Ser Leu Thr Ile
                85                  90                  95
Ser Arg Val Glu Ala Glu Asp Ala Ala Thr Tyr Tyr Cys Gln Gln Trp
            100                 105                 110
Ser Phe Asn Pro Pro Thr Phe Gly Ala Gly Thr Lys Leu Glu Leu Lys
        115                 120                 125
Asp Gly Gly Gly Ser Gly Gly Gly Gly Ser Gly Gly Gly Gly Ser Ser
    130                 135                 140
Gln Ala Tyr Leu Gln Gln Ser Gly Ala Glu Leu Val Arg Pro Gly Ala
145                 150                 155                 160
Ser Val Lys Met Ser Cys Lys Ala Ser Gly Tyr Thr Phe Thr Ser Tyr
                165                 170                 175
Asn Met His Trp Val Lys Gln Thr Pro Arg Gln Gly Leu Glu Trp Ile
            180                 185                 190
Gly Ala Ile Tyr Pro Gly Asn Gly Asp Thr Ser Tyr Asn Gln Lys Phe
        195                 200                 205
Lys Gly Lys Ala Thr Leu Thr Val Asp Lys Ser Ser Ser Thr Ala Tyr
    210                 215                 220
Met Gln Leu Ser Ser Leu Thr Ser Glu Asp Ser Ala Val Tyr Phe Cys
225                 230                 235                 240
Ala Arg Val Val Tyr Tyr Ser Asn Ser Tyr Trp Tyr Phe Asp Val Trp
                245                 250                 255
Gly Thr Gly Thr Thr Val Thr Val Ser Asp Gln Glu Pro Lys Ser Ser
            260                 265                 270
Asp Lys Thr His Thr Ser Pro Pro Ser Pro Ala Pro Glu Leu Leu Gly
        275                 280                 285
Gly Ser Ser Val Phe Leu Phe Pro Pro Lys Pro Lys Asp Thr Leu Met
    290                 295                 300
Ile Ser Arg Thr Pro Glu Val Thr Cys Val Val Val Asp Val Ser His

305                 310                 315                 320
Glu Asp Pro Glu Val Lys Phe Asn Trp Tyr Val Asp Gly Val Glu Val
                325                 330                 335
His Asn Ala Lys Thr Lys Pro Arg Glu Glu Gln Tyr Asn Ser Thr Tyr
            340                 345                 350
Arg Val Val Ser Val Leu Thr Val Leu His Gln Asp Trp Leu Asn Gly
        355                 360                 365
Lys Glu Tyr Lys Cys Lys Val Ser Asn Lys Ala Leu Pro Ala Pro Ile
    370                 375                 380
Glu Lys Thr Ile Ser Lys Ala Lys Gly Gln Pro Arg Glu Pro Gln Val
385                 390                 395                 400
Tyr Thr Leu Pro Pro Ser Arg Asp Glu Leu Thr Lys Asn Gln Val Ser
                405                 410                 415
Leu Thr Cys Leu Val Lys Gly Phe Tyr Pro Ser Asp Ile Ala Val Glu
            420                 425                 430
Trp Glu Ser Asn Gly Gln Pro Glu Asn Asn Tyr Lys Thr Thr Pro Pro
        435                 440                 445
Val Leu Asp Ser Asp Gly Ser Phe Phe Leu Tyr Ser Lys Leu Thr Val
    450                 455                 460
Asp Lys Ser Arg Trp Gln Gln Gly Asn Val Phe Ser Cys Ser Val Met
465                 470                 475                 480
His Glu Ala Leu His Asn His Tyr Thr Gln Lys Ser Leu Ser Leu Ser
                485                 490                 495
Pro Gly Lys


<210>  17
<211>  499
<212>  PRT
<213>  Artificial Sequence

<220>
<223>  MOUSE-HUMAN HYBRID FUSION PROTEIN

<220>
<221>  SITE
<222>  (1)..(265)
<223>  MOUSE ANTI-HUMAN CD20 SCFV:  2H7

<220>
<221>  DOMAIN
<222>  (266)..(499)
<223>  HINGE CYSTEINES MUTATED TO SERINES (AMINO ACIDS 272, 278, 281)
       CH2 AND CH3 DOMAINS ARE WILD TYPE IN SEQUENCE

<400>  17
Met Asp Phe Gln Val Gln Ile Phe Ser Phe Leu Leu Ile Ser Ala Ser
1               5                   10                  15
Val Ile Ile Ala Arg Gly Gln Ile Val Leu Ser Gln Ser Pro Ala Ile
            20                  25                  30
Leu Ser Ala Ser Pro Gly Glu Lys Val Thr Met Thr Cys Arg Ala Ser
        35                  40                  45
Ser Ser Val Ser Tyr Met His Trp Tyr Gln Gln Lys Pro Gly Ser Ser
    50                  55                  60
Pro Lys Pro Trp Ile Tyr Ala Pro Ser Asn Leu Ala Ser Gly Val Pro
65                  70                  75                  80
Ala Arg Phe Ser Gly Ser Gly Ser Gly Thr Ser Tyr Ser Leu Thr Ile
                85                  90                  95
Ser Arg Val Glu Ala Glu Asp Ala Ala Thr Tyr Tyr Cys Gln Gln Trp
            100                 105                 110
Ser Phe Asn Pro Pro Thr Phe Gly Ala Gly Thr Lys Leu Glu Leu Lys
        115                 120                 125

Asp Gly Gly Gly Ser Gly Gly Gly Gly Ser Gly Gly Gly Gly Ser Ser
    130                 135                 140
Gln Ala Tyr Leu Gln Gln Ser Gly Ala Glu Leu Val Arg Pro Gly Ala
145                 150                 155                 160
Ser Val Lys Met Ser Cys Lys Ala Ser Gly Tyr Thr Phe Thr Ser Tyr
                165                 170                 175
Asn Met His Trp Val Lys Gln Thr Pro Arg Gln Gly Leu Glu Trp Ile
            180                 185                 190
Gly Ala Ile Tyr Pro Gly Asn Gly Asp Thr Ser Tyr Asn Gln Lys Phe
        195                 200                 205
Lys Gly Lys Ala Thr Leu Thr Val Asp Lys Ser Ser Ser Thr Ala Tyr
    210                 215                 220
Met Gln Leu Ser Ser Leu Thr Ser Glu Asp Ser Ala Val Tyr Phe Cys
225                 230                 235                 240
Ala Arg Val Val Tyr Tyr Ser Asn Ser Tyr Trp Tyr Phe Asp Val Trp
                245                 250                 255
Gly Thr Gly Thr Thr Val Thr Val Ser Asp Gln Glu Pro Lys Ser Ser
            260                 265                 270
Asp Lys Thr His Thr Ser Pro Pro Ser Pro Ala Pro Glu Leu Leu Gly
        275                 280                 285
Gly Pro Ser Val Phe Leu Phe Pro Pro Lys Pro Lys Asp Thr Leu Met
    290                 295                 300
Ile Ser Arg Thr Pro Glu Val Thr Cys Val Val Val Asp Val Ser His
305                 310                 315                 320
Glu Asp Pro Glu Val Lys Phe Asn Trp Tyr Val Asp Gly Val Glu Val
                325                 330                 335
His Asn Ala Lys Thr Lys Pro Arg Glu Glu Gln Tyr Asn Ser Thr Tyr
            340                 345                 350
Arg Val Val Ser Val Leu Thr Val Leu His Gln Asp Trp Leu Asn Gly
        355                 360                 365
Lys Glu Tyr Lys Cys Lys Val Ser Asn Lys Ala Leu Pro Ala Pro Ile
    370                 375                 380
Glu Lys Thr Ile Ser Lys Ala Lys Gly Gln Pro Arg Glu Pro Gln Val
385                 390                 395                 400
Tyr Thr Leu Pro Pro Ser Arg Asp Glu Leu Thr Lys Asn Gln Val Ser
                405                 410                 415
Leu Thr Cys Leu Val Lys Gly Phe Tyr Pro Ser Asp Ile Ala Val Glu
            420                 425                 430
Trp Glu Ser Asn Gly Gln Pro Glu Asn Asn Tyr Lys Thr Thr Pro Pro
        435                 440                 445
Val Leu Asp Ser Asp Gly Ser Phe Phe Leu Tyr Ser Lys Leu Thr Val
    450                 455                 460
Asp Lys Ser Arg Trp Gln Gln Gly Asn Val Phe Ser Cys Ser Val Met
465                 470                 475                 480
His Glu Ala Leu His Asn His Tyr Thr Gln Lys Ser Leu Ser Leu Ser
                485                 490                 495
Pro Gly Lys

<210>  18
<211>  505
<212>  PRT
<213>  Artificial Sequence

<220>
<223>  MOUSE-HUMAN FUSION PROTEIN

<220>
<221>  SITE
<222>  (1)..(265)
<223>  MOUSE ANTI-HUMAN CD20 SCFV:  2H7

<220>
<221>  DOMAIN
<222>  (266)..(288)
<223>  WILD TYPE IGA HINGE

<220>
<221>  DOMAIN
<222>  (289)..(505)
<223>  HUMAN IGG1 CH2 AND CH3 DOMAINS, WILD TYPE SEQUENCE

<400>  18
Met Asp Phe Gln Val Gln Ile Phe Ser Phe Leu Leu Ile Ser Ala Ser
1               5                   10                  15
Val Ile Ile Ala Arg Gly Gln Ile Val Leu Ser Gln Ser Pro Ala Ile
            20                  25                  30
Leu Ser Ala Ser Pro Gly Glu Lys Val Thr Met Thr Cys Arg Ala Ser
        35                  40                  45
Ser Ser Val Ser Tyr Met His Trp Tyr Gln Gln Lys Pro Gly Ser Ser
    50                  55                  60
Pro Lys Pro Trp Ile Tyr Ala Pro Ser Asn Leu Ala Ser Gly Val Pro
65                  70                  75                  80
Ala Arg Phe Ser Gly Ser Gly Ser Gly Thr Ser Tyr Ser Leu Thr Ile
                85                  90                  95
Ser Arg Val Glu Ala Glu Asp Ala Ala Thr Tyr Tyr Cys Gln Gln Trp
            100                 105                 110
Ser Phe Asn Pro Pro Thr Phe Gly Ala Gly Thr Lys Leu Glu Leu Lys
        115                 120                 125
Asp Gly Gly Gly Ser Gly Gly Gly Gly Ser Gly Gly Gly Gly Ser Ser
    130                 135                 140
Gln Ala Tyr Leu Gln Gln Ser Gly Ala Glu Leu Val Arg Pro Gly Ala
145                 150                 155                 160
Ser Val Lys Met Ser Cys Lys Ala Ser Gly Tyr Thr Phe Thr Ser Tyr
                165                 170                 175
Asn Met His Trp Val Lys Gln Thr Pro Arg Gln Gly Leu Glu Trp Ile
            180                 185                 190
Gly Ala Ile Tyr Pro Gly Asn Gly Asp Thr Ser Tyr Asn Gln Lys Phe
        195                 200                 205
Lys Gly Lys Ala Thr Leu Thr Val Asp Lys Ser Ser Ser Thr Ala Tyr
    210                 215                 220
Met Gln Leu Ser Ser Leu Thr Ser Glu Asp Ser Ala Val Tyr Phe Cys
225                 230                 235                 240
Ala Arg Val Val Tyr Tyr Ser Asn Ser Tyr Trp Tyr Phe Asp Val Trp
                245                 250                 255
Gly Thr Gly Thr Thr Val Thr Val Ser Asp Gln Pro Val Pro Ser Thr
            260                 265                 270
Pro Pro Thr Pro Ser Pro Ser Thr Pro Pro Thr Pro Ser Pro Ser Cys
        275                 280                 285
Ala Pro Glu Leu Leu Gly Gly Pro Ser Val Phe Leu Phe Pro Pro Lys
    290                 295                 300
Pro Lys Asp Thr Leu Met Ile Ser Arg Thr Pro Glu Val Thr Cys Val
305                 310                 315                 320
Val Val Asp Val Ser His Glu Asp Pro Glu Val Lys Phe Asn Trp Tyr
                325                 330                 335
Val Asp Gly Val Glu Val His Asn Ala Lys Thr Lys Pro Arg Glu Glu
            340                 345                 350
Gln Tyr Asn Ser Thr Tyr Arg Val Val Ser Val Leu Thr Val Leu His
        355                 360                 365
Gln Asp Trp Leu Asn Gly Lys Glu Tyr Lys Cys Lys Val Ser Asn Lys
    370                 375                 380
Ala Leu Pro Ala Pro Ile Glu Lys Thr Ile Ser Lys Ala Lys Gly Gln
385                 390                 395                 400
Pro Arg Glu Pro Gln Val Tyr Thr Leu Pro Pro Ser Arg Asp Glu Leu

405                 410                 415
Thr Lys Asn Gln Val Ser Leu Thr Cys Leu Val Lys Gly Phe Tyr Pro
            420                 425                 430
Ser Asp Ile Ala Val Glu Trp Glu Ser Asn Gly Gln Pro Glu Asn Asn
        435                 440                 445
Tyr Lys Thr Thr Pro Pro Val Leu Asp Ser Asp Gly Ser Phe Phe Leu
    450                 455                 460
Tyr Ser Lys Leu Thr Val Asp Lys Ser Arg Trp Gln Gln Gly Asn Val
465                 470                 475                 480
Phe Ser Cys Ser Val Met His Glu Ala Leu His Asn His Tyr Thr Gln
                485                 490                 495
Lys Ser Leu Ser Leu Ser Pro Gly Lys
            500                 505

<210>  19
<211>  234
<212>  PRT
<213>  Homo sapiens

<220>
<221>  DOMAIN
<222>  (1)..(234)
<223>  MUTANT IGG1 HINGE (AMINO ACIDS 7, 13, 16)
       WILD TYPE CH2 AND CH3 DOMAINS
       ALTERNATIVE CARBOXY TERMINUS OF SCFVIG FUSION PROTEINS

<400>  19
Asp Gln Glu Pro Lys Ser Ser Asp Lys Thr His Thr Ser Pro Pro Ser
1               5                   10                  15
Pro Ala Pro Glu Leu Leu Gly Gly Pro Ser Val Phe Leu Phe Pro Pro
            20                  25                  30
Lys Pro Lys Asp Thr Leu Met Ile Ser Arg Thr Pro Glu Val Thr Cys
        35                  40                  45
Val Val Val Asp Val Ser His Glu Asp Pro Glu Val Lys Phe Asn Trp
    50                  55                  60
Tyr Val Asp Gly Val Glu Val His Asn Ala Lys Thr Lys Pro Arg Glu
65                  70                  75                  80
Glu Gln Tyr Asn Ser Thr Tyr Arg Val Val Ser Val Leu Thr Val Leu
                85                  90                  95
His Gln Asp Trp Leu Asn Gly Lys Glu Tyr Lys Cys Lys Val Ser Asn
            100                 105                 110
Lys Ala Leu Pro Ala Pro Ile Glu Lys Thr Ile Ser Lys Ala Lys Gly
        115                 120                 125
Gln Pro Arg Glu Pro Gln Val Tyr Thr Leu Pro Pro Ser Arg Asp Glu
    130                 135                 140
Leu Thr Lys Asn Gln Val Ser Leu Thr Cys Leu Val Lys Gly Phe Tyr
145                 150                 155                 160
Pro Ser Asp Ile Ala Val Glu Trp Glu Ser Asn Gly Gln Pro Glu Asn
                165                 170                 175
Asn Tyr Lys Thr Thr Pro Pro Val Leu Asp Ser Asp Gly Ser Phe Phe
            180                 185                 190
Leu Tyr Ser Lys Leu Thr Val Asp Lys Ser Arg Trp Gln Gln Gly Asn
        195                 200                 205
Val Phe Ser Cys Ser Val Met His Glu Ala Leu His Asn His Tyr Thr
    210                 215                 220
Gln Lys Ser Leu Ser Leu Ser Pro Gly Lys
225                 230

<210>  20
<211>  240
<212>  PRT

<213>  Homo sapiens

<220>
<221>  SITE
<222>  (1)..(23)
<223>  ALTERNATIVE CARBOXY TERMINUS OF SCFVIG FUSION PROTEINS

<220>
<221>  DOMAIN
<222>  (24)..(240)
<223>  HUMAN IGG1 WILD TYPE CH2 AND CH3 FC

<400>  20
Asp Gln Pro Val Pro Ser Thr Pro Pro Thr Pro Ser Pro Ser Thr Pro
1               5                   10                  15
Pro Thr Pro Ser Pro Ser Cys Ala Pro Glu Leu Leu Gly Gly Pro Ser
            20                  25                  30
Val Phe Leu Phe Pro Pro Lys Pro Lys Asp Thr Leu Met Ile Ser Arg
        35                  40                  45
Thr Pro Glu Val Thr Cys Val Val Val Asp Val Ser His Glu Asp Pro
    50                  55                  60
Glu Val Lys Phe Asn Trp Tyr Val Asp Gly Val Glu Val His Asn Ala
65                  70                  75                  80
Lys Thr Lys Pro Arg Glu Glu Gln Tyr Asn Ser Thr Tyr Arg Val Val
                85                  90                  95
Ser Val Leu Thr Val Leu His Gln Asp Trp Leu Asn Gly Lys Glu Tyr
            100                 105                 110
Lys Cys Lys Val Ser Asn Lys Ala Leu Pro Ala Pro Ile Glu Lys Thr
        115                 120                 125
Ile Ser Lys Ala Lys Gly Gln Pro Arg Glu Pro Gln Val Tyr Thr Leu
    130                 135                 140
Pro Pro Ser Arg Asp Glu Leu Thr Lys Asn Gln Val Ser Leu Thr Cys
145                 150                 155                 160
Leu Val Lys Gly Phe Tyr Pro Ser Asp Ile Ala Val Glu Trp Glu Ser
                165                 170                 175
Asn Gly Gln Pro Glu Asn Asn Tyr Lys Thr Thr Pro Pro Val Leu Asp
            180                 185                 190
Ser Asp Gly Ser Phe Phe Leu Tyr Ser Lys Leu Thr Val Asp Lys Ser
        195                 200                 205
Arg Trp Gln Gln Gly Asn Val Phe Ser Cys Ser Val Met His Glu Ala
    210                 215                 220
Leu His Asn His Tyr Thr Gln Lys Ser Leu Ser Leu Ser Pro Gly Lys
225                 230                 235                 240

<210>  21
<211>  1470
<212>  DNA
<213>  Artificial Sequence

<220>
<223>  MOUSE-HUMAN HYBRID

<220>
<221>  misc_feature
<222>  (1)..(808)
<223>  MOUSE ANTI-HUMAN CD20 SCFV

<220>
<221>  misc_feature
<222>  (814)..(1455)
<223>  HUMAN EXTRACELLULAR DOMAIN LONG FORM, CD154

<400>  21
aagcttgccg ccatggattt tcaagtgcag attttcagct tcctgctaat cagtgcttca      60
gtcataattg ccagaggaca aattgttctc tcccagtctc cagcaatcct gtctgcatct     120
ccaggggaga aggtcacaat gacttgcagg gccagctcaa gtgtaagtta catgcactgg     180
taccagcaga agccaggatc ctcccccaaa ccctggattt atgccccatc caacctggct     240
tctggagtcc ctgctcgctt cagtggcagt gggtctggga cctcttactc tctcacaatc     300
agcagagtgg aggctgaaga tgctgccact tattactgcc agcagtggag ttttaaccca     360
cccacgttcg gtgctgggac caagctggag ctgaaagatg gcggtggctc gggcggtggt     420
ggatctggag gaggtgggag ctctcaggct tatctacagc agtctggggc tgagctggtg     480
aggcctgggg cctcagtgaa gatgtcctgc aaggcttctg gctacacatt taccagttac     540
aatatgcact gggtaaagca gacacctaga cagggcctgg aatggattgg agctatttat     600
ccaggaaatg gtgatacttc ctacaatcag aagttcaagg gcaaggccac actgactgta     660
gacaaatcct ccagcacagc ctacatgcag ctcagcagcc tgacatctga agactctgcg     720
gtctatttct gtgcaagagt ggtgtactat agtaactctt actggtactt cgatgtctgg     780
ggcacaggga ccacggtcac cgtctctgat ccaagaaggt tggacaagat agaagatgaa     840
aggaatcttc atgaagattt tgtattcatg aaaacgatac agagatgcaa cacaggagaa     900
agatccttat ccttactgaa ctgtgaggag attaaaagcc agtttgaagg ctttgtgaag     960
gatataatgt taaacaaaga ggagacgaag aaagaaaaca gctttgaaat gcaaaaaggt    1020
gatcagaatc ctcaaattgc ggcacatgtc ataagtgagg ccagcagtaa aacaacatct    1080
gtgttacagt gggctgaaaa aggatactac accatgagca acaacttggt aaccctggaa    1140
aatgggaaac agctgaccgt taaaagacaa ggactctatt atatctatgc ccaagtcacc    1200
ttctgttcca atcgggaagc ttcgagtcaa gctccattta tagccagcct ctgcctaaag    1260
tcccccggta gattcgagag aatcttactc agagctgcaa atacccacag ttccgccaaa    1320
ccttgcgggc aacaatccat tcacttggga ggagtatttg aattgcaacc aggtgcttcg    1380
gtgtttgtca atgtgactga tccaagccaa gtgagccatg gcactggctt cacgtccttt    1440
ggcttactca aactcgagtg ataatctaga                                      1470


<210>  22
<211>  1290
<212>  DNA
<213>  Artificial Sequence

<220>
<223>  MOUSE-HUMAN HYBRID

<220>
<221>  misc_feature
<222>  (13)..(808)
<223>  MOUSE ANTI-HUMAN CD20 SCFV

<220>

<221>  misc_feature
<222>  (814)..(1275)
<223>  HUMAN EXTRACELLULAR DOMAIN, SHORT FORM, CD154

<400>  22
aagcttgccg ccatggattt tcaagtgcag attttcagct tcctgctaat cagtgcttca      60
gtcataattg ccagaggaca aattgttctc tcccagtctc cagcaatcct gtctgcatct     120
ccaggggaga aggtcacaat gacttgcagg gccagctcaa gtgtaagtta catgcactgg     180
taccagcaga agccaggatc ctcccccaaa ccctggattt atgccccatc caacctggct     240
tctggagtcc ctgctcgctt cagtggcagt gggtctggga cctcttactc tctcacaatc     300
agcagagtgg aggctgaaga tgctgccact tattactgcc agcagtggag ttttaaccca     360
cccacgttcg gtgctgggac caagctggag ctgaaagatg gcggtggctc gggcggtggt     420
ggatctggag gaggtgggag ctctcaggct tatctacagc agtctggggc tgagctggtg     480
aggcctgggg cctcagtgaa gatgtcctgc aaggcttctg gctacacatt taccagttac     540
aatatgcact gggtaaagca gacacctaga cagggcctgg aatggattgg agctatttat     600
ccaggaaatg gtgatacttc ctacaatcag aagttcaagg gcaaggccac actgactgta     660
gacaaatcct ccagcacagc ctacatgcag ctcagcagcc tgacatctga agactctgcg     720
gtctatttct gtgcaagagt ggtgtactat agtaactctt actggtactt cgatgtctgg     780
ggcacaggga ccacggtcac cgtctctgat ccagaaaaca gctttgaaat gcaaaaaggt     840

gatcagaatc ctcaaattgc ggcacatgtc ataagtgagg ccagcagtaa aacaacatct 900
gtgttacagt gggctgaaaa aggatactac accatgagca acaacttggt aaccctggaa 960
aatgggaaac agctgaccgt taaaagacaa ggactctatt atatctatgc ccaagtcacc 1020
ttctgttcca atcgggaagc ttcgagtcaa gctccattta tagccagcct ctgcctaaag 1080
tcccccggta gattcgagag aatcttactc agagctgcaa atacccacag ttccgccaaa 1140
ccttgcgggc aacaatccat tcacttggga ggagtatttg aattgcaacc aggtgcttcg 1200
gtgtttgtca atgtgactga tccaagccaa gtgagccatg gcactggctt cacgtccttt 1260
ggcttactca aactcgagtg ataatctaga 1290

<210> 23
<211> 43
<212> DNA
<213> Artificial Sequence

<220>
<223> OLIGONUCLEOTIDE

<400> 23
gtcaagcttg ccgccatgga ttttcaagtg cagatttttc agc 43

<210> 24
<211> 74
<212> DNA
<213> Artificial Sequence

<220>
<223> OLIGONUCLEOTIDE

<400> 24
gtcgtcgagc tcccacctcc tccagatcca ccaccgcccg agccaccgcc acctttcagc 60
tccagcttgg tccc 74

<210> 25
<211> 37
<212> DNA
<213> Artificial Sequence

<220>
<223> OLIGONUCLEOTIDE

<400> 25
gctgctgagc tctcaggctt atctacagca agtctgg 37

<210> 26
<211> 32
<212> DNA
<213> Artificial Sequence

<220>
<223> OLIGONUCLEOTIDE

<400> 26
gttgtctgat cagagacggt gaccgtggtc cc 32

<210> 27
<211> 34

<212>  DNA
<213>  Artificial Sequence

<220>
<223>  OLIGONUCLEOTIDE

<400>  27
gttgtcggat ccagaaaaca gctttgaaat gcaa                                34


<210>  28
<211>  44
<212>  DNA
<213>  Artificial Sequence

<220>
<223>  OLIGONUCLEOTIDE

<400>  28
gttgtttcta gattatcact cgagtttgag taagccaaag gacg                     44


<210>  29
<211>  35
<212>  DNA
<213>  Artificial Sequence

<220>
<223>  OLIGONUCLEOTIDE

<400>  29
gttgtcggat ccaagaaggt tggacaagat agaag                               35


<210>  30
<211>  23
<212>  DNA
<213>  Artificial Sequence

<220>
<223>  OLIGONUCLEOTIDE

<400>  30
gtctatataa gcagagctct ggc                                            23


<210>  31
<211>  25
<212>  DNA
<213>  Artificial Sequence

<220>
<223>  OLIGONUCLEOTIDE

<400>  31
cgaggctgat cagcgagctc tagca                                          25


<210>  32
<211>  25
<212>  DNA

<213>  Artificial Sequence

<220>
<223>  OLIGONUCLEOTIDE

<400>  32
ccgcaatttg aggattctga tcacc                                                25


<210>  33
<211>  482
<212>  PRT
<213>  Artificial Sequence

<220>
<223>  MOUSE-HUMAN HYBRID FUSION PROTEIN

<220>
<221>  SITE
<222>  (1)..(266)
<223>  MOUSE ANTI-HUMAN CD20 SCFV

<220>
<221>  DOMAIN
<222>  (268)..(481)
<223>  EXTRACELLULAR DOMAIN, LONG FORM, HUMAN CD154

<400>  33
Met Asp Phe Gln Val Gln Ile Phe Ser Phe Leu Leu Ile Ser Ala Ser
1               5                   10                  15
Val Ile Ile Ala Arg Gly Gln Ile Val Leu Ser Gln Ser Pro Ala Ile
            20                  25                  30
Leu Ser Ala Ser Pro Gly Glu Lys Val Thr Met Thr Cys Arg Ala Ser
        35                  40                  45
Ser Ser Val Ser Tyr Met His Trp Tyr Gln Gln Lys Pro Gly Ser Ser
    50                  55                  60
Pro Lys Pro Trp Ile Tyr Ala Pro Ser Asn Leu Ala Ser Gly Val Pro
65                  70                  75                  80
Ala Arg Phe Ser Gly Ser Gly Ser Gly Thr Ser Tyr Ser Leu Thr Ile
                85                  90                  95
Ser Arg Val Glu Ala Glu Asp Ala Ala Thr Tyr Tyr Cys Gln Gln Trp
            100                 105                 110
Ser Phe Asn Pro Pro Thr Phe Gly Ala Gly Thr Lys Leu Glu Leu Lys
        115                 120                 125
Asp Gly Gly Gly Ser Gly Gly Gly Gly Ser Gly Gly Gly Gly Ser Ser
    130                 135                 140
Gln Ala Tyr Leu Gln Gln Ser Gly Ala Glu Leu Val Arg Pro Gly Ala
145                 150                 155                 160
Ser Val Lys Met Ser Cys Lys Ala Ser Gly Tyr Thr Phe Thr Ser Tyr
                165                 170                 175
Asn Met His Trp Val Lys Gln Thr Pro Arg Gln Gly Leu Glu Trp Ile
            180                 185                 190
Gly Ala Ile Tyr Pro Gly Asn Gly Asp Thr Ser Tyr Asn Gln Lys Phe
        195                 200                 205
Lys Gly Lys Ala Thr Leu Thr Val Asp Lys Ser Ser Ser Thr Ala Tyr
    210                 215                 220
Met Gln Leu Ser Ser Leu Thr Ser Glu Asp Ser Ala Val Tyr Phe Cys
225                 230                 235                 240
Ala Arg Val Val Tyr Tyr Ser Asn Ser Tyr Trp Tyr Phe Asp Val Trp
                245                 250                 255
Gly Thr Gly Thr Thr Val Thr Val Ser Asp Pro Arg Arg Leu Asp Lys
            260                 265                 270

Ile Glu Asp Glu Arg Asn Leu His Glu Asp Phe Val Phe Met Lys Thr
        275                 280                 285
Ile Gln Arg Cys Asn Thr Gly Glu Arg Ser Leu Ser Leu Leu Asn Cys
    290                 295                 300
Glu Glu Ile Lys Ser Gln Phe Glu Gly Phe Val Lys Asp Ile Met Leu
305                 310                 315                 320
Asn Lys Glu Glu Thr Lys Lys Glu Asn Ser Phe Glu Met Gln Lys Gly
                325                 330                 335
Asp Gln Asn Pro Gln Ile Ala Ala His Val Ile Ser Glu Ala Ser Ser
            340                 345                 350
Lys Thr Thr Ser Val Leu Gln Trp Ala Glu Lys Gly Tyr Tyr Thr Met
        355                 360                 365
Ser Asn Asn Leu Val Thr Leu Glu Asn Gly Lys Gln Leu Thr Val Lys
    370                 375                 380
Arg Gln Gly Leu Tyr Tyr Ile Tyr Ala Gln Val Thr Phe Cys Ser Asn
385                 390                 395                 400
Arg Glu Ala Ser Ser Gln Ala Pro Phe Ile Ala Ser Leu Cys Leu Lys
                405                 410                 415
Ser Pro Gly Arg Phe Glu Arg Ile Leu Leu Arg Ala Ala Asn Thr His
            420                 425                 430
Ser Ser Ala Lys Pro Cys Gly Gln Gln Ser Ile His Leu Gly Gly Val
        435                 440                 445
Phe Glu Leu Gln Pro Gly Ala Ser Val Phe Val Asn Val Thr Asp Pro
    450                 455                 460
Ser Gln Val Ser His Gly Thr Gly Phe Thr Ser Phe Gly Leu Leu Lys
465                 470                 475                 480
Leu Glu

<210>  34
<211>  422
<212>  PRT
<213>  Artificial Sequence

<220>
<223>  MOUSE-HUMAN HYBRID FUSION PROTEIN

<220>
<221>  SITE
<222>  (1)..(266)
<223>  MOUSE ANTI-HUMAN SCFV

<220>
<221>  DOMAIN
<222>  (268)..(421)
<223>  EXTRACELLULAR DOMAIN, SHORT FORM, HUMAN CD154

<400>  34
Met Asp Phe Gln Val Gln Ile Phe Ser Phe Leu Leu Ile Ser Ala Ser
1               5                   10                  15
Val Ile Ile Ala Arg Gly Gln Ile Val Leu Ser Gln Ser Pro Ala Ile
            20                  25                  30
Leu Ser Ala Ser Pro Gly Glu Lys Val Thr Met Thr Cys Arg Ala Ser
        35                  40                  45
Ser Ser Val Ser Tyr Met His Trp Tyr Gln Gln Lys Pro Gly Ser Ser
    50                  55                  60
Pro Lys Pro Trp Ile Tyr Ala Pro Ser Asn Leu Ala Ser Gly Val Pro
65                  70                  75                  80
Ala Arg Phe Ser Gly Ser Gly Ser Gly Thr Ser Tyr Ser Leu Thr Ile
                85                  90                  95
Ser Arg Val Glu Ala Glu Asp Ala Ala Thr Tyr Tyr Cys Gln Gln Trp
            100                 105                 110

Ser Phe Asn Pro Pro Thr Phe Gly Ala Gly Thr Lys Leu Glu Leu Lys
        115                 120                 125
Asp Gly Gly Gly Ser Gly Gly Gly Gly Ser Gly Gly Gly Gly Ser Ser
    130                 135                 140
Gln Ala Tyr Leu Gln Gln Ser Gly Ala Glu Leu Val Arg Pro Gly Ala
145                 150                 155                 160
Ser Val Lys Met Ser Cys Lys Ala Ser Gly Tyr Thr Phe Thr Ser Tyr
                165                 170                 175
Asn Met His Trp Val Lys Gln Thr Pro Arg Gln Gly Leu Glu Trp Ile
            180                 185                 190
Gly Ala Ile Tyr Pro Gly Asn Gly Asp Thr Ser Tyr Asn Gln Lys Phe
        195                 200                 205
Lys Gly Lys Ala Thr Leu Thr Val Asp Lys Ser Ser Ser Thr Ala Tyr
    210                 215                 220
Met Gln Leu Ser Ser Leu Thr Ser Glu Asp Ser Ala Val Tyr Phe Cys
225                 230                 235                 240
Ala Arg Val Val Tyr Tyr Ser Asn Ser Tyr Trp Tyr Phe Asp Val Trp
                245                 250                 255
Gly Thr Gly Thr Thr Val Thr Val Ser Asp Pro Glu Asn Ser Phe Glu
            260                 265                 270
Met Gln Lys Gly Asp Gln Asn Pro Gln Ile Ala Ala His Val Ile Ser
        275                 280                 285
Glu Ala Ser Ser Lys Thr Thr Ser Val Leu Gln Trp Ala Glu Lys Gly
    290                 295                 300
Tyr Tyr Thr Met Ser Asn Asn Leu Val Thr Leu Glu Asn Gly Lys Gln
305                 310                 315                 320
Leu Thr Val Lys Arg Gln Gly Leu Tyr Tyr Ile Tyr Ala Gln Val Thr
                325                 330                 335
Phe Cys Ser Asn Arg Glu Ala Ser Ser Gln Ala Pro Phe Ile Ala Ser
            340                 345                 350
Leu Cys Leu Lys Ser Pro Gly Arg Phe Glu Arg Ile Leu Leu Arg Ala
        355                 360                 365
Ala Asn Thr His Ser Ser Ala Lys Pro Cys Gly Gln Gln Ser Ile His
    370                 375                 380
Leu Gly Gly Val Phe Glu Leu Gln Pro Gly Ala Ser Val Phe Val Asn
385                 390                 395                 400
Val Thr Asp Pro Ser Gln Val Ser His Gly Thr Gly Phe Thr Ser Phe
                405                 410                 415
Gly Leu Leu Lys Leu Glu
            420

<210>  35
<211>  63
<212>  DNA
<213>  Homo sapiens

<220>
<221>  N_region
<222>  (1)..(63)
<223>  PORTION OF HUMAN IGA HINGE DOMAIN CONTAINING ONLY 1 CYSTEINE

<400>  35
ccagttccct caactccacc taccccatct ccctcaactc cacctacccc atctccctca      60
tgc                                                                    63

<210>  36
<211>  21
<212>  PRT
<213>  Homo sapiens

<400>  36

Pro Val Pro Ser Thr Pro Pro Thr Pro Ser Pro Ser Thr Pro Pro Thr
1               5                   10                  15
Pro Ser Pro Ser Cys
            20

<210>  37
<211>  763
<212>  DNA
<213>  Homo sapiens

<220>
<221>  misc_feature
<222>  (1)..(6)
<223>  BCLI SITE FOR FUSION TO AMIN TERMINAL SCFVS

<220>
<221>  N_region
<222>  (8)..(752)
<223>  WILD TYPE IGA HINGE, CH2, CH3 DOMAINS
       TRUNCATED TO REMOVE SECRETORY COMPONENT ATTACHMENT

<400>  37
tgatcagcca gttccctcaa ctccacctac cccatctccc tcaactccac ctaccccatc      60
tccctcatgc tgccaccccc gactgtcact gcaccgaccg gccctcgagg acctgctctt     120
aggttcagaa gcgatcctca cgtgcacact gaccggcctg agagatgcct caggtgtcac     180
cttcacctgg acgccctcaa gtgggaagag cgctgttcaa ggaccacctg accgtgacct     240
ctgtggctgc tacagcgtgt ccagtgtcct gccgggctgt gccgagccat ggaaccatgg     300
gaagaccttc acttgcactg ctgcctaccc cgagtccaag accccgctaa ccgccaccct     360
ctcaaaatcc ggaaacacat tccggcccga ggtccacctg ctgccgccgc cgtcggagga     420
gctggccctg aacgagctgg tgacgctgac gtgcctggca cgtggcttca gccccaagga     480
tgtgctggtt cgctggctgc aggggtcaca ggagctgccc cgcgagaagt acctgacttg     540
ggcatcccgg caggagccca gccagggcac caccaccttc gctgtgacca gcatactgcg     600
cgtggcagcc gaggactgga agaaggggga caccttctcc tgcatggtgg gccacgaggc     660
cctgccgctg gccttcacac agaagaccat cgaccgcttg gcgggtaaac ccacccatgt     720
caatgtgtct gttgtcatgg cggaggtgga ctgataatct aga                       763

<210>  38
<211>  250
<212>  PRT
<213>  Homo sapiens

<220>
<221>  DOMAIN
<222>  (3)..(250)
<223>  TRUNCATED FORM, REMOVAL OF LAST THREE AMINO ACIDS THAT MEDIATE AT
       TACHMENT TO SECRETORY COMPONEN

<400>  38
   Asp Gln Pro Val Pro Ser Thr Pro Pro Thr Pro Ser Pro Ser Thr Pro
   1               5                   10                  15
   Pro Thr Pro Ser Pro Ser Cys Cys His Pro Arg Leu Ser Leu His Arg
               20                  25                  30
   Pro Ala Leu Glu Asp Leu Leu Leu Gly Ser Glu Ala Ile Leu Thr Cys
           35                  40                  45
   Thr Leu Thr Gly Leu Arg Asp Ala Ser Gly Val Thr Phe Thr Trp Thr
       50                  55                  60
   Pro Ser Ser Gly Lys Ser Ala Val Gln Gly Pro Pro Asp Arg Asp Leu
   65                  70                  75                  80
   Cys Gly Cys Tyr Ser Val Ser Ser Val Leu Pro Gly Cys Ala Glu Pro
                   85                  90                  95
   Trp Asn His Gly Lys Thr Phe Thr Cys Thr Ala Ala Tyr Pro Glu Ser
               100                 105                 110
   Lys Thr Pro Leu Thr Ala Thr Leu Ser Lys Ser Gly Asn Thr Phe Arg
           115                 120                 125
   Pro Glu Val His Leu Leu Pro Pro Pro Ser Glu Glu Leu Ala Leu Asn
       130                 135                 140
   Glu Leu Val Thr Leu Thr Cys Leu Ala Arg Gly Phe Ser Pro Lys Asp
   145                 150                 155                 160
   Val Leu Val Arg Trp Leu Gln Gly Ser Gln Glu Leu Pro Arg Glu Lys
                   165                 170                 175
   Tyr Leu Thr Trp Ala Ser Arg Gln Glu Pro Ser Gln Gly Thr Thr Thr
               180                 185                 190
   Phe Ala Val Thr Ser Ile Leu Arg Val Ala Ala Glu Asp Trp Lys Lys
           195                 200                 205
   Gly Asp Thr Phe Ser Cys Met Val Gly His Glu Ala Leu Pro Leu Ala
       210                 215                 220
   Phe Thr Gln Lys Thr Ile Asp Arg Leu Ala Gly Lys Pro Thr His Val
   225                 230                 235                 240
   Asn Val Ser Val Val Met Ala Glu Val Asp
                   245                 250

## Description

### BACKGROUND OF THE INVENTION

The present invention relates generally to immunologically active, recombinant binding proteins, and in particular, to molecularly engineered binding domain-immunoglobulin fusion proteins, including single chain Fv-immunoglobulin fusion proteins The present invention also relates to compositions and methods for treating malignant conditions and B-cell disorder, including diseases characterized by autoantibody production,

An immunoglobulin molecule is composed of two identical light chains and two identical heavy chains that are joined into a macromolecular complex by interchain disulfide bonds. Intrachain disulfide bonds join different areas of the same polypeptide chain, which results in the formation of loops that along with adjacent amino acids constitute the immunoglobulin domains. Each light chain and each heavy chain has a single variable region that shows considerable variation in amino acid composition from one antibody to another. The light chain variable region, V_{L}, associates with the variable region of a heavy chain, V_{H}, to form the antigen binding site of the immunoglobulin, Fv. Light chains have a single constant region domain and heavy chains have several constant region domains Classes IgG, IgA, and IgE have three constant region domains, which are designated CH1, CH2, and CH3, and the IgM and IgE classes have four constant region domains

The heavy chains of' immunoglobulins can be divided into three functional regions: Fd, hinge, and Fc. The Fd region comprises the V_{H} and CH1 domains and in combination with the light chain forms Fab, The Fc fragment is generally considered responsible for the effector functions of an immunoglobulin, such as, complement fixation and binding to Fc receptors. The hinge region, found in IgG, IgA, and IgD classes, acts as a flexible spacer, allowing the Fab portion to move freely in space.. In contrast to the constant regions, the hinge domains are structurally diverse, varying in both sequence and length among immunoglobulin classes and subclasses, For example, three human IgG subclasses, IgG1, IgG2, and IgG4, have hinge regions of' 12-15 amino acids while IgG3 comprises approximately 62 amino acids, including 21 proline residues and 11 cysteine residue. According to crystallographic studies, the hinge can be further subdivided functionally into three regions: the upper hinge, the core, and the lower hinge (Shin et al., Immunological Reviews 130:87 (1992)). The upper hinge includes amino acids from the carboxyl end of CH1 to the first residue in the hinge that restricts motion, generally the first cysteine residue that forms an interchain disulfide bond between the two heavy chains. The length of the upper hinge region correlates with the segmental flexibility of the antibody The core hinge region contains the inter-heavy chain disulfide bridges, and the lower hinge region joins the amino terminal end of the CH2 domain and includes residues in CH2 *(Id)* The core hinge region of human IgG1 contains the sequence, Cys-Pro-Pro-Cys, which when disulfide bonds are formed results in a cyclic octa peptide believed to act as a pivot, thus conferring flexibility. The hinge region may also contain carbohydrate attachment sites. For example, IgA1 contains five carbohydrate sites within a 17 amino acid segment of the hinge region, convening exception resistance of the hinge to intestinal proteases, considered an advantageous property for a secretory immunoglobulin.

Conformational changes permitted by the structure and flexibility of the hinge region may affect the effector functions of the Fc portion of the antibody. Three general categories of effector functions associated with the Fc region include (1) activation of the classical complement cascade, (2) interaction with effector cells, and (3) compartmentalization of immunoglobulins. The different human IgG subclasses vary in their relative efficacy to activate and amplify the steps of the complement cascade In general, IgG1 and IgG3 most effectively fix complement, IgG2 is less effective, and IgG4 does not activate complement Complement activation is initiated by binding of C1q, a subunit of the first component C1 in the cascade, to an antigen antibody complex. Even though the binding site for C1q is located in the CH2 domain of the antibody, the hinge regions influences the ability of the antibody to activate the cascade. For example, recombinant immunoglobulins lacking a hinge region are unable to activate complement *(Id)* Without the flexibility conferred by the hinge region, the Fab portion of' the antibody bound to the antigen may not be able to adopt the conformation required to permit C1q to bind to CH2. *(See id.)* Studies have indicated that hinge length and segmental flexibility correlate with complement activation; however, the correlation is not absolute Human IgG3 molecules with altered hinge regions that are as rigid as IgG4 still effectively activate the cascade.

Lack of the hinge region also affects the ability of' human IgG immunoglobulins to bind Fc receptors on immune effector cells. Binding of' an immunoglobulin to an Fc receptor facilitates antibody-dependent cellular cytotoxicity (ADCC), which is presumed to be an important means to eliminate tumor cells, The human IgG Fc receptor family is divided into three groups, FcγRI (CD64), which is capable of binding IgG with high affinity, FeγRII (CD32), and FcγRIII (CD16), both of which are low affinity receptors. The molecular interaction between each of the three receptors and an immunoglobulin has not been defined precisely, but experiments indicate that residues in the hinge proximal region of the CH2 domain are important to the specificity of the interaction between the antibody and the Fc receptor. In addition, IgG1 myeloma proteins and recombinant IgG3 chimeric antibodies that lack a hinge region are unable to bind FcγRI, likely because accessibility to CH2 is decreased. (Shin et al, Intern Rev Immunol 10: 177, 178-79 (1993)).

Monoclonal antibody technology and genetic engineering methods have led to rapid development of immunoglobulin molecules for diagnosis and treatment of human diseases. Protein engineering has been applied to improve the affinity of an antibody for its cognate antigen, to diminish problems related to immunogenicity, and to alter an antibody's effector functions. The domain structure of immunoglobulins is amenable to engineering, in that the antigen binding domains and the domains conferring effector functions may be exchanged between immunoglobulin classes and subclasses.

In addition, smaller immunoglobulin molecules have been constructed to overcome problems associated with whole immunoglobulin therapy. Single chain Fv (scFv) comprise the heavy chain variable domain joined via a short linker peptide to the light chain variable domain (Huston et al. Proc. Natl Acad Sci. USA, 85: 5879-83, 1988). Because of the small size of scFv molecules, they exhibit very rapid clearance from plasma and tissues and more effective penetration into tissues than whole immunoglobulin. An anti-tumor scFv showed more rapid tumor penetration and more even distribution through the tumor mass than the corresponding chimeric antibody (Yokota et al., Cancer Res. 52, 3402-08 (1992)). Fusion of an scFv to another molecule, such as a toxin, takes advantage of the specific antigen-binding activity and the small size of an scfv to deliver the toxin to a target tissue. (Chaudary et al., Nature 339:394 (1989); Batra et al., Mol. Cell Biol 11:2200 (1991))

Despite the advantages that scFv molecules bring to serotherapy, several drawbacks to this therapeutic approach exist. While rapid clearance of scFv may reduce toxic effects in normal cells, such rapid clearance may prevent delivery of' a minimum effective dose to the target tissue. Manufacturing adequate amounts of scFv for administration to patients has been challenging due to difficulties in expression and isolation of scFv that adversely affect the yield. During expression, scFv molecules lack stability and often aggregate due to pairing of variable regions from different molecules. Furthermore, production levels of scFv molecules in mammalian expression systems are low, limiting the potential for efficient manufacturing of scFv molecules for therapy (Davis et al, J. Biol Chem 265:10410-18 (1990); Traunecker et al., EMBO J 10: 3655-59 (1991)) Strategies for improving production have been explored, including addition of glycosylation sites to the variable regions (Jost, C. R. US Patent # 5,888,773, Jost et al, J. Biol. Chem 269: 26267-73 (1994)).

Conjugation or fusion of toxins to scFV provides a very potent molecule, but dosing is limited by toxicity from the toxin molecule Toxic effects include elevation of liver enzymes and vascular leak syndrome In addition, immunotoxins are highly immunogenic, and host antibodies generated against the toxin limit its potential for repeated treatment

An additional disadvantage to using scFv for therapy is the lack of effector function An scFv without the cytolytic functions, ADCC and complement dependent-cytotoxicity (CDC), associated with the constant region of an immunoglobulin may be ineffective for treating disease. Even though development of' scFv technology began over 12 years ago, currently no scFv products are approved for therapy.

The benefit of antibody constant region-associated effector functions to treatment of a disease has prompted development of fusion proteins in which nonimmunoglobulin sequences are substituted for the antibody variable region. For example, CD4, the T cell surface protein recognized by HTV, was recombinantly fused to an immunoglobulin Fc effector domain (*See* Sensel et al,, Chem Immunol 65:129-158 (1997)) The biological activity of such a molecule will depend in part on the class or subclass of the constant region chosen An IL-2-IgG1 fusion protein effected complement-mediated lysis of IL-2 receptor-bearing cells *(See id),* Use of immunoglobulin constant regions to construct these and other fusion proteins may also confer improved pharmacokinetic properties.

Diseases and disorders thought to be amenable to some type of immunoglobulin therapy include cancer and immune system disorders. Cancer includes a broad range of' diseases, affecting approximately one in four individuals worldwide, Rapid and unregulated proliferation of malignant cells is a hallmark of many types of cancel, including hematological malignancies Patients with a hematologic malignant condition have benefited most from advances in cancer therapy in the past two decades (Multani et al , J. Clin. Oncology 16: 3691-3710, 1998). Although remission rates have increased, most patients still relapse and succumb to their disease. Barriers to cure with cytotoxic drugs include tumor cell resistance and the high toxicity of chemotherapy, which prevents optimal dosing in many patients. New treatments based on targeting with molecules that specifically bind to a malignant cell, including monoclonal antibodies (mAbs), can improve effectiveness without increasing toxicity

Since mAbs were first described in 1975 (Kohler et al., Nature 256:495-97 (1975)), many patients have been treated with mAbs to antigens expressed on tumor cells. These studies have yielded important lessons regarding the selection of target antigens suitable for therapy. First and most importantly, the target antigen should not be expressed by crucial normal tissues. Fortunately, hematologic malignant cells express many antigens that are not expressed on stem cells or other essential cells. Treatment of a hematologic malignant condition that depletes both normal and malignant cells of hematological origin has been acceptable because regeneration of normal cells from progenitors occurs after therapy has ended. Second, the target antigen should be expressed on all clonogenic populations of' tumor cells, and expression should persist despite the selective pressure from immunoglobulin therapy. Thus, the choice of surface idiotype for therapy of B cell malignancy has been limited by the outgrowth of tumor cell variants with altered surface idiotype expression even though the antigen exhibits a high degree of tumor selectivity (Meeker et al.., N Engl J Med. 312:1658-65 (1985)). Third, the selected antigen must traffic properly after an immunoglobulin binds to it. Shedding or internalization of a target antigen after an immunoglobulin binds to the antigen may allow tumor cells to escape destruction, thus limiting the effectiveness of serotherapy Fourth, binding of an immunoglobulin to target antigens that transmit activation signals may result in improved functional responses in tumor cells that lead to growth arrest and apoptosis. While all of these properties are important, the triggering of apoptosis after an immunoglobulin binds to the antigen may be a critical factor in achieving successful serotherapy

Antigens that have been tested as targets for serotherapy of B and T cell malignancies include Ig idiotype (Brown et al,., Blood 73:651-61 (1989)), CD19 (Hekman et al., Cancer Immunol. Immunother 32:364-72 (1991); Vlasveld et al., Cancer Immunol Immunother 40: 37-47 (1995)), CD20 (Press et al, Blood 69: 584-91 (1987); Maloney et al., J. Clin. Oncol 15:3266-74, (1997)) CD21 (Scheinberg et al., J. Clin. Oncol. 8:792-803, (1990)), CD5 (Dillman et al, J Biol Respn Mod 5:394-410 (1986)), and CD52 (CAMPATH) (Pawson et al , J. Clin Oncol 15:2667-72, (1997)). Of these, the most success has been obtained using CD20 as a target for therapy of B cell lymphomas. Each of the other targets has been limited by the biological properties of the antigen. For example, surface idiotype can be altered through somatic mutation, allowing tumor cell escape CD5, CD21, and CD19 are rapidly internalized after mAb binding, allowing tumor cells to escape destruction unless mAbs are conjugated with toxin molecules. CD22 is expressed on only a subset of B cell lymphoma, while CD52 is expressed on both T cells and B cells and generates immunosuppression from T cell depletion.

CD20 fulfills the basic criteria described above for selection of an appropriate target antigen for therapy of' a B cell malignant condition Treatment of patients with low grade or follicular B cell lymphoma using chimeric CD20 mAb induces partial or complete responses in many patients (McLaughlin et al, Blood 88:90a (abstract, suppl. 1) (1996); Maloney et al, Blood 90: 2188-95 (1997)). However, tumor relapse commonly occurs within six months to one year. Therefore, further improvements in serotherapy are needed to induce more durable responses in low grade B cell lymphoma, and to allow effective treatment of high grade lymphoma and other B cell diseases.

One approach to improving CD20 serotherapy has been to target radioisotopes to B cell lymphomas using mAbs specific for CD20. While the effectiveness of therapy is increased, associated toxicity from the long *in vivo* half-life of the radioactive antibody increased also, sometimes requiring that the patient undergo stem cell rescue (Press et al., N. Eng J Med 329: 1219-1224, 1993; Kaminski et al., N Eng. J. Med 329:459-65 (1993)). MAbs to CD20 have been cleaved with proteases to yield F(ab')₂ or Fab fragments prior to attachment of the radioisotope, This improves penetration of the radioisotope conjugate into the tumor, and shortens *the in vivo* half-life, thus reducing the toxicity to normal tissues. However, the advantages of effector functions, including complement fixation and ADCC, that are provided by the Fc region of the CD20 mAb are lost. Therefore, for improved delivery of radioisotopes, a strategy is needed to make a CD20 mAb derivative that retains Fc-dependent effector functions but is smaller in size, thereby increasing tumor penetration and shortening mAb half-life

CD20 was the first human B cell lineage-specific surface molecule identified by a monoclonal antibody, but the function of CD20 in B cell biology is still incompletely understood, CD20 is a non-glycosylated, hydrophobic 35 kDa phosphoprotein that has both amino and carboxy ends in the cytoplasm (Einfeld et al, EMBO J. 7:711-17 (1988)). Natural ligands for CD20 have not been identified CD20 is expressed by all normal mature B cells, but is not expressed by precursor B cells

CD20 mAbs deliver signals to normal B cells that affect viability and growth (Clark et al., Proc. Natl, Acad Sci USA 83:4494-98 (1986)), Recent data has shown that extensive cross, linking of CD20 can induce apoptosis of B lymphoma cell lines (Shan et al., Blood 91:1644-52 (1998)). Cross-linking of CD20 on the cell surface increases the magnitude and kinetics of' signal transduction, which was detected by measuring phosphorylation of cellular substrates on tyrosine residues (Deans et al., J. Immunol 146:846-53 (1993)). Importantly, apoptosis of Ramos B lymphoma cells was also be induced by cross-linking of CD20 mAbs by addition of Fc-receptor positive cells (Shan et al., Blood 91: 1644-52 (1998)) Therefore, in addition to cellular depletion by complement and ADCC mechanisms, Fc-receptor binding by CD20 mAbs *in vivo* could promote apoptosis of' malignant B cells by CD20 cross-linking This theory is consistent with experiments showing that effectiveness of CD20 therapy of human lymphoma in a SCID mouse model was dependent upon Fc-receptor binding by the CD20 mAb (Funakoshi et al., J. Immunotherapy 19:93.101 (1996)).

The CD20 polypeptide contains four transmembrane domains (Einfeld et al., EMBO J. 7: 711-17, (1988); Stamenkovic et al., J. Exp. Med 167:1975-80 (1988); Tedder et. al., J. Immunol 141:4388.4394 (1988)). The multiple membrane spanning domains prevent CD20 internalization after antibody binding, This property of' CD20 was recognized as an important feature for effective therapy of' B cell malignancies when a murine CD20 mAb, 1F5, was injected into patients with B cell lymphoma, resulting in significant depletion of malignant cells and partial clinical responses (Press et al., Blood 69: 584.91 (1987)).

Because normal mature B cells also express CD20, formal B cells are depleted during CD20 antibody therapy (Reff, M.E. et al, Blood 83: 435-445, 1994), However, after treatment is completed, normal B cells are regenerated from CD20 negative B cell precursors; therefore, patients treated with anti-CD20 therapy do not experience significant immunosuppression. Depletion of normal B cells may be beneficial in diseases that involve inappropriate production of autoantibodies or other diseases where B cells may play a role A chimeric mAb specific for CD20, consisting of heavy and light chain variable regions of mouse origin fused to human IgG1 heavy chain and human kappa light chain constant regions, retained binding to CD20 and the ability to mediate ADCC and to fix complement (Liu et al., J Immunol 139:3521-26 (1987); Robinson et al., U.S. Patent No. 5,500,362) This work led to development of a chimeric CD20 mAb, Rituximab^{™}, currently approved by the U.S. Food and Drug Administration for approval for therapy of B cell lymphomas. While clinical responses are frequently observed after treatment with Rituximab, patients often relapse after about 6-12 months,

High doses of Rituximab^{™} are required for intravenous injection because the molecule is large, approximately 150 kDa, and diffusion is limited into the lymphoid tissues where many tumor cells reside, The mechanism of anti tumor activity of Rituximab^{™} is thought to be a combination of several activities, including ADCC, fixation of complement, and triggering of signals in malignant B cells that promote apoptosis. The large size of Rituximab^{™} prevents optimal diffusion of the molecule into lymphoid tissues that contain malignant B cells, thereby limiting these anti-tumor activities.. As discussed above, cleavage of CD20 mAbs with proteases into Fab or F(ab')₂ fragments makes them smaller and allows better penetration into lymphoid tissues, but the effector functions important for anti-tumor activity are lost. While CD20 mAb fragments may be more effective than intact antibody for delivery of radioisotopes, it would be desirable to construct a CD20 mAb derivative that retains the effector functions of the Fc portion, but is smaller in size, facilitating better tumor penetration and resulting in a shorter half-life,

CD20 is expressed by malignant cells of B cell origin, including B cell lymphoma and chronic lymphocytic leukemia (CLL). CD20 is not expressed by malignancies of pre-B cells, such as acute lymphoblastic leukemia. CD20 is therefore a good target for therapy of B cell lymphoma, CLL, and other diseases in which B cells are involved in the disease activity. Other B cell disorders include autoimmune diseases in which autoantibodies are produced during the differentiation of B cells into plasma cells. Examples of B cell disorders include autoimmune thyroid disease, including Graves^{t} disease and Hashimoto's thyroiditis, rheumatoid arthritis, systemic lupus erythematosus (SLE), Sjogrens syndrome, immune thrombocytopenic purpura (IIP), multiple sclerosis (MS), myasthenia gravis (MG), psoriasis, scleroderma, and inflammatory bowel disease, including Crohn's disease and ulcerative colitis.

From the foregoing, a clear need is apparent for improved compositions and methods to treat malignant conditions and B cell disorders. The compositions and methods of the present invention overcome the limitations of the prior art by providing a binding domain-immunoglobulin fusion protein comprising a binding domain polypeptide that is fused to an immunoglobulin hinge region polypeptide, which is fused to an immunoglobulin heavy chain CH2 constant region polypeptide fused to an immunoglobulin heavy chain CH3 constant region polypeptide, wherein the binding domain-immunoglobulin fusion protein is capable of mediating ADCC or complement fixation. Furthermore, the compositions and methods offer other related advantages.

### SUMMARY OF THE INVENTION

It is an aspect of the present invention to provide a binding domain-immunoglobulin fusion protein, comprising (a) a binding domain polypeptide that is fused to an immunoglobulin hinge region polypeptide, wherein said hinge region polypeptide is selected from the group consisting of (i) a mutated hinge region polypeptide that contains no cysteine residues and that is derived from a wild-type immunoglobulin hinge region polypeptide having one or more cysteine residues, (ii) a mutated hinge region polypeptide that contains one cysteine residue and that is derived from a wild-type immunoglobulin hinge region polypeptide having two or more cysteine residues, (iii) a wild-type human IgA hinge region polypeptide, (iv) a mutated human IgA hinge region polypeptide that contains no cysteine residues and that is derived from a wild-type human IgA region polypeptide, and (v) a mutated human IgA hinge region polypeptide that contains one cysteine residue and that is derived from a wild-type human IgA region polypeptide; (b) an immunoglobulin heavy chain CH2 constant region polypeptide that is fused to the hinge region polypeptide; and (c) an immunoglobulin heavy chain CH3 constant region polypeptide that is fused to the CH2 constant region polypeptide, wherein: (1) the binding domain-immunoglobulin fusion protein is capable of at least one immunological activity selected from the group consisting of antibody dependent cell-mediated cytotoxicity and complement fixation, and (2) the binding domain polypeptide is capable of specifically binding to an antigen In one embodiment the immunoglobulin hinge region polypeptide is a mutated hinge region polypeptide and exhibits a reduced ability to dimerize, relative to a wild-type human immunoglobulin G hinge region polypeptide In another embodiment the binding domain polypeptide comprises at least one immunoglobulin variable region polypeptide that is an immunoglobulin light chain variable region polypeptide or an immunoglobulin heavy chain variable region polypeptide. In a further embodiment the immunoglobulin variable region polypeptide is derived from a human immunoglobulin.

In another embodiment the binding domain Fv-immunoglobulin fusion protein binding domain polypeptide comprises (a) at least one immunoglobulin light chain variable region polypeptide; (b) at least one immunoglobulin heavy chain variable region polypeptide; and (c) at least one linker peptide that is fused to the polypeptide of (a) and to the polypeptide of (b) In a further embodiment the immunoglobulin light chain variable region and heavy chain variable region polypeptides are derived from human immunoglobulins.

In another embodiment at least one of' the immunoglobulin heavy chain CH2 constant region polypeptide and the immunoglobulin heavy chain CH3 constant region polypeptide is derived from a human immunoglobulin heavy chain In another embodiment the immunoglobulin heavy chain constant region CH2 and CH3 polypeptides are of an isotype selected from human IgG and human IgA. In another embodiment the antigen is selected from the group consisting of CD19, CD20, CD37, CD40 and L6. In certain further embodiments of the above described fusion protein, the liner polypeptide comprises at least one polypeptide having as an amino acid sequence Gly-Gly-Gly-Gly-Ser [SEQ ID NO:21], and in certain other embodiments the linker polypeptide comprises at least three repeats of a polypeptide having as an amino acid sequence Gly-Gly-Gly-Gly-Ser [SEQ ID NO:21] In certain embodiments the immunoglobulin hinge region polypeptide comprises a human IgA hinge region polypeptide. In certain embodiments the binding domain polypeptide comprises a CD154 extracellular domain. In certain embodiments the binding domain polypeptide comprise a CD154 extracellular domain and at least one immunoglobulin variable region polypeptide.

In other embodiments the invention provides an isolated polynucleotide encoding any of the above described binding domain-immunoglobulin fusion proteins, and in related embodiments the invention provides a recombinant expression construct comprising such a polynucleotide, and in certain further embodiments the invention provides a host cell transformed or transfected with suc a recombinant expression construct In another embodiment the invention provides a method of producing a binding domain immunoglobulin fusion protein, comprising the steps of (a) culturing the host cell as just described, under conditions that permit expression of' the binding domain-immunoglobulin fusion protein; and (b) isolating the binding domain-immunoglobulin fusion protein from the host cell culture.

The present invention also provides in certain embodiments a pharmaceutical composition comprising a binding domain-immunoglobulin fusion protein as described above, in combination with a physiologically acceptable carrier. In another embodiment there is provided a method of treating a subject having or suspected of' having a malignant condition or a B-cell disorder, comprising administering to a patient a therapeutically effective amount of an above described binding domain-immunoglobulin fusion protein.. In certain further embodiments the malignant condition or B-cell disorder is a B-cell lymphoma or a disease characterized by autoantibody production, and in certain other further embodiments the malignant condition or B-cell disorder is rheumatoid arthritis, myasthenia gravis, Grave's disease, type I diabetes mellitus, multiple sclerosis or an autoimmune disease.

These and other aspects of the present invention will become apparent upon reference to the following detailed description and attached drawings.. All references disclosed herein are hereby incorporated by reference in their entirety as if each was incorporated individually

### BRIEF DESCRIPTION OF THE DRAWINGS

Figure 1 shows DNA and deduced amino acid sequences [SEQ ID NOS: ] of 2H7scFv-Ig, a binding domain-immunoglobulin fusion protein capable of specifically binding CD20.
Figure 2 shows production levels of 2H7 scFv-Ig by transfected, stable CHO lines and generation of a standard curve by binding of purified 2H7 scFv-Ig to CHO cells expressing CD20.
Figure 3 shows SDS-PAGE analysis of multiple preparations of isolated 2H7scFv-Ig protein.
Figure 4 shows complement fixation (Fig. 4A) and mediation of antibody-dependent cellular cytotoxicity (ADCC, Fig. 4B)) by 2H7scFv-Ig.
Figure 5 shows the effect of simultaneous ligation of CD20 and CD40 on growth of normal B cells.
Figure 6 shows the effect of simultaneous ligation of CD20 and CD40 on CD95 expression and induction of apoptosis in a B lymphoblastoid cell line.
Figure 7 shows DNA and deduced amino acid sequences [SEQ ID NOS: ] of 2H7scFv-CD154 L2 (Fig 7A, SEQ ID NOS: ) and 2H7scFv-CD154 S4 (Fig. 7B, SEQ ID NOS: ) binding domain-immunoglobulin fusion proteins capable of specifically binding CD20 and CD40.
Figure 8 shows binding of 2H7scFv-CD154 binding domain-immunoglobulin fusion proteins to CD20+ CHO cells by flow immunocyofluorimetry,
Figure 9 shows binding of Annexin V to B cell lines Ramos, BIAB, and T51 after binding of 2H7scFv-CD154 binding domain-immunoglobulin fusion protein to cells
Figure 10 shows effects on proliferation of B cell line T51 following binding of 2H7scFv-CD154 binding domain-immunoglobulin fusion protein.

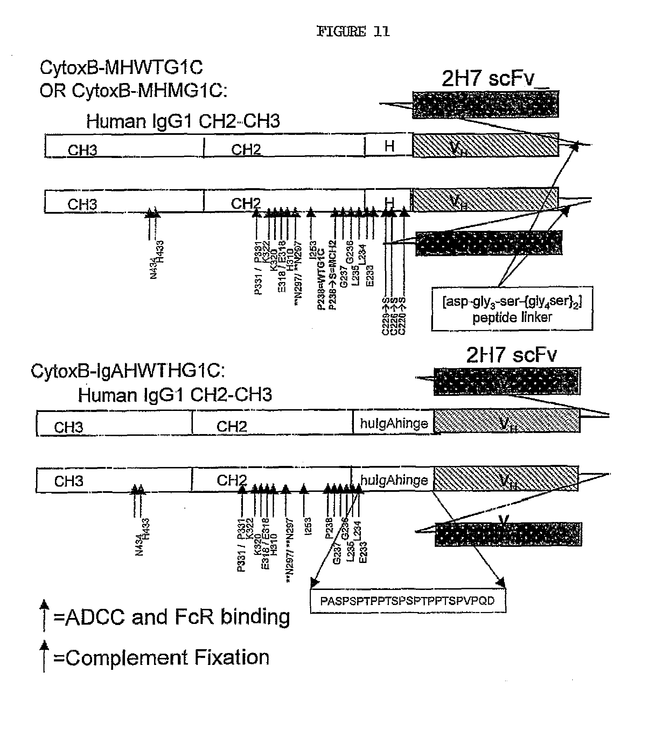

Figure 11 depicts schematic representations of the structures of 2H7ScFv-Ig fusion proteins [SEQ ID NOS: ] referred to as CytoxB or CytoxB derivatives: CytoxB-MHWTGIC (2H7 ScFv, mutant hinge, wild-type human IgG1 Fc domain), CytoxB-MHMGIC (2H7 ScFv, mutant hinge, mutated human IgG1 Fc domain) and CytoxB-IgAHWTHG1C (2H7 ScFv, human IgA-derived hinge [SEQ ID NO: ], wild-type human IgG1 Fc domain) Arrows indicate position numbers of amino acid residues believed to contribute to FcR binding and ADCC activity (heavy arrows), and to complement fixation (light arrows) Note absence of interchain disulfide bonds.
Figure 12 shows SDS-PAGE analysis of isolated CytoxB and 2H7scFv-CD154 binding domain-immunoglobulin fusion proteins
Figure 13 shows antibody dependent cell-mediated cytotoxicity (ADCC) activity of CytoxB derivatives.
Figure 14 shows complement dependent cytotoxicity (CDC) of CytoxB derivatives.
Figure 15 shows serum half-life determinations of CytoxB-MHWTG1C in macaque blood samples.
Figure 16 shows effects of CytoxB-MHWTG1C on levels of circulating CD40+ B cells in macaque blood samples
Figure 17 shows production levels of HD37 (CD19-specific) ScFv-Ig by transfected mammalian cell lines and generation of a standard curve by binding of' purified HD37 ScFv-Ig to cells expressing CD19.
Figure 18 shows production levels of L6 (carcinoma antigen) ScFv-Ig by transfected, stable CHO lines and generation of a standard curve by binding of purified L6 ScFv-Ig to cells expressing L6 antigen.
Figure 19 shows ADCC activity of binding domain-immunoglobulin fusion proteins 2H7 ScFv-Ig, HD37 ScFv-Ig and G28-1 (CD37-specific) ScFv- Ig, Figure 20 shows ADCC activity of L6 ScFv-Ig fusion protein.
Figure 21 shows SDS-PAGE analysis of L5 ScFv-Ig and 2H7 ScFv-Ig fusion proteins.
Figure 22 shows SDS-PAGE analysis of G28-1 ScFv-Ig and HD37 ScFv-Ig fusion proteins.

### DETAILED DESCRIPTION OF THE INVENIION

The present invention is directed to binding domain-immunoglobulin fusion proteins and to related compositions and methods, which will be useful in immunotherapeutic and immunodiagnostic applications, and which offer certain advantages over antigen-specific polypeptides of the prior art. The fusion proteins of the present invention are preferably single polypeptide chains that comprise, in pertinent part, the following fused domains: a binding domain polypeptide, an immunoglobulin hinge region polypeptide, an immunoglobulin heavy chain CH2. constant region polypeptide, and an immunoglobulin heavy chain CH3 constant region polypeptide. In particularly preferred embodiments, the polypeptide domains of' which the binding domain-immunoglobulin fusion protein is comprised are, or are derived from, polypeptides that are the products of human gene sequences, but the invention need not be so limited and may in fact relate to binding domain-immunoglobulin fusion proteins as provided herein that are derived from any natural or artificial source, including genetically engineered and/or mutated polypeptides.

The present invention relates in part to the surprising observation that the binding domain-immunoglobulin fusion proteins described herein are capable of immunological activity. More specifically, these proteins retain the ability to participate in well known immunological effector activities including antibody dependent cell mediated cytotoxicity (ADCC, *e.g*., subsequent to antigen binding on a cell surface, engagement and induction of cytotoxic effector cells bearing appropriate Fc receptors, such as natural killer (NK) cells bearing FcRγIII, under appropriate conditions) and/or complement fixation in complement dependent cytotoxicity (CDC, *e.g*,, subsequent to antigen binding on a cell surface, recruitment and activation of cytolytic proteins that are components of the blood complement cascade), despite having structures that would not be expected to be capable of promoting such effector activities. As described in greater detail below, ADCC and CDC are unexpected functions for monomeric proteins comprising immunoglobulin heavy chain regions, which are favored by the structures selected for the subject fusion proteins, and particularly by the selection of hinge region polypeptides that are compromised in their ability to form interchain, homodimeric disulfide bonds,

Another advantage afforded by the present invention is a binding domain-immunoglobulin fusion polypeptide that can be produced in substantial quantities that are typically greater than those routinely attained with single-chain antibody constructs of the prior art. In preferred embodiments, the binding domain-immunoglobulin fusion polypeptides of the present invention are recombinantly expressed in mammalian expression systems, which offer the advantage of providing polypeptides that are stable *in vivo* (*e.g.,* under physiological conditions). According to non-limiting theory, such stability may derive in part from posttranslational modifications, and specifically glycosylation, of the fusion proteins. Production of' the present binding domain-immunoglobulin fusion proteins via recombinant mammalian expression has been attained in static cell cultures at a level of' greater than 50 mg protein per liter culture supernatant and has been routinely observed in such cultures at 10-50 mg/l, such that preferably at least 10-50 mg/l may be produced under static culture conditions; also contemplated are enhanced production of the fusion proteins using art-accepted scale-up methodologies such as "fed batch" (*i*.*e*., non-static) production, where yields of at least 5-500 mg/l, and in some instances at least 05-1 gm/l, depending on the particular protein product, are obtained.

A binding domain polypeptide according to the present invention may be any polypeptide that possesses the ability to specifically recognize and bind to a cognate biological molecule or complex of more than one molecule or assembly or aggregate, whether stable or transient, of' such a molecule, which includes a protein, polypeptide, peptide, amino acid, or derivative thereof; a lipid, fatty acid or the like, or derivative thereof; a carbohydrate, saccharide or the like or derivative thereof, a nucleic acid, nucleotide, nucleoside, purine, pyrimidine or related molecule, or derivative thereof, or the like; or any combination thereof such as, for example, a glycoprotein, a glycopeptide, a glycolipid, a lipoprotein, a proteolipid; or any other biological molecule that may be present in a biological sample. Biological samples may be provided by obtaining a blood sample, biopsy specimen, tissue explant, organ culture, biological fluid or any other tissue or cell preparation from a subject or a biological source. The subject or biological source may be a human or non-human animal, a primary cell culture or culture adapted cell line including but not limited to genetically engineered cell lines that may contain chromosomally integrated or episomal recombinant nucleic acid sequences, immortalized or immortalizable cell lines, somatic cell hybrid cell lines, differentiated or differentiatable cell lines, transformed cell lines and the like. In certain preferred embodiments of the invention, the subject or biological source may be suspected of having or being at risk for having a malignant condition or a B-cell disorder as provided herein, which in certain further preferred embodiments may be an autoimmune disease, and in certain other preferred embodiments of' the invention the subject or biological source may be known to be free of a risk or presence of such disease.

A binding domain polypeptide may therefore be any naturally occurring or recombinantly produced binding partner for a cognate biological molecule as provided herein that is a target structure of interest, herein referred to as an "antigen" but intended according to the present disclosure to encompass any target biological molecule to which it is desirable to have the subject invention fusion protein specifically bind. Binding domain immunoglobulin fusion proteins are defined to be "immunospecific" or capable of specifically binding if they bind a desire target molecule such as an antigen as provided herein, with a Kₐ of greater than or equal to about 10⁴ M⁻¹, preferably of greater than or equal to about 10⁵ M⁻¹, more preferably of greater than or equal to about 10⁶ M⁻¹ and still more preferably of greater than or equal to about 10⁷ M⁻¹. Affinities of binding domain-immunoglobulin fusion proteins according to the present invention can be readily determined using conventional techniques, for example those described by Scatchard et al., Ann. N, Y Acad Sci 51:660 (1949) Such determination of fusion protein binding to target antigens of interest can also be performed using any of a number of known methods for identifying and obtaining proteins that specifically interact with other proteins or polypeptides, for example, a yeast two-hybrid screening system such as that described in U.S Patent No. 5,283,173 and US, Patent No. 5,468,614, or the equivalent.

Preferred embodiments of the subject invention binding domain-immunoglobulin fusion protein comprise binding domains that include at least one immunoglobulin variable region polypeptide, such as all or a portions or fragment of a heavy chain or a light chain V-region, provided it is capable of specifically binding an antigen or other desired target structure of interest as described herein In other preferred embodiments the binding domain comprises a single chain immunoglobulin-derived Fv product, which may include all or a potion of' at least one immunoglobulin light chain V-region and all or a portion of' at least one immunoglobulin heavy chain V-regions, and which further comprises a linker fused to the V-regions; preparation and testing such constructs are described in greater detail herein and are well known in the art Other binding domain polypeptides may comprise any protein or portion thereof that retains the ability to specifically bind an antigen as provided herein, including non-immunoglobulins, Accordingly the invention contemplates fusion proteins comprising binding domain polypeptides that are derived from polypeptide ligands such as hormones, cytokines, chemokines, and the like; cell surface or soluble receptors for such polypeptide ligands; lectins; intercellular adhesion receptors such as specific leukocyte integrins, selectins, immunoglobulin gene superfamily members, intercellular adhesion molecules (ICAM-1, -2, -3) and the like; histocompatibility antigens; etc

Examples of cell surface receptors that may provide a binding domain polypeptide, and that may also be selected as the target molecule or antigen to which a binding domain-Ig fusion protein of the present invention desirably binds, include the following, or the like: HER1 (*e.g*., GenBank Accession Nos U48722, SEG_HEGFREXS, KO3193) HER2 (Yoshino et al, 1994 J. Immunol. 152:2393; Disis et al., 1994 Canc Res. 54:16; see also, *e.g*., GenBank Acc. Nos. X03363, M17730, SEG_HUMHER20), HER3 (*e.g*., GenBank Acc. Nos. U29339, M34309), HER4 (Plowman et al, 1993 Nature 366:473; see also *e g.,* GenBank Acc Nos, L07868, T64105), epidermal growth factor receptor (EGFR) (*e.g.*, GenBank Acc. Nos U48722, SEG_HEGFREXS, KO3193), vascular endothelial cell growth factor (*e.g*, GenBank No. M32977), vascular endothelial cell growth factor receptor (*e.g*., GenBank Acc Nos. AF022375, 1680143, U48801, X62568), insulin-like growth factor-I (*e.g.,* GenBank Acc Nos X00173, X56774, X56773, X06043, *see also* European Patent No. GB 2241703), insulin-like growth factor-II (*e.g* , GenBank Acc. Nos.. X03562, X00910, SEG_HUMGPIA, SEG_HUMGFI2, M1786.3, M17862), transferrin receptors (Trowbridge and Omary, 1981 Proc Nat. Acad. USA 78:30.39; see also *e.g.,* GenBank Acc. Nos. X01060, M11507), estrogen receptor (*e.g*, GenBank Acc. Nos. M38651, X03635, X99101, U47678, M12674), progesterone receptor (*e.g*., GenBank Acc. Nos. X51730, X69068, M15716), follicle stimulating hormone receptor (FSH-R) (*e.g.,* GenBank Acc. Nos. Z34260, M65085), retinoic acid receptor (*e.g*., GenBank Acc. Nos. L12060, M60909, X77664, X57280, X07282, X065.38), MUC-1 (Barnes et al., 1989 Proc Nat Acad Sci. USA 86:7159; see also *e.g.,* GenBank Acc. Nos. SEG_MUSMUCIO, M65132, M64928) NY-ESQ-1 (*e.g*., GenBank Acc. Nos. AJ003149, U87459), NA 17-A (*e.g*., European Patent No. WO 96/40039), Melan-A/MART-1 (Kawakami et al., 1994 Proc Nat. Acad. Sci. USA 91:3515; see also *e.g*., GenBank Acc. Nos. U06654, U06452), tyrosinase (Topalian et al., 1994 Proc Nat Acad Sci USA 91:9461; see also *e.g*., GenBank Acc. Nos. M26729, SEG_HUMTYR0, *see also* Weber et al., J. Clin. Invest (1998) 102:1258), Gp-100 (Kawakami et al., 1994 Proc. Nat Acad Sci. USA 91:3515; see also *e.g.,* GenBank Acc. No. S73003, *see also* European Patent No., EP 668354; Adema et al , 1994 J. Biol. Chem. 269:20126), MAGE (van den Bruggen et al., 1991 Science 254:1643; see also *e.g,* GenBank Acc Nos. U93163, AF064589, U66083, D32077, D32076, D32075, U10694, U10693, U10691, U10690, U10689, U10688, U10687, U10686, U10685, L18877, U10340, U10339, L18920, U03735, M77481), BAGE (*e.g.*, GenBank Acc No. U19180, *see also* U.S. Patent Nos 5,683,886 and 5,571,711), GAGE (*e.g*., GenBank Acc. Nos AF055475, AF055474, AF055473, U19147, U19146, U19145, U19144, U19143, U19142), any of the CTA class of receptors including in particular HOM-MEL-40 antigen encoded by the SSX2 gene (*e*.*g.*, GenBank Acc. Nos. X86175, U90842, U90841, X86174), carcinoembyonic antigen (CEA, Gold and Freedman, 1985 J. Exp Med 121:439; see also *e.g*, GenBank Acc Nos. SEG_HUMCEA, M59710, M59255, M29540), and PyLT (*e.g.,* GenBank Acc Nos. J02289, J02038).

Additional cell surface receptors that may be sources of binding domain polypeptides or that may be cognate antigens include the following, or the like: CD2 (*e.g*., GenBank Acc. Nos. Y00023, SEG_HUMCD2, M16336, M16445, SEG_MUSCD2, M14362), 4-1BB (CDw137, Kwon et al., 1989 Proc. Nat. Acad. Sci. US4 86:1963, 4-1BB ligand (Goodwin et al., 1993 Eur. J Immunol. 23:2361; Melero et al., 1998 Eur. J. Immunol. 3:116), CD5 (*e.g.,* GenBank Acc. Nos, X78985, X89405), CD10 (*e.g*., GenBank Acc. Nos. M81591, X76732) CD27 (*e.g.,* GenBank Acc. Nos M63928, L24495, L08096), CD28 (June et al., 1990 Immunol. Today 11:211; see also, *e.g.,* GenBank Acc. Nos. J02988, SEG_HUMCD28, M3456.3), CTLA-4 (*e.g*., GenBank Acc. Nos. L15006, X05719, SEG_HUMIGCTL), CD40 (*e.g*., GenBank Acc. Nos. M83312, SEG_MUSC040AO, Y10507, X67878, X96710, U15637, L07414), interferon-γ (IFN-γ; see, *e.g.,* Farrar et al 1993 Ann Rev Immunol 11:571 and references cited therein, Gray et al. 1982 Nature 295:503, Rinderknecht et al. 1984 J Biol Chem 259:6790, DeGrado et al, 1982 Nature 300:379), interleukin-4 (IL-4; see, e.g.*, .*53rd Forum in Immunology, 1993 Research in Immunol 144:553-643; Banchereau et al, 1994 in The Cytokine Handbook, 2nd ed., A. Thomson, ed., Academic Press, NY, p. 99; Keegan et al., 1994 J Leukocyt. Biol, 55:272, and references cited therein), interleukin-17 (IL-17) (*e*.*g*., GenBank Acc. Nos. U32659, U43088) and interleukin-17 receptor (IL-17R) (*e*.*g*., GenBank Acc Nos. U31993, U58917). Notwithstanding the foregoing, the present invention expressly does not encompass any immunoglobulin fusion protein that is disclosed in U.S. 5,807,734, U.S. 5,795,572 or U.S. 5,807,734.

Additional cell surface receptors that may be sources of binding domain polypeptides or that may be cognate antigens include the following, or the like: CD59 (*e.g*., GenBank Acc. Nos. SEG_HUMCD590, M95708, M34671), CD48 (*e.g,* GenBank Acc. Nos. M59904), CD58/LFA-3 (*e.g*., GenBank Acc. No. A25933, Y00636, E12817; see also JP 1997075090-A) , CD72 (*e.g*, GenBank Acc. Nos AA311036, S40777, L35772), CD70 (*e.g*., GenBank Acc. Nos. Y13636, S69339), CD80/B7.1 (Freeman et al., 1989 J. Immunol. 43:2714; Freeman et al., 1991 J Exp Med 174:625; see also *e.g*., GenBank Acc. Nos. U33208, I683379), CD86/B7.2 (Freeman et al., 1993 J. Exp. Med 178:2185, Boriello et al., 1995 J. Immunol 155:5490; see also, *e.g.,* GenBank Acc. Nos. AF099105, SEG_MMB72G, U39466, U04343, SEG_HSB725, L25606, L25259), CD40 ligand (*e.g*., GenBank Acc, Nos SEG_HUMCD40L, X67878, X65453, L07414), IL-17 (*e*.*g*., GenBank Acc. Nos U32659, U43088), CD4'3 (*e.g*., GenBank Acc. Nos X52075, J04536) and VLA-4 (α₄β₇) (*e.g*, GenBank Acc. Nos. L12002, X16983, L20788, U97031, L24913, M68892, M95632). The following cell surface receptors are typically associated with B cells: CD19 (*e*.*g.*, GenBank Acc Nos. SEG_HUMCD19W0, M84371, SEG_MUSCD19W, M62542), CD20 (*e.g.*, GenBank Acc. Nos. SEG_HUMCD20, M62541), CD22 (*e.g.*, GenBank Acc. Nos. I680629, Y10210, X59350, U62631, X52782, L16928), CD30 ligand (*e.g*, GenBank Acc. Nos. L09753, M83554), CD37 (*e.g.*, GenBank Acc. Nos. SEG_MMCD37X, X14046, X53517), CD106 (VCAM-1) (*e.g.*, GenBank Acc. Nos. X53051, X67783, SEG_MMVCAM1C, *see also* U.S. Patent No. 5,596,090), CD54 (ICAM-1) (*e.g*, GenBank Acc. Nos. X84737, S82847, X06990, J03132, SEG_MUSICAM0), interleukin-12 (see, *e.g.*, Reiter et al, 1993 *Crit. Rev. Immunol.* 13:1, and references cited wherein). Accessory cell agents may also include any of the following cell surface receptors typically associated with dendritic cells: CD83 (*e.g*, GenBank Acc. Nos. AF001036, AL021918), DEC-205 (*e.g.*, GenBank Acc. Nos. AF011333, UI9271),

An immunoglobulin hinge region polypeptide, as discussed above, includes any hinge peptide or polypeptide that occurs naturally, as an artificial peptide or as the result of genetic engineering and that is situated in an immunoglobulin heavy chain polypeptide between the amino acid residues responsible for forming intrachain immunoglobulin-domain disulfide bonds in CH1 and CH2 regions; hinge region polypeptides for use in the present invention may also include a mutated hinge region polypeptide. Accordingly, an immunoglobulin hinge region polypeptide may be derived from, or may be a portion or fragment of (*i*.*e*., one or more amino acids in peptide linkage, typically 5-65 amino acids, preferably 10-50, more preferable 15-35, still more preferably 18-32, still more preferably 20-30, still more preferably 21, 22,23, 24, 25, 26, 27, 28 or 29 amino acids) an immunoglobulin polypeptide chain region classically regarded as having hinge function, as described above, but a hinge region polypeptide for use in the instant invention need not be so restricted and may include amino acids situated (according to structural criteria for assigning a particular residue to a particular domain that may vary, as known in the art) in an adjoining immunoglobulin domain such as a CH1 domain or a CH2 domain, or in the case of certain artificially engineered immunoglobulin constructs, an immunoglobulin variable region domain,

Wild-type immunoglobulin hinge region polypeptides include any naturally occurring hinge region that is located between the constant region domains, CH1 and CH2, of an immunoglobulin, The wild-type immunoglobulin hinge region polypeptide is preferably a human immunoglobulin hinge region polypeptide, preferably comprising a hinge region from a human IgG immunoglobulin, and more preferably, a hinge region polypeptide from a human IgG1 isotope. As is known to the art, despite the tremendous overall diversity in immunoglobulin amino acid sequences, immunoglobulin primary structure exhibits a high degree of sequence conservation in particular portions of immunoglobulin polypeptide chains, notably with regard to the occurrence of cysteine residues which, by virtue of then sulfyhydyl groups, offer the potential for disulfide bond formation with other available sulfydryl groups, Accordingly, in the context of the present invention wild-type immunoglobulin hinge region polypeptides may be regarded as those that feature one or more highly conserved (*e.g*., prevalent in a population in a statistically significant manner) cysteine residues, and in certain preferred embodiments a mutated hinge region polypeptide may be selected that contains zero or one cysteine residue and that is derived from such a wild-type hinge region.

A mutated immunoglobulin hinge region polypeptide may comprise a hinge region that has its origin in an immunoglobulin of a species, of an immunoglobulin isotype or class, or of an immunoglobulin subclass that is different from that of the CH2 and CH3 domains For instance, in certain embodiments of the invention, the binding domain-immunoglobulin fusion protein may comprises a binding domain polypeptide that is fused to an immunoglobulin hinge region polypeptide comprising: a wild-type human IgA hinge region polypeptide, or a mutated human IgA hinge region polypeptide that contains zero or only one cysteine residues, as described herein. Such a hinge region polypeptide may be fused to an immunoglobulin heavy chain CH2 region polypeptide Horn a different Ig isotype or class, for example an IgG subclass, which in certain preferred embodiments will be the IgG1 subclass,

For example, and as described in greater detail below, in certain embodiments of the present invention an immunoglobulin hinge region polypeptide is selected which is derived from a wild-type human IgA hinge region that naturally comprises three cysteines, where the selected hinge region polypeptide is truncated relative to the complete hinge region such that only one of the cysteine residues remains (*e.g*., SEQ ID NOS:35-36), Similarly, in certain other embodiments of the invention, the binding domam-immunoglobulm fusion protein comprises a binding domain polypeptide that is fused to an immunoglobulin hinge region polypeptide comprising a mutated hinge region polypeptide in which the number of cysteine residues is reduced by amino acid substitution or deletion. A mutated hinge region polypeptide may thus be derived from a wild type immunoglobulin hinge region that contains one or more cysteine resides. In certain embodiments, a mutated hinge region polypeptide may contain zero or only one cysteine residue, wherein the mutated hi-ngetegion polypeptide is derived from a wild type immunoglobulin hinge region that contains, respectively, one or more or two or more cysteine residues In the mutated hinge region polypeptide, the cysteine residues of' the wild-type immunoglobulin hinge region are preferably substituted with amino acids that are incapable of forming a disulfide bond. In one embodiment of the invention, the mutated hinge region polypeptide is derived from a human IgG wild-type hinge region polypeptide, which may include any of the four human IgG isotype subclasses, IgG1, IgG2, IgG3 or IgG4, In certain preferred embodiments, the mutated hinge region polypeptide is derived from a human IgG1 wild-type hinge region polypeptide, By way of example, a mutated hinge region polypeptide derived from a human IgG1 wild-type hinge region polypeptide may comprise mutations at two of the three cysteine residues in the wild-type immunoglobulin hinge region, or mutations at all three cysteine residues

The cysteine residues that are present in a wild-type immunoglobulin hinge region and that are removed by mutagenesis according to particularly preferred embodiments of the preset invention include cysteine residues that form, or that are capable of forming, interchain disulfide bonds Without wishing to be bound by theory, the present invention contemplates that mutation of such hinge regions cysteine residues, which are believed to be involved in formation of interchain disulfide bridges, reduces the ability of the subject invention binding domain-immunoglobulin fusion protein to dimerize (or form higher oligomers) via interchain disulfide bond formation, while surprisingly not ablating the ability of the fusion protein to promote antibody dependent cell-mediated cytotoxicity (ADCC) or to fix complement. In particular, the Fc receptors (FcR) which mediate ADCC (e g , FcRIII, CD16) exhibit low affinity for immunoglobulin Fc domains, suggesting that functional binding of Fc to FcR requires avidity stabilization of the Fc-FcR complex by virtue of the dimeric structure of heavy chains in a conventional antibody, and/or FcR aggregation and cross-linking by a conventional Ab Fc structure. (Sonderman et al., 2000 Nature 406:267; Radaev et al., 2001 J. Biol. Chem. 276:16469; Radaev et al., 2001 J Biol. Chem. 276:16478; Koolwijk et al., 1989 J Immunol 143:1656; Kato et al.., 2000 Immunol. Today 21:310) Hence, the binding domain-immunoglobulin fusion proteins of the present invention provide the advantages associated with single-chain immunoglobulin fusion proteins while also unexpectedly retaining immunological activity Similarly, the ability to fix complement is typically associated with immunoglobulins that are dimeric with respect to heavy chain constant regions such as those that comprise Fc, while the binding domain-immunoglobulin fusion proteins of the present invention exhibit the unexpected ability to fix complement.

As noted above, binding domain-immunoglobulin fusion proteins are believed, according to non-limiting theory, to be compromised in their ability to dimerize, and further according to theory, this property is a consequence of a reduction in the number of cysteine residues that are present in the immunoglobulin hinge region polypeptide selected for inclusion in the construction of the fusion protein. Determination of the relative ability of a polypeptide to dimerize is well within the knowledge of the relevant art, where any of a number of established methodologies may be applied to detect protein dimerization (see, *e.g*, Scopes, Protein Purification Principles and Practice, 1987 Springer-Verlag, New York). For example, biochemical separation techniques for resolving proteins on the basis of molecular size (*e.g*., gel electrophoresis, gel filtration chromatography, analytical ultracentrifugation, etc.), and/or comparison of protein physicochemical properties before and after introduction of sulfhydryl-active (*e.g*., iodoacetamide, N-ethylmaleimide) or disulfide-reducing (*e.g*., 2-mercaptoethanol, dithiothreitol) agents, or other equivalent methodologies, may all be employed for determining a degree of polypeptide dimerization or oligomerization, and for determining possible contribution of disulfide bonds to such potential quaternary structure. In certain embodiments, the invention relates to a binding domain-immunoglobulin fusion protein that exhibits a reduced (*i.e*., in a statistically significant manner relative to an appropriate IgG-derived control) ability to dimerize, relative to a wild-type human immunoglobulin G hinge region polypeptide as provided herein. Accordingly, those familiar with the art will be able readily to determine whether a particular fusion protein displays such reduced ability to dimerize,

Compositions and methods for preparation of immunoglobulin fusion proteins are well known in the art, as described for example, in U.S. Patent No 5,892,019, which discloses recombinant antibodies that are the products of' a single encoding polynucleotide but which are not binding domain-immunoglobulin fusion proteins according to the present invention.

For an immunoglobulin fusion protein of the invention which is intended for use in human, the constant regions will typically be of human sequence origin, to minimize a potential anti-human immune response and to provide appropriate effector functions. Manipulation of sequences encoding antibody constant regions is described in the PCT publication of Morrison and Oi, WO 89/07142. In particularly preferred embodiments, the CH1 domain is deleted and the carboxyl end of the binding domain, or where the binding domain comprises two immunoglobulin variable region polypeptides, the second (*i.e*., more proximal to the C-terminus) variable region is joined to the amino terminus of CH2 through the hinge region. A schematic diagram depicting the structures of two exemplary binding domain-immunoglobulin fusion proteins is shown in FIG. 11, where it should be noted that in particularly preferred embodiments no interchain disulfide bonds are present, and in other embodiments a restricted number of interchain disulfide bonds may be present relative to the number of such bonds that would be present if wild-type hinge legion polypeptides were instead present, and that in other embodiments the fusion protein comprises a mutated hinge region polypeptide that exhibits a reduced ability to dimerize, relative to a wild-type human IgG hinge region polypeptide. Thus, the isolated polynucleotide molecule codes for a single chain immunoglobulin fusion protein having a binding domain that provides specific binding affinity for a selected antigen.

As noted above, in certain embodiments the binding protein-immunoglobulin fusion protein comprises at least one immunoglobulin variable region polypeptide, which may be a light chain or a heavy chain variable region polypeptide, and in certain embodiments the fusion protein comprises at least one such light chain V-region and one such heavy chain V-region and at least one linker peptide that is fused to each of the V-regions, Construction of such binding domains, for example single chain Fv domains, is well known in the art and is described in greater detail in the Examples below, and has been described, for example, in U.S. Patent Nos. 5,892,019 and references cited therein; selection and assembly of single-chain variable regions and of linker polypeptides that may be fused to each of a heavy chain derived and a light chain-derived V region (*e.g*, to generate a binding domain that comprises a single-chain Fv polypeptide) is also known to the art and described herein and, for example, in U.S. Patent Nos 5,869,620, U.S. 4,704,692 and U.S. 4,946,778. In certain embodiments all or a portion of an immunoglobulin sequence that is derived from a non-human source may be "humanized" according to recognized procedures for generating humanized antibodies, *i.e.,* immunoglobulin sequences into which human Ig sequences are introduced to reduce the degree to which a human immune system would perceive such proteins as foreign (see, *e*.*g*., U.S. Patent Nos. 5,693,762; 5,585,089; 4,816,567; 5,225,5:33; 5,530,101; and references cited therein)

Once a binding domain-immunoglobulin fusion protein as provided herein has been designed, DNAs encoding the polypeptide may be synthesized via oligonucleotide synthesis as described, for example, in Sinha et al., Nucleic Acids Res., 12,4539-4557 (1984); assembled via PCR as described, for example in Innis, Ed., PCR Protocols, Academic Press (1990) and also in Better et al, J Biol. Chem. 267, 16712-16118 (1992); cloned and expressed via standard procedures as described, for example, in Ausubel et al., Eds., Current Protocols in Molecular Biology, John Wiley & Sons, New York (1989) and also in Robinson et al., Hum Antibod Hybridomas, 2, 84-93 (1991); and tested for specific antigen binding activity, as described, for example, in Harlow et al., Eds., Antibodies. A Laboratory Manual, Chapter 14, Cold Spring Harbor Laboratory, Cold Spring Harbor (1988) and Munson et al., Anal. Biochem., 107, 220-239 (1980).

The preparation of single polypeptide chain binding molecules of the Fv region, single-chain Fv molecules, is described in U.S. Pat. No 4,946,778, which is incorporated herein by reference" In the present invention, single-chain Fv-like molecules are synthesized by encoding a first variable region of the heavy or light chain, followed by one or more linkers to the variable region of the corresponding light or heavy chain, respectively The selection of appropriate linker(s) between the two variable regions is described in U.S. Pat. No. 4,946,778. An exemplary linker described herein is (Gly-Gly-Gly-Gly-Ser)₃. The linker is used to convert the naturally aggregate but chemically separate heavy and light chains into the amino terminal antigen binding portion of a single polypeptide chain, wherein this antigen binding portion will fold into a structure similar to the original structure made of two polypeptide chains and thus retain the ability to bind to the antigen of interest. The nucleotide sequences encoding the variable regions of the heavy and light chains, joined by a sequence encoding a linker, are joined to a nucleotide sequence encoding antibody constant regions. The constant regions are those which permit the resulting polypeptide to form interchain disulfide bonds to form a dimer, and which contain desired effector functions, such as the ability to mediate antibody-dependent cellular cytotoxicity (ADCC). For an immunoglobulin like molecule of the invention which is intended for use in humans, the constant regions will typically be substantially human to minimize a potential anti-human immune response and to provide approbate effector functions Manipulation of sequences encoding antibody constant regions is described in the PCT publication of Morisson and Oi, WO 89/07142, which is incorporated herein by reference. In preferred embodiments, the CH1 domain is deleted and the carboxyl end of the second variable region is joined to the amino terminus of CH2 through the hinge region. The Cys residue of the hinge which makes a disulfide bond with a corresponding Cys of the light chain, to hold the heavy and light chains of the native antibody molecule, can be deleted or, preferably, is substituted with, *e.g*., a Pro residue or the like.

As described above, the present invention provides recombinant expression constructs capable of' directing the expression of binding domain-immunoglobulin fusion proteins as provided herein. The amino acids, which occur in the various amino acid sequences referred to herein, are identified according to their well known three letter or one letter abbreviations The nucleotides, which occur in the various DNA sequences or fragments thereof referred herein, are designated with the standard single letter designations used routinely in the art. A given amino acid sequence may also encompass similar amino acid sequences having only minor changes, for example by way of illustration and not limitation, covalent chemical modifications, insertions, deletions and substitutions, which may further include conservative substitutions. Amino acid sequences that are similar to one another may share substantial regions of sequence homology. In like fashion, nucleotide sequences may encompass substantially similar nucleotide sequences having only minor changes, for example by way of illustration and not limitation, covalent chemical modifications, insertions, deletions and substitutions, which may further include silent mutations owing to degeneracy of the genetic code. Nucleotide sequences that are similar to one another may share substantial regions of sequence homology.

The presence of a malignant condition in a subject refers to the presence of dysplastic, cancerous and/or transformed cells in the subject, including, for example neoplastic, tumor, non-contact inhibited or oncogenically transformed cells, or the like. In preferred embodiments contemplated by the present invention, for example, such cancer cells are malignant hematopoietic cells, such as transformed cells of lymphoid lineage and in particular, B-cell lymphomas and the like; cancer cells may in certain preferred embodiments also be epithelial cells such as carcinoma cells. The invention also contemplates B-cell disorder, which may include certain malignant conditions that affect B-cells (*e g*., B-cell lymphoma) but which is not intended to be so limited, and which is also intended to encompass autoimmune diseases and in particular, diseases, disorders and conditions that are characterized by autoantibody production.

Autoantibodies are antibodies that react with self antigens, Autoantibodies are detected in several autoimmune diseases (i e, a disease, disorder or condition wherein a host immune system generates an inappropriate anti-"self" immune reaction) where they are involved in disease activity. The current treatments for these autoimmune diseases are immunosuppressive drugs that require continuing administration, lack specificity, and cause significant side effects. New approaches that can eliminate autoantibody production with minimal toxicity will address an unmet medical need for a spectrum of diseases that affect many people. The subject invention binding domain-immunoglobulin fusion protein is designed for improved penetration into lymphoid tissues.. Depletion of' B lymphocytes interrupts the autoantibody production cycle, and allows the immune system to reset as new B lymphocytes are produced from precursors in the bone marrow.

A number of diseases have been identified for which beneficial effects are believed, according to non-limiting theory, to result from B cell depletion therapy; a brief description of several exemplars of these diseases follows.

Autoimmune thyroid disease includes Graves' disease and Hashimoto's thyroiditis. In the United States alone, there are about 20 million people who have some form of autoimmune thyroid disease. Autoimmune thyroid disease results from the production of autoantibodies that either stimulate the thyroid to cause hyperthyroidism (Graves' disease) or destroy the thyroid to cause hypothyroidism (Hashimoto's thyroiditis). Stimulation of' the thyroid is caused by autoantibodies that bind and activate the thyroid stimulating hormone (TSH) receptor. Destruction of the thyroid is caused by autoantibodies that react with other thyroid antigens.

Current therapy for Graves' disease includes surgery, radioactive iodine, or antithyroid drug therapy Radioactive iodine is widely used, since antithyroid medications have significant side effects and disease recurrence is high. Surgery is reserved for patients with large goiters or where there is a need for very rapid normalization of thyroid function. There are no therapies that target the production of autoantibodies responsible for stimulating the TSH receptor. Current therapy for Hashimoto's thyroiditis is levothyroxine sodium, and therapy is usually lifelong because of the low likelihood of remission. Suppressive therapy has been shown to shrink goiters in Hashimoto's thryoiditis, but no therapies that reduce autoantibody production to target the disease mechanism are known.

Rheumatoid arthritis (RA) is a chronic disease characterized by inflamation of the joints, leading to swelling, pain, and loss of function, RA effects an estimated 2.5 million people in the United States. RA is caused by a combination of events including an initial infection or injury, an abnormal immune response, and genetic factors While autoreactive I cells and B cells are present in RA, the detection of high levels of' antibodies that collect in the joints, called rheumatoid factor, is used in the diagnosis of RA. Current therapy for RA includes many medications for managing pain and slowing the progression of the disease. No therapy has been found that can cure the disease, Medications include nonsteroidal antiinflamatory drugs (NSAIDS), and disease modifying antirheumatic drugs (DMARDS). NSAIDS are effective in benign disease, but fail to prevent the progression to joint destruction and debility in severe RA. Both NSAIDS and DMARDS are associated with signficant side effects. Only one new DMARD, Leflunomide, has been approved in over 10 years Leflunomide blocks production of' autoantibodies, reduces inflamation, and slows progression of RA. However, this drug also causes severe side effects including nausea, diarrhea, hair loss, rash, and liver injury.

Systemic Lupus Erythematosus (SLE) is an autoimmune disease caused by recurrent injuries to blood vessels in multiple organs, including the kidney, skin, and joints. SLE effects over 500,000 people in the United States. In patients with SLE, a faulty interaction between T cells and B cells results in the production of autoantibodies that attack the cell nucleus. These include anti-double stranded DNA and anti-Sm antibodies. Autoantibodies that bind phospholipids are also found in about half of SLE patients, and are responsible for blood vessel damage and low blood counts. Immune complexes accumulate the kidneys, blood vessels, and joints of SLE patients, where they cause inflamation and tissue damage No treatment for SLE has been found to cure the disease. NSAIDS and DMARDS are used for therapy depending upon the severity of the disease. Plasmapheresis with plasma exchange to remove autoantibodies can cause temporary improvement in SLE patients. There is general agreement that autoantibodies are responsible for SLE, so new therapies that deplete the B cell lineage, allowing the immune system to reset as new B cells are generated from precursors, offer hope for long lasting benefit in SLE patients.

Sjogrens syndrome is an autoimmune disease characterized by destruction of the body's moisture producing glands. Sjogrens syndrome is one of the most prevelant autoimmune disorders, striking up to 4 million people in the United States. About half of people with Sjognen's also have a connective tissue disease, such as rheumatoid arthritis, while the other half have primary Sjogren's with no other concurrent autoimmune disease. Autoantibodies, including anti-nuclear antibodies, rheumatoid factor, anti-fodrin, and anti-muscarinic receptors are often present in patients with Sjogrens syndrome, Conventional therapy includes corticosteroids.

Immune Thrombocytopenic purpura (ITP) is caused by autoantibodies that bind to blood platelets and cause their destruction. Some cases of ITP are caused by drugs, and others are associated with infection, pregnancy, or autoimmune disease such as SLE. About half of all cases are classified as "idiopathic", meaning the cause is unknown. The treatment of ITP is determine by the severity of the symptoms. In some cases, no therapy is needed. In most cases, immunosuppressive drugs, including corticosteroids or intravenous infusions of immune globulin to deplete T cells, Another treatment that usually results in an increased number of platelets is removal of the spleen, the organ that destroys antibody-coated platelets. More potent immunosuppressive drugs, including cyclosporine, cyclophosphamide, or azathioprine are used for patients with severe cases. Removal of autoantibodies by passage of patients' plasma over a Protein A column is used as a second line treatment in patients with severe disease.

Multiple Sclerosis (MS) is an autoimmune disease characterized by inflamation of the central nervous system and destruction of myelin, which insulates nerve cell fibers in the brain, spinal cord, and body. Although the cause of MS is unknown, it is widely believed that autoimmune T cells are primary contributors to the pathogenesis of the disease. However, high levels of antibodies are present in the cerebral spinal fluid of patients with MS, and some theories predict that the B cell response leading to antibody production is important for mediating the disease. No B cell depletion therapies have been studies in patients with MS. There is no cure for MS Current therapy is corticosteroids, which can reduce the duration and severity of' attacks, but do not affect the course of MS over time. New biotechnology interferon (IFN) therapies for MS have recently been approved.

Myasthenia Gravis (MG) is a chronic autoimmune neuromuscular disorder that is characterized by weakness of the voluntary muscle groups. MG effects about 40,000 people in the United States. MG is caused by autoantibodies that bind to acetylcholine preceptors expressed at neuromuscular junctions. The autoantibodies reduce or block acetylcholine receptors, preventing the transmission of signals from nerves to muscles, There is no known cure for MG. Common treatments include immunosuppression with corticosteroids, cyclosporine, cyclophosphamide, or azathioprine. Surgical removal of the thymus is often used to blunt the autoimmune response. Plasmapheresis, used to reduce autoantibody levels in the blood, is effective in MG, but is short-lived because the production of autoantibodies continues. Plasmapheresis is usually reserved for severe muscle weakness prior to surgery.

Psoriasis effects approximately five million people, Autoimmune inflamation in the skin. Psoriasis associated with arthritis in 30% (psoriatic arthritis) Many treatments, including steroids, UV light retenoids, vitamin D derivatives, cyclosporine, methotrexate.

Scleroderma is a chronic autoimmune disease of' the connective tissue that is also known as systemic sclerosis. Scleroderma is characterized by an overproduction of collagen, resulting in a thickening of the skin Approxiamtely 300,000 people in the United States have scleroderma.

Inflamatory Bowel Disease including Crohn's disease and Ulcerative colitis, are autoimmune diseases of the digestive system..

The present invention further relates to constructs encoding binding domain-immunoglobulin fusion proteins, and in particular to methods for administering recombinant constructs encoding such proteins that may be expressed, for example, as fragments, analogs and derivatives of such polypeptides. The terms "fragment" "derivative" and "analog" when referring to binding domain-immunoglobulin fusion polypeptides or fusion proteins, refers to any binding domain-immunoglobulin fusion polypeptide or fusion protein that retains essentially the same biological function or activity as such polypeptide. Thus, an analog includes a proprotein which can be activated by cleavage of the proprotein portion to produce an active binding domain-immunoglobulin fusion polypeptide.

A fragment, derivative or analog of an binding domain-immunoglobulin fusion polypeptide or fusion protein, including binding domain-immunoglobulin fusion polypeptides or fusion proteins encoded by the cDNAs referred to herein, may be (i) one in which one or more of the amino acid residues are substituted with a conserved or non-conserved amino acid residue (preferably a conserved amino acid residue) and such substituted amino acid residue may or may not be one encoded by the genetic code, or (ii) one in which one or more of the amino acid residues includes a substituent group, or (iii) one in which additional amino acids are fused to the binding domain immunoglobulin fusion polypeptide, including amino acids that are employed for detection or specific functional alteration of the binding domain-immunoglobulin fusion polypeptide or a proprotein sequence. Such fragments, derivatives and analogs are deemed to be within the scope of those skilled in the art from the teachings herein.

The polypeptides of the present invention include binding domain-immunoglobulin fusion polypeptides and fusion proteins having binding domain polypeptide amino acid sequences that are identical or similar to sequences known in the art, or fragments or portions thereof. For example by way of illustration and not limitation, the human CD154 molecule extracellular domain is contemplated for use according to the instant invention, as are polypeptides having at least 70% similarity (preferably a 70% identity) and more preferably 90% similarity (more preferably a 90% identity) to the reported polypeptide and still more preferably a 95% similarity (still more preferably a 95% identity) to the reported polypeptides and to portions of' such polypeptides, wherein such portions of a binding domain-immunoglobulin fusion polypeptide generally contain at least 30 amino acids and more preferably at least 50 amino acids.

As known in the art "similarity" between two polypeptides is determined by comparing the amino acid sequence and conserved amino acid substitutes thereto of' the polypeptide to the sequence of a second polypeptide. Fragments or portions of the nucleic acids encoding polypeptides of the present invention may be used to synthesize full-length nucleic acids of the present invention. As used herein, "% identity" refers to the percentage of identical amino acids situated at corresponding amino acid residue positions when two or more polypeptide are aligned and their sequences analyzed using a gapped BLAST algorithm (*e.g.*, Altschul et al., 1997 Nucl. Ac. Res 25:3389) which weights sequence gaps and sequence mismatches according to the default weightings provided by the National Institutes of Health/ NCBI database (Bethesda, MD; *see* www.ncbi.nhn.nih.gov/cgi-bin/BLAST/nph-newblast).

The term "isolated" means that the material is removed from its original environment (*e*.*g*., the natural environment if it is naturally occurring). For example, a naturally occurring nucleic acid or polypeptide present in a living animal is not isolated, but the same nucleic acid or polypeptide, separated from some or all of the co-existing materials in the natural system, is isolated. Such nucleic acids could be part of a vector and/or such nucleic acids or polypeptides could be part of a composition, and still be isolated in that such vector or composition is not part of its natural environment

The term "gene" means the segment of DNA involved in producing a polypeptide chain; it includes regions preceding and following the coding region "leader and trailer" as well as intervening sequences (introns) between individual coding segments (exons).

As described herein, the invention provides binding domain-immunoglobulin fusion proteins encoded by nucleic acids that have the binding domain coding sequence fused in frame to an additional immunoglobulin domain encoding sequence to provide for expression of a binding domain polypeptide sequence fused to an additional functional polypeptide sequence that permits, for example by way of illustration and not limitation, detection, functional alteration, isolation and/or purification of the fusion protein. Such fusion proteins may permit functional alteration of a binding domain by containing additional immunoglobulin-derived polypeptide sequences that influence behavior of the fusion product, for example (and as described above) by reducing the availability of sufhydryl groups for participation in disulfide bond formation, and by conferring the ability to potentiate ADCC and/or CDC.

Modification of the polypeptide may be effected by any means known to those of skill in this art. The preferred methods herein rely on modification of DNA encoding the fusion protein and expression of the modified DNA DNA encoding one of the binding domain-immunoglobulin fusions discussed above may be mutagenized using standard methodologies, including those described below. For example, cysteine residues that may otherwise facilitate multimer formation or promote particular molecular conformations can be deleted from a polypeptide or replaced, *e*.*g*., cysteine residues that are responsible for aggregate formation If necessary, the identity of cysteine residues that contribute to aggregate formation may be determined empirically, by deleting and/or replacing a cysteine residue and ascertaining whether the resulting protein aggregates in solutions containing physiologically acceptable buffers and salts. In addition, fragments of binding domain-immunoglobulin fusions may be constructed and used. As noted above, the counterreceptor/ ligand binding domains for many candidate binding domain-immunoglobulin fusion have been delineated, such that one having ordinary skill in the art may readily select appropriate polypeptide domains for inclusion in the encoded products of the instant expression constructs.

Conservative substitutions of amino acids are well-known and may be made generally without altering the biological activity of the resulting binding domain-immunoglobulin fusion protein molecule. For example, such substitutions are generally made by interchanging within the groups of polar residues, charged residues, hydrophobic residues, small residues, and the like. If necessary, such substitutions may be determined empirically merely by testing the resulting modified protein for the ability to bind to the appropriate cell surface receptors in *in vitro* biological assays, or to bind to appropriate antigens or desired target molecules.

The present invention further relates to nucleic acids which hybridize to binding domain-immunoglobulin fusion protein encoding polynucleotide sequences as provided herein, or their complements, as will be readily apparent to those familiar with the art, if there is at least 70%, preferably at least 90%, and more preferably at least 95% identity between the sequences. The present invention particularly relates to nucleic acids which hybridize under stringent conditions to the binding domain-immunoglobulin fusion encoding nucleic acids referred to herein. As used herein, the term "stringent conditions" means hybridization will occur only if there is at least 95% and preferably at least 97% identity between the sequences. The nucleic acids which hybridize to binding domain-immunoglobulin fusion encoding nucleic acids referred to herein, in preferred embodiments, encode polypeptides which retain substantially the same biological function or activity as the binding domain-immunoglobulin fusion polypeptides encoded by the cDNAs of the references cited herein.

As used herein, to "hybridize" under conditions of a specified stringency is used to describe the stability of hybrids formed between two single-stranded nucleic acid molecules Stringency of hybridization is typically expressed in conditions of ionic strength and temperature at which such hybrids are annealed and washed. Typically "high", "medium" and "low" stringency encompass the following conditions or equivalent conditions thereto: high stringency: 0.1 x SSPE or SSC, 0.1% SDS, 65° C; medium stringency: 0.2 x SSPE or SSC, 0.1% SDS, 50°C; and low stringency: 1.0 x SSPE or SSC, 0.1% SDS, 50°C, As known to those having ordinary skill in the art, variations in stringency of hybridization conditions may be achieved by altering the time, temperature and/or concentration of the solutions used for prehybridization, hybridization and wash steps, and suitable conditions may also depend in part on the particular nucleotide sequences of the probe used, and of' the blotted, proband nucleic acid sample. Accordingly, it will be appreciated that suitably stringent conditions can be readily selected without undue experimentation where a desired selectivity of the probe is identified, based on its ability to hybridize to one or more certain proband sequences while not hybridizing to certain other proband sequences.

The nucleic acids of the present invention, also referred to herein as polynucleotides, may be in the form of RNA or in the form of DNA, which DNA includes cDNA, genomic DNA, and synthetic DNA. The DNA may be double-stranded or single-stranded, and if single stranded may be the coding strand or non-coding (anti-sense) strand. A coding sequence which encodes an binding domain-immunoglobulin fusion polypeptide for use according to the invention may be identical to the coding sequence known in the art for any given binding domain-immunoglobulin fusion, or may be a different coding sequence, which, as a result of the redundancy or degeneracy of the genetic code, encodes the same binding domain-immunoglobulin fusion polypeptide.

The nucleic acids which encode binding domain-immunoglobulin fusion polypeptides for use according to the invention may include, but are not limited to: only the coding sequence for the binding domain-immunoglobulin fusion polypeptide; the coding sequence for the binding domain-immunoglobulin fusion polypeptide and additional coding sequence; the coding sequence for the binding domain-immunoglobulin fusion polypeptide (and optionally additional coding sequence) and non-coding sequence, such as introns or non-coding sequences 5' and/or 3' of the coding sequence for the binding domain-immunoglobulin fusion polypeptide, which for example may further include but need not be limited to one or more regulatory nucleic acid sequences that may be a regulated or regulatable promoter, enhancer, other transcription regulatory sequence, repressor binding sequence, translation regulatory sequence or any other regulatory nucleic acid sequence. Thus, the term "nucleic acid encoding" or "polynucleotide encoding" a binding domain-immunoglobulin fusion protein encompasses a nucleic acid which includes only coding sequence for a binding domain-immunoglobulin fusion polypeptide as well as a nucleic acid which includes additional coding and/or non-coding sequence(s).

Nucleic acids and oligonucleotides for use as described herein can be synthesized by any method known to those of skill in this art (*see, e.g.,* WO 93/01286, U.S. Application Serial No. 07/723,454; U.S. Patent No. 5,218,088; U.S. Patent No. 5,175,269; U.S. Patent No. 5,109,124). Identification of oligonucleotides and nucleic acid sequences for use in the present invention involves methods well known in the art. For example, the desirable properties, lengths and other characteristics of useful oligonucleotides are well known. In certain embodiments, synthetic oligonucleotides and nucleic acid sequences may be designed that resist degradation by endogenous host cell nucleolytic enzymes by containing such linkages as: phosphorothioate, methylphosphonate, sulfone, sulfate, ketyl, phosphorodithioate, phosphoramidate, phosphate esters, and other such linkages that have proven useful in antisense applications (*see, e.g,* Agrwal et al., Tetrehedron Lett 28:3539-3542 (1987); Miller et al., J. Am. Chem Soc 93:6657-6665 (1971); Stec et al., Tetrehedron Lett 26:2191-2194 (1985); Moody et al.., Nucl. Acids Res. 12:4769-4782 (1989); Uznanski et al., Nucl. Acids Res. (1989); Letsinger et al., Tetrahedron 40:137.143 (1984); Eckstein, Annu. Rev Biochem 54:367-402 (1985); Eckstein, Trends Biol. Sci. 14:97-100 (1989); Stein In: Oligodeoxynucleotides Antisense Inhibitors of Gene Expression, Cohen, Ed, Macmillan Press, London, pp. 97-117 (1989); Jager et al., Biochemistry 27:7237-7246 (1988)).

In one embodiment, the present invention provides truncated components (*e*.*g*., binding domain polypeptide, hinge region polypeptide, linker, etc.) for use in a binding domain-immunoglobulin fusion protein, and in another embodiment the invention provides nucleic acids encoding a binding domain-immunoglobulin fusion protein having such truncated components. A truncated molecule may be any molecule that comprises less than a full length version of the molecule. Truncated molecules provided by the present invention may include truncated biological polymers, and in preferred embodiments of the invention such truncated molecules may be truncated nucleic acid molecules or truncated polypeptides. Truncated nucleic acid molecules have less than the full length nucleotide sequence of a known or described nucleic acid molecule, where such a known or described nucleic acid molecule may be a naturally occurring, a synthetic or a recombinant nucleic acid molecule, so long as one skilled in the art would regard it as a full length molecule Thus, for example, truncated nucleic acid molecules that correspond to a gene sequence contain less than the full length gene where the gene comprises coding and non-coding sequences, promoters, enhancers and other regulatory sequences, flanking sequences and the like, and other functional and non-functional sequences that are recognized as part of the gene. In another example, truncated nucleic acid molecules that correspond to a mRNA sequence contain less than the full length mRNA transcript, which may include various translated and non-translated regions as well as other functional and non-functional sequences.

In other preferred embodiments, truncated molecules are polypeptides that comprise less than the full length amino acid sequence of a particular protein or polypeptide component. As used herein "deletion" has its common meaning as understood by those familiar with the art, and may refer to molecules that lack one or more of a portion of' a sequence from either terminus or from a non-terminal region, relative to a corresponding full length molecule, for example, as in the case of truncated molecules provided herein. Truncated molecules that are linear biological polymers such as nucleic acid molecules or polypeptides may have one or more of a deletion from either terminus of the molecule or a deletion from a non-terminal region of' the molecule, where such deletions may be deletions of 1-1500 contiguous nucleotide or amino acid residues, preferably 1-500 contiguous nucleotide or amino acid residues and more preferably 1-300 contiguous nucleotide or amino acid residues. In certain particularly preferred embodiments truncated nucleic acid molecules may have a deletion of 270-330 contiguous nucleotides. In certain other particularly preferred embodiments truncated polypeptide molecules may have a deletion of 80-140 contiguous amino acids.

The present invention further relates to variants of the herein referenced nucleic acids which encode fragments, analogs and/or derivatives of a binding domain-immunoglobulin fusion polypeptide. The variants of the nucleic acids encoding binding domain-immunoglobulin fusion may be naturally occurring allelic variants of the nucleic acids or non-naturally occurring variants. As is known in the art, an allelic variants is an alternate form of a nucleic acid sequence which may have at least one of a substitution, a deletion or an addition of one or more nucleotides, any of which does not substantially alter the function of the encoded binding domain-immunoglobulin fusion polypeptide.

Variants and derivatives of binding domain-immunoglobulin fusion may be obtained by mutations of nucleotide sequences encoding binding domain-immunoglobulin fusion polypeptides Alterations of the native amino acid sequence may be accomplished by any of' a number of conventional methods. Mutations can be introduced at particular loci by synthesizing oligonucleotides containing a mutant sequence, flanked by restriction sites enabling ligation to fragments of the native sequence. Following ligation, the resulting reconstructed sequence encodes an analog having the desired amino acid insertion, substitution, or deletion.

Alternatively, oligonucleotide-directed site-specific mutagenesis procedures can be employed to provide an altered gene wherein predetermined codons can be altered by substitution, deletion or insertion. Exemplary methods of making such alterations are disclosed by Walder et al. (Gene 42:113, 1986); Bauer et al. (Gene 37:73, 1985); Craik (BioTechniques, January 1985, 12-19); Smith et al. (Genetic Engineering: Principles and Methods BioTechniques, January 1985, 12-19); Smith et al. (Genetic Engineering Principles and Methods, Plenum Press, 1981); Kunkel (Proc. Natl. Acad. Sci. USA 82:488, 1985); Kunkel et al. (Methods in Enzymol. 154:367, 1987); and U.S. Patent Nos. 4,518,584 and 4,737,462.

As an example, modification of DNA may be performed by site-directed mutagenesis of DNA encoding the protein combined with the use of DNA amplification methods using primers to introduce and amplify alterations in the DNA template, such as PCR splicing by overlap extension (SOE) Site-directed mutagenesis is typically effected using a phage vector that has single- and double-stranded forms, such as M13 phage vectors, which are well-known and commercially available. Other suitable vectors that contain a single-stranded phage origin of replication may be used (*see, e.g.,* Veira et al., *Meth Enzymol. 15*:3, 1987). In general, site directed mutagenesis is performed by preparing a single-stranded vector that encodes the protein of interest (*e.g*., all or a component portion of a given binding domain-immunoglobulin fusion protein). An oligonucleotide primer that contains the desired mutation within a region of homology to the DNA in the single-stranded vector is annealed to the vector followed by addition of a DNA polymerase, such as *E*. *coli* DNA polymerase I (Klenow fragment), which uses the double stranded region as a primer to produce a heteroduplex in which one strand encodes the altered sequence and the other the original sequence. The heteroduplex is introduced into appropriate bacterial cells and clones that include the desired mutation are selected. The resulting altered DNA molecules may be expressed recombinantly in appropriate host cells to produce the modified protein.

Equivalent DNA constructs that encode various additions or substitutions of amino acid residues or sequences, or deletions of terminal or internal residues or sequences not needed for biological activity are also encompassed by the invention. For example, and as discussed above, sequences encoding Cys residues that are not desirable or essential for biological activity can be altered to cause the Cys residues to be deleted or replaced with other amino acids, preventing formation of incorrect intramolecular disulfide bridges upon renaturation.

Host organisms include those organisms in which recombinant production of binding domain-immunoglobulin fusion products encoded by the recombinant constructs of' the present invention may occur, such as bacteria (for example, *E. coli*), yeast (for example, *Saccharomyces cerevisiae* and *Pichia pastoris*), insect cells and mammals, including *in vitro* and *in* vivo expression. Host organisms thus may include organisms for the construction, propagation, expression or other steps in the production of the vaccines provided herein; hosts also include subjects in which immune responses take place, as described above. Presently preferred host organisms are *E coli* bacterial strains, inbred murine strains and murine cell lines, and human cells, subjects and cell lines,

The DNA construct encoding the desired binding domain-immunoglobulin fusion is introduced into a plasmid for expression in an appropriate host. In preferred embodiments, the host is a bacterial host. The sequence encoding the ligand or nucleic acid binding domain is preferably codon-optimized for expression in the particular host. Thus, for example, if a human binding domain-immunoglobulin fusion is expressed in bacteria, the codons would be optimized for bacterial usage. For small coding region, the gene can be synthesized as a single oligonucleotide. For larger proteins, splicing of multiple oligonucleotides, mutagenesis, or other techniques known to those in the art may be used. The sequences of' nucleotides in the plasmids that are regulatory regions, such as promoters and operators, are operationally associated with one another for transcription. The sequence of nucleotides encoding a binding domain-immunoglobulin fusion protein may also include DNA encoding a secretion signal, whereby the resulting peptide is a precursor protein The resulting processed protein may be recovered from the periplasmic space or the fermentation medium.

In preferred embodiments, the DNA plasmids also include a transcription terminator sequence As used herein, a "transcription terminator region" is a sequence that signals transcription termination. The entire transcription terminator may be obtained from a protein encoding gene, which may be the same or different from the inserted binding domain-immunoglobulin fusion encoding gene or the source of the promoter. Transcription terminators are optional components of the expression systems herein, but are employed in preferred embodiments.

The plasmids used herein include a promoter in operative association with the DNA encoding the protein or polypeptide of interest and are designed for expression of proteins in a suitable host as described above (e *g.,* bacterial, murine or human) depending upon the desired use of the plasmid (*e.g.,* administration of a vaccine containing binding domam-immunoglobulin fusion encoding sequence Suitable promoters for expression of proteins and polypeptides herein are widely available and are well known in the art. Inducible promoters or constitutive promoters that are linked to regulatory regions are preferred. Such promoters include, but are not limited to, the T7 phage promoter and other T7-like phage promoters, such as the T3, T5 and SP6 promoters, the trp, lpp, and lac promoters, such as the lacUV5, from *E*. *coli*; the P10 or polyhedrin gene promoter of baculovirus/insect cell expression systems (*see, e.g.,* U.S. Patent Nos.. 5,243,041, 5,242,687, 5,266,317, 4,745,051, and 5,169,784) and inducible promoters from other eukaryotic expression systems. For expression of the proteins such promoters are inserted in a plasmid in operative linkage with a control region such as the lac operon.

Preferred promoter regions are those that are inducible and functional in *E coli.* Examples of suitable inducible promoters and promoter regions include, but are not limited to: the *E. coli* lac operator responsive to isopropyl β -D-thiogalactopyranoside (IPTG; *see* Nakamura et al., Cell 18:1109-1117, 1979); the metallothionein promoter metal-regulatory-elements responsive to heavy-metal (*e.g*., zinc) induction (*see, e.g.,* U.S. Patent No. 4,870,009 to Evans et al.); the phage T7lac promoter responsive to IPTG (*see, e.g.,* U.S. Patent No. 4,952,496; and Studier et al., Meth Enzymol. 185:60-89, 1990) and the TAC promoter.

The plasmids may optionally include a selectable marker gene or genes that are functional in the host. A selectable marker gene includes any gene that confers a phenotype on bacteria that allows transformed bacterial cells to be identified and selectively grown from among a vast majority of untransformed cells. Suitable selectable marker genes for bacterial hosts, for example, include the ampicillin resistance gene (Amp^{r}), tetracycline resistance gene (Tc^{r}) and the kanamycin resistance gene (Kan^{r}). The kanamycin resistance gene is presently preferred.

The plasmids may also include DNA encoding a signal for secretion of the operably linked protein. Secretion signals suitable for use are widely available and are well known in the art. Prokaryotic and eukaryotic secretion signals functional in E *coli* may be employed. The presently preferred secretion signals include, but are not limited to, those encoded by the following *E. coli* genes: ompA, ompT, ompF, ompC, beta-lactamase, and alkaline phosphatase, and the like (von Heijne, J. Mol Biol. 184:99-105, 1985). In addition, the bacterial pelB gene secretion signal (Lei et al., J Bacteriol 169:4379, 1987), the phoA secretion signal, and the cek2 functional in insect cell may be employed.. The most preferred secretion signal is the *E*. *coli* ompA secretion signal.. Other prokaryotic and eukaryotic secretion signals known to those of' skill in the art may also be employed (*see, e.g.,* von Heijne, J. Mol. Biol. 184:99-105, 1985). Using the methods described herein, one of skill in the art can substitute secretion signals that are functional in either yeast, insect or mammalian cells to secrete proteins from those cells.

Preferred plasmids for transformation of *E*. *coli* cells include the pET expression vectors (*e*.*g*., pEI-11a, pET-12ac, pEI-15b; *see* U.S. Patent No. 4,952,496; available from Novagen, Madison, WI.). Other preferred plasmids include the pKK plasmids, particularly pKK 223-3, which contains the tac promoter (Brosius et al., Proc. Natl. Acad. Sci. 81:6929, 1984; Ausubel et al., Current Protocols in Molecular Biology; U.S. Patent Nos. 5,122,463, 5,173,403, 5,187,153, 5,204,254, 5,212,058, 5,212,286, 5,215,907, 5,220,013, 5,223,483, and 5,229,279). Plasmid pKK has been modified by replacement of the ampicillin resistance gene with a kanamycin resistance gene. (Available from Pharmacia; obtained from pUC4K, *see, e.g.,* Vieira et al. (Gene 19:259-268, 1982; and U.S. Patent No. 4,719,179.) Baculovirus vectors, such as pBlueBac (also called pJVETL and derivatives thereof), particularly pBlueBac III (*see, e.g*., U.S. Patent Nos. 5,278,050, 5,244,805, 5,243,041, 5,242,687, 5,266,317, 4,745,051, and 5,169,784; available from Invitrogen, San Diego) may also be used for expression of the polypeptides in insect cells. Other plasmids include the pIN-IIIompA plasmids (*see* U.S. Patent No 4,575,013; *see also* Duffaud et al., Meth. Enz. 153:492-507, 1987), such as pIN-IIIompA2.

Preferably, the DNA molecule is replicated in bacterial cells, preferably in *E. coli.* The preferred DNA molecule also includes a bacterial origin of replication, to ensure the maintenance of' the DNA molecule from generation to generation of' the bacteria In this way, large quantities of the DNA molecule can be produced by replication in bacteria. Preferred bacterial origins of replication include, but are not limited to, the f1-ori and col E1 origins of replication. Preferred hosts contain chromosomal copies of DNA encoding T7 RNA polymerase operably linked to an inducible promoter, such as the lacUV promoter (*see* U.S. Patent No. 4,952,496). Such hosts include, but are not limited to, lysogens *E coli* strains HMS174(DE3)pLysS, BL21(DE3)pLysS, HMS174(DE3) and BL21(DE3). Strain BL21(DE3) is preferred. The pLys strains provide low levels of' T7 lysozyme, a natural inhibitor of 17 RNA polymerase.

The DNA molecules provided may also contain a gene coding for a repressor protein. The repressor protein is capable of repressing the transcription of a promoter that contains sequences of nucleotides to which the repressor protein binds. The promoter can be derepressed by altering the physiological conditions of the cell. For example, the alteration can be accomplished by adding to the growth medium a molecule that inhibits the ability to interact with the operator or with regulatory proteins or other regions of the DNA or by altering the temperature of the growth media. Preferred represses proteins include, but are not limited to the *E. coli* lacI repressor responsive to IPTG induction, the temperature sensitive λ cI857 repressor, and the like, The *E*. *coli* lacI repressor is preferred

In general, recombinant constructs of' the subject invention will also contain elements necessary for transcription and translation. In particular, such elements are preferred where the recombinant expression construct containing nucleic acid sequences encoding binding domain-immunoglobulin fusion proteins is intended for expression in a host cell or organism. In certain embodiments of the present invention, cell type preferred or cell type specific expression of' a cell binding domain-immunoglobulin fusion encoding gene may be achieved by placing the gene under regulation of a promoter. The choice of the promoter will depend upon the cell type to be transformed and the degree or type of control desired. Promoters can be constitutive or active and may further be cell type specific, tissue specific, individual cell specific, event specific, temporally specific or inducible. Cell-type specific promoters and event type specific promoters are preferred Examples of constitutive or nonspecific promoters include the SV40 early promoter (U.S. Patent No. 5,118,627), the SV40 late promoter (U.S. Patent No. 5,118,627), CMV early gene promoter (U.S. Patent No. 5,168,062), and adenovirus promoter. In addition to vital promoters, cellular promoters are also amenable within the context of this invention. In particular, cellular promoters for the so-called housekeeping genes are useful. Viral promoters are preferred, because generally they are stronger promoters than cellular promoters. Promoter regions have been identified in the genes of many eukaryotes including higher eukaryotes, such that suitable promoters for use in a particular host can be readily selected by those skilled in the art.

Inducible promoters may also be used These promoters include MMTV LTR (PCI WO 91/13160), inducible by dexamethasone; metallothionein promoter, inducible by heavy metals; and promoters with cAMP response elements, inducible by cAMP. By using an inducible promoter, the nucleic acid sequence encoding a binding domain-immunoglobulin fusion protein may be delivered to a cell by the subject invention expression construct and will remain quiescent until the addition of the inducer. This allows further control on the timing of production of the gene product

Event-type specific promoters are active or up-regulated only upon the occurrence of an event, such as tumorigenicity or viral infection. The HIV LTR is a well known example of an event-specific promoter. The promoter is inactive unless the *tat* gene product is present, which occurs upon viral infection. Some event-type promoters are also tissue-specific

Additionally, promoters that are coordinately regulated with a particular cellular gene may be used.. For example, promoters of' genes that are coordinately expressed may be used when expression of a particular binding domain-immunoglobulin fusion protein-encoding gene is desired in concert with expression of one or more additional endogenous or exogenously introduced genes. This type of promoter is especially useful when one knows the pattern of gene expression relevant to induction of an immune response in a particular tissue of the immune system, so that specific immunocompetent cells within that tissue may be activated or otherwise recruited to participate in the immune response.

In addition to the promoter, repressor sequences, negative regulators, or tissue-specific silencers may be inserted to reduce non-specific expression of binding domain-immunoglobulin fusion protein encoding genes in certain situations, such as, for example, a host that is transiently immunocompromised as part of a therapeutic strategy. Multiple represser elements may be inserted in the promoter region. Repression of transcription is independent on the orientation of represser elements or distance from the promote One type of repressor sequence is an insulator sequence. Such sequences inhibit transcription (Dunaway et al., Mol Cell Biol 17: 182-9, 1997; Gdula et al., Proc Natl Acad Sci USA 93:9378-83, 1996, Chan et al., J Virol 70: 5312-28, 1996; Scott and Geyer, EMBO J 14:6258-67, 1995; Kalos and Fournier, Mol Cell Biol 15:198-207, 1995; Chung et al., Cell 74: 505-14, 1993) and will silence background transcription.

Repressor elements have also been identified in the promoter regions of the genes for type II (cartilage) collagen, choline acetyltransferase, albumin (Hu et al., J Cell Growth Differ 3(9):577-588, 1992), phosphoglycerate kinase (PGK-2) (Misuno et al., Gene 119(2):293-297, 1992), and in the 6-phosphofructo-2-kinase/fructose.2, 6-bisphosphatase gene. (Lemaigre et al., Mol. Cell Biol 11(2):1099-1106.) Furthermore, the negative regulator element Tse-1 has been identified in a number of liver specific genes, and has been shown to block cAMP response element- (CRE) mediated induction of gene activation in hepatocytes. (Boshart et al., Cell 61(5):905-916, 1990).

In preferred embodiments, elements that increase the expression of the desired product are incorporated into the construct. Such elements include internal ribosome binding sites (IRES; Wang and Siddiqui, Curr. Top. Microbial Immunol 203:99, 1995; Ehrenfeld and Semler, Curr. Top Microbial Immunol. 203:65, 1995; Rees et al., Biotechniques 20:102, 1996; Sugimoto et al., Biotechnology 12:694, 1994). IRES increase translation efficiency. As well, other sequences may enhance expression. For some genes, sequences especially at the 5' end inhibit transcription and/or translation. These sequences are usually palindromes that can form hairpin structures. Any such sequences in the nucleic acid to be delivered are generally deleted Expression levels of' the transcript or translated product are assayed to confirm or ascertain which sequences affect expression. Transcript levels may be assayed by any known method, including Northern blot hybridization, RNase probe protection and the like. Protein levels may be assayed by any known method, including ELISA, western blot, immunocytochemistry or other well known technique.

Other elements may be incorporated into the binding domain-immunoglobulin fusion protein encoding constructs of the present invention. In preferred embodiments, the construct includes a transcription terminator sequence, including a polyadenylation sequence, splice donor and acceptor sites, and an enhancer. Other elements useful for expression and maintenance of the construct in mammalian cells or other eukaryotic cells may also be incorporated (*e.g*., origin of replication) Because the constructs are conveniently produced in bacterial cells, elements that are necessary for, or that enhance, propagation in bacteria are incorporated. Such elements include an origin of replication, a selectable marker and the like

As provided herein, an additional level of controlling the expression of nucleic acids encoding binding domain-immunoglobulin fusion proteins delivered to cells using the constructs of' the invention may be provided by simultaneously delivering two or more differentially regulated nucleic acid constructs. The use of such a multiple nucleic acid construct approach may permit coordinated regulation of an immune response such as, for example, spatiotemporal coordination that depends on the cell type and/or presence of another expressed encoded component. Those familiar with the art will appreciate that multiple levels of regulated gene expression may be achieved in a similar manner by selection of suitable regulatory sequences, including but not limited to promoters, enhancers and other well known gene regulators elements.

The present invention also relates to vectors, and to constructs prepared from known vectors that include nucleic acids of the present invention, and in particular to "recombinant expression constructs" that include any nucleic acids encoding binding domain-immunoglobulin fusion proteins and polypeptides according to the invention as provided above; to host cells which are genetically engineered with vectors and/or constructs of the invention and to methods of' administering expression constructs comprising nucleic acid sequences encoding such binding domain-immunoglobulin fusion polypeptides and fusion proteins of the invention, or fragments or variants thereof, by recombinant techniques. Binding domain-immunoglobulin fusion proteins can be expressed in virtually any host cell under the control of appropriate promoters, depending on the nature of the construct (e g, type of promoter, as described above), and on the nature of' the desired host cell (*e.g*., whether postmitotic terminally differentiated or actively dividing; *e.g*., whether the expression construct occurs in host cell as an episome or is integrated into host cell genome). Appropriate cloning and expression vectors for use with prokaryotic and eukaryotic hosts are described by Sambrook, et al., Molecular Cloning. A Laboratory Manual, Second Edition, Cold Spring Harbor, NY, (1989); as noted above, in particularly preferred embodiments of the invention, recombinant expression is conducted in mammalian cells that have been transfected or transformed with the subject invention recombinant expression construct

Typically, the constructs are derived from plasmid vectors. A preferred construct is a modified pNASS vector (Clontech, Palo Alto, CA), which has nucleic acid sequences encoding an ampicillin resistance gene, a polyadenylation signal and a T7 promoter site. Other suitable mammalian expression vectors are well known *(see, e g.,* Ausubel et al., 1995; Sambrook et al., *supra; see also, e g,* catalogues from Invitrogen, San Diego, CA; Novagen, Madison, WI; Pharmacia, Piscataway, NJ; and others) Presently preferred constructs may be prepared that include a dihydrofolate reductase (DHFR) encoding sequence under suitable regulatory control, for promoting enhanced production levels of the binding domain-immunoglobulin fusion protei, which levels result from gene amplification following application of an appropriate selection agent (*e.g*., methetrexate).

Generally, recombinant expression vectors will include origins of replication and selectable markers permitting transformation of the host cell, and a promoter derived from a highly-expressed gene to direct transcription of' a downstream structural sequence, as described above. The heterologous structural sequence is assembled in appropriate phase with translation initiation and termination sequences. Thus, for example, the binding domain-immunoglobulin fusion protein encoding nucleic acids as provided herein may be included in any one of a variety of expression vector constructs as a recombinant expression construct for expressing a binding domain-immunoglobulin fusion polypeptide in a host cell. In certain preferred embodiments the constructs are included in formulations that are administered in vivo. Such vectors and constructs include chromosomal, nonchromosomal and synthetic DNA sequences, *e.g*., derivatives of SV40; bacterial plasmids; phage DNA; yeast plasmids; vectors derived from combinations of plasmids and phage DNA, viral DNA, such as vaccinia, adenovirus, fowl pox virus, and pseudorabies, or replication deficient retroviruses as described below. However, any other vector may be used for preparation of a recombinant expression construct, and in referred embodiments such a vector will be replicable and viable in the host.

The appropriate DNA sequence(s) may be inserted into the vector by a variety of procedures. In general, the DNA sequence is inserted into an appropriate restriction endonuclease site(s) by procedures known in the art, Standard techniques for cloning, DNA isolation, amplification and purification, for enzymatic reactions involving DNA ligase, DNA polymerase, restriction endonucleases and the like, and various separation techniques are those known and commonly employed by those skilled in the art. A number of standard techniques are described, for example, in Ausubel et al., (1993 Current Protocols in Molecular Biology, Greene Publ. Assoc. Inc. & John Wiley & Sons, Inc., Boston, MA); Sambrook et at (1989 Molecular Cloning, Second Ed., Cold Spring Harbor Laboratory, Plainview, NY); Maniatis et al. (1982 Molecular Cloning, Cold Spring Harbor Laboratory, Plainview, NY); Glover (Ed.) (1985 DNA Cloning Vol I and II, IRL Press, Oxford, UK); Hames and Higgins (Eds.), (1985 Nucleic Acid Hybridization, IRL Press, Oxford, UK); and elsewhere.

The DNA sequence in the expression vector is operatively linked to at least one appropriate expression control sequences (*e.g*., a constitutive promoter or a regulated promoter) to direct RNA synthesis. Representative examples of' such expression control sequences include promoters of eukaryotic cells or their viruses, as described above. Promoter regions can be selected from any desired gene using CAT (chloramphenicol transferase) vectors or other vectors with selectable markers Eukaryotic promoters include CMV immediate early, HSV thymidine kinase, early and late SV40, LTRs from netrovirus, and mouse metallothionein-I. Selection of the appropriate vector and promoter is well within the level of ordinary skill in the art, and preparation of' certain particularly preferred recombinant expression constructs comprising at least one promoter or regulate promoter operably linked to a nucleic acid encoding an binding domain-immunoglobulin fusion polypeptide is described herein.

Transcription of the DNA encoding the polypeptides of the present invention by higher eukaryotes may be increased by inserting an enhancer sequence into the vector. Enhancers are cis-acting elements of DNA, usually about from 10 to 300 bp that act on a promoter to increase its transcription. Examples including the SV40 enhancer on the late side of' the replication origin bp 100 to 270, a cytomegalovirus early promoter enhancer, the polyoma enhancer on the late side of the replication origin, and adenovirus enhancers.

As provided herein, in certain embodiments the vector may be a viral vector such as a retroviral vector. (Miller et al., 1989 BioTechniques 7:980; Coffin and Varmus, 1996 Retroviruses, Cold Spring Harbor Laboratory Press, NY.) For example, retroviruses from which the retroviral plasmid vectors may be derived include, but are not limited to, Moloney Murine Leukemia Virus, spleen necrosis virus, retroviruses such as Rous Sarcoma Virus, Harvey Sarcoma virus, avian leukosis virus, gibbon ape leukemia virus, human immunodeficiency virus, adenovirus, Myeloproliferative Sarcoma Virus, and mammary tumor virus

Retroviruses are RNA viruses which can implicate and integrate into the genome of a host cell via a DNA intermediate. This DNA intermediate, or provirus, may be stably integrated into the host cell DNA According to certain embodiments of the present invention, a vaccine may comprise a retrovirus into which a foreign gene that encodes a foreign protein is incorporated in place of normal retroviral RNA. When retroviral RNA enters a host cell coincident with infection, the foreign gene is also introduced into the cell, and may then be integrated into host cell DNA as if it were part of the retroviral genome. Expression of this foreign gene within the host results in expression of the foreign protein.

Most retroviral vector systems which have been developed for gene therapy are based on marine retroviruses. Such retroviruses exist in two forms, as free viral particles referred to as virions, or as proviruses integrated into host cell DNA The virion form of the virus contains the structural and enzymatic proteins of the retrovirus (including the enzyme reverse transcriptase), two RNA copies of the viral genome, and portions of the source cell plasma membrane containing viral envelope glycoprotein. The retroviral genome is organized into four main regions: the Long Terminal Repeat (LTR), which contains cis-acting elements necessary for the initiation and termination of transcription and is situated both 5' and 3' of the coding genes, and the three coding genes *gag, pol,* and *env.* These three genes *gag, pol,* and env encode, respectively, internal viral structures, enzymatic proteins (such as integrase), and the envelope glycoprotein (designated gp70 and p15e) which confers infectivity and host range specificity of the virus, as well as the "R" peptide of undetermined function.

Separate packaging cell lines and vector producing cell lines have been developed because of safety concerns regarding the uses of retroviruses, including their use in vaccines as provided by the present invention Briefly, this methodology employs the use of two components, a retroviral vector and a packaging cell line (PCL). The retroviral vector contains long terminal repeats (LTRs), the foreign DNA to be transferred and a packaging sequence (y). This retroviral vector will not reproduce by itself because the genes which encode structural and envelope proteins are not included within the vector genome. The PCL contains genes encoding the *gag, pol,* and env proteins, but does not contain the packaging signal "y". Thus, a PCL can only form empty virion particles by itself. Within this general method, the retroviral vector is introduced into the PCL, thereby creating a vector-producing cell line (VCL). This VCL manufactures virion particles containing only the retroviral vector's (foreign) genome, and therefore has previously been considered to be a safe retrovirus vector for therapeutic use.

"Retroviral vector construct" refers to an assembly which is, within preferred embodiments of' the invention, capable of directing the expression of a sequence(s) or gene(s) of interest, such as binding domain-immunoglobulin fusion encoding nucleic acid sequences. Briefly, the retroviral vector construct must include a 5' LTR, a tRNA binding site, a packaging signal, an origin of second strand DNA synthesis and a 3' LTR A wide variety of heterologous sequences may be included within the vector construct, including for example, sequences which encode a protein (*e.g*., cytotoxic protein, disease-associated antigen, immune accessory molecule, or replacement gene), or which are useful as a molecule itself (*e.g.,* as a ribozyme or antisense sequence).

Retroviral vector constructs of the present invention may be readily constructed from a wide variety of retroviruses, including for example, B, C, and D type retroviruses as well as spumaviruses and lentiviruses (*see, e.g.,* RNA Tumor Viruses, Second Edition, Cold Spring Harbor Laboratory, 1985). Such retroviruses may be readily obtained from depositories or collections such as the American Type Culture Collection ("ATCC"; Rockville, Maryland), or isolated from known sources using commonly available techniques. Any of the above retroviruses may be readily utilized in order to assemble or construct retroviral vector constructs, packaging cells, or producer cells of the present invention given the disclosure provided herein, and standard recombinant techniques (*e.g*., Sambrook et al, Molecular Cloning: A Laboratory Manual, 2d ed., Cold Spring Harbor Laboratory Press, 1989; Kunkle, PNAS 82:488,1985).

Suitable promoters for use in viral vectors generally may include, but are not limited to, the retroviral LTR; the SV40 promoter; and the human cytomegalovirus (CMV) promoter described in Miller, et al., Biotechniques 7:980-990 (1989), or any other promoter (*e.g*., cellular promoters such as eukaryotic cellular promoters including, but not limited to, the histone, pol III, and β-action promoters). Other viral promoters which may be employed include, but are not limited to, adenovirus promoters, thymidine kinase (IK) promoters, and B19 parvovirus promoters. The selection of a suitable promoter will be apparent to those skilled in the art from the teachings contained herein, and may be from among either regulated promoters or promoters as described above.

As described above, the retroviral plasmid vector is employed to transduced packaging cell lines to form producer cell lines. Examples of packaging cells which may be transfected include, but are not limited to, the PE501, PA317, ψ-2, ψ-AM, PA12, T19-14X, VT-19-17-H2, ψCRE, ψCRIP, GP+E-86, GP+envAm12, and DAN cell lines as described in Miller, Human Gene Therapy, 1:5-14 (1990). The vector may transduce the packaging cells through any means known in the art. Such means include, but are not limited to, electroporation, the use of liposomes, and CaPO₄ precipitation. In one alternative, the retroviral plasmid vector may be encapsulated into a liposome, or coupled to a lipid, and then administered to a host.

The producer cell line generates infectious retroviral vector particles which include the nucleic acid sequence(s) encoding the binding domain-immunoglobulin fusion polypeptides or fusion proteins. Such retroviral vector particles then may be employed, to transduce eukaryotic cells, either *in vitro* or *in vivo*, The transduced eukaryotic cells will express the nucleic acid sequence(s) encoding the binding domain-immunoglobulin fusion polypeptide or fusion protein Eukaryotic cells which may be transduced include, but are not limited to, embryonic stem cells, as well as hematopoietic stem cells, hepatocytes, fibroblasts, circulating peripheral blood mononuclear and polymorphonuclear cells including myelomonocytic cells, lymphocytes, myoblasts, tissue macrophages, dendritic cells, Kupffer cells, lymphoid and reticuloendothelia cells of the lymph nodes and spleen, keratinocytes) endothelial cells, and bronchial epithelial cells

As another example of an embodiment of the invention in which a viral vector is used to prepare the recombinant binding domain-immunoglobulin fusion expression construct, in one preferred embodiment, host cells transduced by a recombinant viral construct directing the expression of binding domain-immunoglobulin fusion polypeptides or fusion proteins may produce viral particles containing expressed binding domain-immunoglobulin fusion polypeptides or fusion proteins that are derived from portions of a host cell membrane incorporated by the viral particles during viral budding.

In another aspect, the present invention relates to host cells containing the above described recombinant binding domain-immunoglobulin fusion expression constructs. Host cells are genetically engineered (transduced, transformed or transfected) with the vectors and/or expression constructs of this invention which may be, for example, a cloning vector, a shuttle vector or an expression construct. The vector or construct may be, for example, in the form of a plasmid, a viral particle, a phage, etc. The engineered host cells can be cultured in conventional nutrient media modified as appropriate for activating promoters, selecting transformants or amplifying particular genes such as genes encoding binding domain immunoglobulin fusion polypeptides or binding domain immunoglobulin fusion fusion proteins. The culture conditions for particular host cells selected for expression, such as temperature, pH and the like, will be readily apparent to the ordinarily skilled artisan.

The host cell can be a higher eukaryotic cell, such as a mammalian cell, or a lower eukaryotic cell, such as a yeast cell, or the host cell can be a prokaryotic cell, such as a bacterial cell. Representative examples of appropriate host cells according to the present invention include, but need not be limited to, bacterial cells, such as *E coli, Streptomyces; Salmonella typhimurium;* fungal cells, such as yeast; insect cells, such as *Drosophila S2* and *Spodoptera Sf9;* animal cells, such as CHO, COS or 293 cells; adenoviruses; plant cells, or any suitable cell already adapted to *in vitro* propagation or so established *de novo.* The selection of an appropriate host is deemed to be within the scope of those skilled in the art from the teachings herein.

Various mammalian cell culture systems can also be employed to express recombinant protein. Examples of mammalian expression systems include the COS-7 lines of monkey kidney fibroblasts, described by Gluzman, Cell 23:175 (1981), and other cell lines capable of expressing a compatible vector, for example, the C127, 3T3, CHO, HeLa and BHK cell lines. Mammalian expression vectors will comprise an origin of replication, a suitable promoter and enhancer, and also any necessary ribosome binding sites, polyadenylation site, splice donor and acceptor sites, transcriptional termination sequences, and 5' flanking nontranscribed sequences, for example as described herein regarding the preparation of binding domain immunoglobulin fusion expression constructs. DNA sequences derived from the SV40 splice, and polyadenylation sites may be used to provide the required nontranscribed genetic elements. Introduction of the construct into the host cell can be effected by a variety of methods with which those skilled in the art will be familiar, including but not limited to, for example, calcium phosphate transfection, DBAE-Dextran mediated transfection, or electroporation (Davis et al.., 1986 Basic Methods in Molecular Biology)*.*

The present invention binding domain-immunoglobulin fusion proteins may be formulated into pharmaceutical compositions for administration according to well known methodologies. Pharmaceutical compositions generally comprise one or more recombinant expression constructs, and/or expression products of such constructs, in combination with a pharmaceutically acceptable carrier, excipient or diluent Such carriers will be nontoxic to recipients at the dosages and concentrations employed. For nucleic acid-based formulations, or for formulations comprising expression products of the subject invention recombinant constructs, about 001 µg/kg to about 100 mg/kg body weight will be adminstered, typically by the intradermal, subcutaneous, intramuscular or intravenous route, or by other routes. A preferred dosage is about 1 µg/kg to about 1 mg/kg, with about 5 µg/kg to about 200 µg/kg particularly preferred It will be evident to those skilled in the art that the number and frequency of administration will be dependent upon the response of the host. Pharmaceutically acceptable carriers" for therapeutic use are well known in the pharmaceutical art, and are described, for example, in Remingtons Pharmaceutical Sciences, Mack Publishing Co. (A.R. Gennaro edit 1985). For example, sterile saline and phosphate-buffered saline at physiological pH may be used. Preservatives, stabilizers, dyes and even flavoring agents may be provided in the pharmaceutical compositions For example, sodium benzoate, sorbic acid and esters of *p*-hydroxybenzoic acid may be added as preservatives. Id. at 1449. In addition, antioxidants and suspending agents may be used Id.

"Pharmaceutically acceptable salt" refers to salts of the compounds of the present invention derived from the combination of such compounds and an organic or inorganic acid (acid addition salts) or an organic or inorganic base (base addition salts). The compounds of the present invention may be used in either the free base or salt forms, with both forms being considered as being within the scope of the present invention,

The pharmaceutical compositions that contain one or more binding domain-immunoglobulin fusion protein encoding constructs (or their expressed products) may be in any form which allows for the composition to be administered to a patient. For example, the composition may be in the form of' a solid, liquid or gas (aerosol), Typical routes of administration include, without limitation, oral, topical, parenteral (*e g*., sublingually or buccally), sublingual, rectal, vaginal, and intranasal. The term parenteral as used herein includes subcutaneous injections, intravenous, intramuscular, intrasternal, intracavernous, intrathecal, intrameatal, intraurethral injection or infusion techniques. The pharmaceutical composition is formulated so as to allow the active ingredients contained therein to be bioavailable upon administration of the composition to a patient. Compositions that will be administered to a patient take the form of one or more dosage units, where for example, a tablet may be a single dosage unit, and a container of one or more compounds of the invention in aerosol form may hold a plurality of dosage units.

For oral administration, an excipient and/or binder may be present. Examples are sucrose, kaolin, glycetin, starch dextrins, sodium alginate, carboxymethylcellulose and ethyl cellulose. Coloring and/or flavoring agents may be present A coating shell may be employed.

The composition may be in the form of' a liquid, *e g*., an elixir, syrup, solution, emulsion or suspension. The liquid may be for oral administration or for delivery by injection, as two examples, When intended for oral administration, preferred compositions contain, in addition to one or more binding domain immunoglobulin fusion construct or expressed product, one or more of a sweetening agent, preservatives, dye/colorant and flavor enhancer. In a composition intended to be administered by injection, one or more of a surfactant, preservative, wetting agent, dispersing agent, suspending agent, buffer, stabilizer and isotonic agent may be included.

A liquid pharmaceutical composition as used herein, whether in the form of' a solution, suspension or other like form, may include one or more of the following adjuvants: sterile diluents such as water for injection, saline solution, preferably physiological saline, Ringer's solution, isotonic sodium chloride, fixed oils such as synthetic mono or digylcerides which may serve as the solvent or suspending medium, polyethylene glycols, glycerin, propylene glycol or other solvents; antibacterial agents such as benzyl alcohol or methyl paraben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampoules, disposable syringes or multiple dose vials made of glass or plastic. Physiological saline is a preferred adjuvant. An injectable pharmaceutical composition is preferably sterile.

It may also be desirable to include other components in the preparation, such as delivery vehicles including but not limited to aluminum salts, water-in-oil emulsions, biodegradable oil vehicles, oil-in water emulsions, biodegradable microcapsules, and liposomes. Examples of immunostimulatory substances (adjuvants) for use in such vehicles include N-acetylmuramyl-L-alanine-D-isoglutamme (MDP), lipopoly-sacchatides (LPS), glucan, IL-12, GM-CSF, gamma interferon and IL-15

While any suitable carrier known to those of ordinary skill in the art may be employed in the pharmaceutical compositions of this invention, the type of carrier will vary depending on the mode of administration and whether a sustained release is desired. For parenteral administration, such as subcutaneous injection, the carrier preferably comprises water, saline, alcohol, a fat, a wax or a buffer. For oral administration, any of the above carriers or a solid carrier, such as mannitol, lactose, starch, magnesium stearate, sodium saccharine, talcum, cellulose, glucose, sucrose, and magnesium carbonate, may be employed. Biodegradable microspheres (*e*.*g*., polylactic galactide) may also be employed as carriers for the pharmaceutical compositions of this invention. Suitable biodegradable microspheres are disclosed, for example, in US Patent Nos. 4,897,268 and 5,075,109. In this regard, it is preferable that the microsphere be larger than approximately 25 microns.

Pharmaceutical compositions may also contain diluents such as buffers, antioxidants such as ascorbic acid, low molecular weight (less than about 10 resides) polypeptides, proteins, amino acids, carbohydrates including glucose, sucrose or dextrins, chelating agents such as EDTA, glutathione and other stabilizers and excipients. Neutral buffered saline or saline mixed with nonspecific serum albumin are exemplary appropriate diluents. Preferably, product is formulated as a lyophilizate using appropriate excipient solutions (*e*.*g*., sucrose) as diluents.

As described above, the subject invention includes compositions capable of delivering nucleic acid molecules encoding binding domain-immunoglobulin fusion proteins. Such compositions include recombinant viral vectors (*e*.*g*, retroviruses (*see* WO 90/07936, WO 91/02805, WO 93/25234, WO 93/25698, and WO 94/03622), adenovirus *(see* Berkner, Biotechniques 6:616-627, 1988; Li et al.., Hum Gene Ther 4:403-409, 1933; Vincent et al., Nat. Genet. 5:130-1.34, 1993; and Kolls et al., Proc Natl Acad. Sci USA 91:215-219, 1994), pox virus *(see* U.S Patent No. 4,769,330; U S Patent No 5,017,487; and WO 89/01973)), recombinant expression construct nucleic acid molecules complexed to a polycationic molecule *(see* WO 93/03709), and nucleic acids associated with liposomes *(see* Wang et al., Proc. Natl, Acad. Sci USA 84:7851, 1987), In certain embodiments, the DNA may be linked to killed or inactivated adenovirus (*see* Curiel et al., Hum Gene Ther. 3:147-154, 1992; Cotton et al., Proc. Natl. Acad. Sci USA 89:6094, 1992). Other suitable compositions include DNA-ligand (*see* Wu et al, J. Biol. Chem. 264:16985-16987, 1989) and lipid-DNA combinations *(see* Felgner et al.,, Proc Natl. Acad Sci. USA 84:7413-7417, 1989).

In addition to direct *in vivo* procedures, *ex vivo* procedures may be used in which cells are removed from a host, modified, and placed into the same or another host animal, It will be evident that one can utilize any of the compositions noted above for introduction of binding domain-immunoglobulin fusion proteins or of binding domain-mununoglobulin fusion protein encoding nucleic acid molecules into tissue cells in an *ex vivo* context. Protocols for vital, physical and chemical methods of uptake are well known in the art.

Accordingly, the present invention is useful for treating a patient having a B-cell disorder or a malignant condition, or for treating a cell culture derived from such a patient. As used herein, the term "patient" refers to any warm-blooded animal, preferably a human. A patient may be afflicted with cancer, such as B-cell lymphoma, or may be normal (*i.e.,* free of detectable disease and infection). A "cell culture" is any preparation amenable to *ex vivo* treatment, for example a preparation containing immunocompetent cells or isolated cells of the immune system (including, but not limited to, T cells, macrophages, monocytes, B cells and dendritic cells) Such cells may be isolated by any of a variety of techniques well known to those of ordinary skill in the art (*e*.*g*, Ficoll-hypaque density centrifugation). The cells may (but need not) have been isolated from a patient afflicted with a B-cell disorder or a malignancy, and may be reintroduced into a patient after treatments

A liquid composition intended for either parenteral or oral administration should contain an amount of binding domain-immunoglobulin fusion protein encoding construct or expressed product such that a suitable dosage will be obtained. Typically, this amount is at least 001 wt% of a binding domain-immunoglobulin fusion construct or expressed product in the composition, When intended for oral administration, this amount may be varied to be between 0,1 and about 70% of the weight of' the composition. Preferred oral compositions contain between about 4% and about 50% of binding domain-immunoglobulin fusion construct or expressed product(s). Preferred compositions and preparations are prepared so that a parenteral dosage unit contains between 001 to 1% by weight of active compound.

The pharmaceutical composition may be intended for topical administration, in which case the carrier may suitably comprise a solution, emulsion, ointment or gel base. The base, for example, may comprise one or more of the following: petrolatum, lanolin, polyethylene glycols, beeswax, mineral oil, diluents such as water and alcohol, and emulsifiers and stabilizers. Thickening agents may be present in a pharmaceutical composition for topical administration If intended for transdermal administration, the composition may include a transdermal patch or iontophoresis device. Topical formulations may contain a concentration of the binding domain-immunoglobulin fusion construct or expressed product of from about 0.1 to about 10% w/v (weight per unit volume).

The composition may be intended for rectal administration, in the form, *e*.*g.,* of' a suppository which will melt in the rectum and release the drug. The composition for rectal administration may contain an oleaginous base as a suitable nonimitating excipient Such bases include, without limitation, lanolin, cocoa butter and polyethylene glycol.

In the methods of' the invention, the binding domain-immunoglobulin fusion encoding constructs or expressed product(s) may be administered through use of insert(s), bead(s), timed-release formulation(s), patch(es) or last-release formulation(s).

The following Examples are offered by way of illustration and not by way of limitation.

### EXAMPLES

### EXAMPLE 1

### CLONING OF THE 2H7 VARIABLE REGIONS AND CONSTRUCTION AND SEQUENCING OF 2H7scFV-IG

This Example illustrates the cloning of cDNA molecules that encode the heavy chain and light chain variable regions of the monoclonal antibody 2H7 This Example also demonstrates the construction, sequencing, and expression of' 2H7scFv-Ig.

Hybridoma cells expressing 2H7 monoclonal antibody that specifically bound to CD20 were provided by Ed Clark at the University of Washington, Seattle, WA. Prior to harvesting, hybridoma cells were kept in log phase growth for several days in RPMI 1640 media (Life Technologies, Gaithersburg, MD) supplemented with glutamine, pyruvate, DMEM non-essential amino acids, and penicillin-streptomycin Cells were pelleted by centrifugation from the culture medium, and 2 x 10⁷ cells were used to prepare RNA. RNA was isolated from the 2H7-producing hybridoma cells using the Pharmingen (San Diego, CA) total RNA isolation kit (Catalog # 45520K) according to the manufacturer's instructions accompanying the kit One microgram (1 □g) of total RNA was used as template to prepare cDNA by reverse transcription. The RNA and 300 ng random primers were combined and denatured at 72 °C for 10 minutes prior to addition of enzyme. Superscript II reverse transcriptase (Life Technologies) was added to the RNA plus primer mixture in a total volume of 25 □1 in the presence of 5X second stand buffer and 0,1 M DTT provided with the enzyme. The reverse transcription reaction was allowed to proceed at 42°C for one hour

The 2H7 cDNA generated in the randomly primed reverse transcriptase reaction and V region specific primers were used to amplify by PCR the variable regions for the light and heavy chain of the 2H7 antibody, The V region specific primers were designed using the published sequence (Genbank accession numbers M17954 for V_{L} and M17953 for V_{H}) as a guide. The two variable chains were designed with compatible end sequences so that an scFv could be assembled by ligation of the two V regions after amplification and restriction enzyme digestion.

A (gly₄ser)₃ peptide linker to be inserted between the two V regions was incorporated by adding the extra nucleotides to the antisense primer for the V_{L} of 2H7 A Sac I restriction site was also introduced at the junction between the two V regions The sense primer used to amplify the 2H7 V_{L}, that included a HindIII restriction site and the light chain leader peptide was 5'-gtc *aag ctt* gcc gcc atg gat ttt caa gtg cag att ttt cag c·3' (SEQ ID NO:__), The antisense primer was 5'-gtc *gtc gag ctc* cca cct cct cca gat cca cca, ccg ccc gag cca ccg cca cct ttc age tcc age ttg gtc cc-3' (SEQ ID NO:_ ) The reading frame of the V region is indicated as a bold, underlined codon. The Hind III and SacI sites axe indicated by underlined italicized sequences.

The V_{H} domain was amplified without a leader peptide, but included a 5' SacI restriction site for fusion to the V_{L} and a Bell restriction site at the 3' end for fusion to various tails, including the human IgG1 Fc domain and the truncated forms of CD40 ligand,CD154. The sense primer was 5'-gct gct *gag ctc* tca ggc tta tct aca gca agt ctg g-3' (SEQ ID NO:_). The SacI site is indicated in italicized and underlined font, and the reading frame of the codon for the first amino acid of the V_{H} domain is indicated in bold, underlined type The antisense primer was 5'-gti gtc *tga tca* gag acg gtg acc gtg gtc cc-3' (SEQ ID NO:_). The Bell site is indicated in italicized, underlined type, and the last serine of the V_{H} domain sequence is indicated in bold, underlined type,

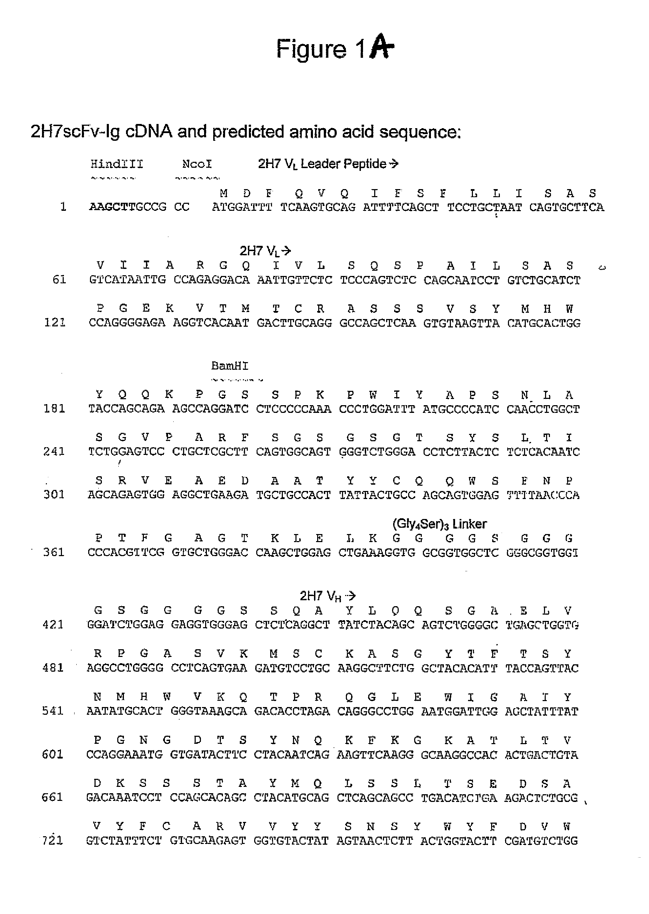

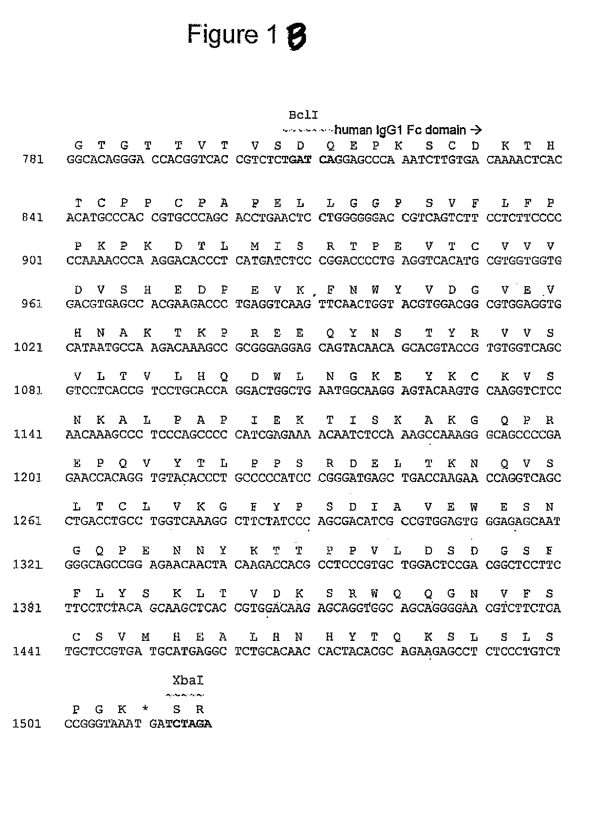

The scFv-Ig was assembled by inserting the 2H7 scFv HindIII-BcII fragment into pUC19 containing the human IgG1 hinge, CH2, and CH3 regions, which was digested with restriction enzymes, HindIII and Bell. After ligation, the ligation products were transformed into DH5α bacteria. Positive clones were screened for the properly inserted fragments using the SacI site at the V_{L}-V_{H} junction of 2H7 as a diagnostic site, The 2H7scFv-Ig cDNA was subjected to cycle sequencing on a PE 9700 thermocycler using a 25-cycle program by denaturing at 96 °C for 10 seconds, annealing at 50°C for 30 seconds, and extending at 72°C for 4 minutes The sequencing primers were pUC forward and reverse primers and an internal primer that annealed to the CH2 domain human in the IgG constant region portion Sequencing reactions were performed using the Big Dye Terminator Ready Sequencing Mix (PE Applied Biosystems, Foster City, CA) according to the manufacturer's instructions. Samples were subsequently purified using Centrisep columns (Catalog # CS-901, Princeton Separations, Adelphia, NJ.), the eluates dried in a Savant vacuum dryer, denatured in Template Suppression Reagent (PE-ABI), and analyzed on an ABI 310 Genetic Analyzer (PE-Applied Biosystems,) The sequence was edited, translated, and analyzed using Vector Nti version 6.0 (Informax, North Bethesda, MD), Figure 1 shows the cDNA and predicted amino acid sequence of the 2H7scFv-Ig construct.

### EXAMPLE 2

### EXPRESSION OF 2H7 ScFv-IG IN STABLE CHO CELL LINES

This Example illustrates expression of 2H7scFv-Ig in a eukaryotic cell line and characterization of the expressed 2H7scFy-Ig by SDS-PAGE and by functional assays, including ADCC and complement fixation.

The 2H7scFv-Ig HindIII-XbaI (-16 kb) fragment with correct sequence was insetted into the mammalian expression vector pD18, and DNA from positive clones was amplified using QIAGEN plasmid preparation kits (QIAGEN, Valencia, CA) The recombinant plasmid DNA (100 □g) was then linearized in a nonessential region by digestion with AscI, purified by phenol extraction, and resuspended in tissue culture media, Excell 302 (Catalog # 14312-79P, JRH Biosciences, Lenexa, KS), Cells for transfection, CHO DG44 cells, were kept in logarithmic growth, and 10⁷ cells harvested for each transfection reaction. Linearized DNA was added to the CHO cells in a total volume of 0.8 ml for electroporation.

Stable production of the 2H7 scFv-Ig fusion protein (SEQ. ID NO:10) was achieved by electroporation of' a selectable, amplifiable plasmid, pD18, containing the 2H7 scFv-Ig cDNA under the control of the CMV promoter, into Chinese Hamster Ovary (CHO) cells. The 2H7 expression cassette was subcloned downstream of the CMV promoter into the vector multiple cloning site as a ~1.6 kb HindIII-XbaI fragment. The pD18 vector is a modified version of pcDNA3 encoding the DHFR selectable marker with an attenuated promoter to increase selection pressure for the plasmid. Plasmid DNA was prepared using Qiagen maxiprep kits, and purified plasmid was linearized at a unique AscI site prior to phenol extraction and ethanol precipitation. Salmon sperm DNA (Sigma-Aldrich, St. Louis, MO) was added as carrier DNA, and 100 □g each of plasmid and carrier DNA was used to transfect 10⁷ CHO DG44 cells by electroporation. Cells were grown to logarithmic phase in Excell 302 media (JRH Biosciences) containing glutamine (4mM), pyruvate, recombinant insulin, penicillin-streptomycin, and 2X DMEM nonessential amino acids (all from Life Technologies, Gaithersburg, Maryland), hereafter referred to as "Excell 302 complete" media, Media for untransfected cells also contained HT (diluted from a 100X solution of' hypoxanthine and thymidine) (Life Technologies) Media for transfections under selection contained varying levels of methotrexate (Sigma-Aldrich) as selective agent, ranging from 50 nM to 5 □M. Electroporations were performed at 275 volts, 950 □F. Transfected cells were allowed to recover overnight in non-selective media prior to selective plating in 96 well flat bottom plates (Costar) at varying serial dilutions ranging from 125 cells/well to 2000 cells/well Culture media for cell cloning was Excell .302 complete, containing 100 nM methotrexate. Once clonal outgrowth was sufficient, serial dilutions of culture supernatants from master wells were screened for binding to CD20-CHO transfected cells. The clones with the highest production of the fusion protein were expanded into T25 and then T75 flasks to provide adequate numbers of cells for freezing and for scaling up production of the 2H7scFvIg., Production levels were further increased in cultures from three clones by progressive amplification in methotrexate containing culture media At each successive passage of cells, the Excell 302 complete media contained an increased concentration of methotrexate, such that only the cells that amplified the DHFR plasmid could survive.

Supernatants were collected from CHO cells expressing the 2H7scFv-Ig, filtered through 0.2 □m PES express filters (Nalgene, Rochester, NY) and were passed over a Protein A-agarose (IPA 300 crosslinked agarose) column (Repligen, Needham, MA), The column was washed with PBS, and then bound protein was eluted using 0.1 M citrate buffer, pH 3.0. Fractions were collected and eluted protein was neutralized using 1M Tris, pH 8.0, prior to dialysis overnight in PBS., Concentration of the purified 2H7scFv-Ig (SEQ ID NO:__) was determined by absorption at 280 nm. An extinction coefficient of 1.77 was determined using the protein analysis tools in the Vector Nti Version 60 Software package (Informax, North Bethesda, MD).. This program uses the amino acid composition data to calculate extinction coefficients

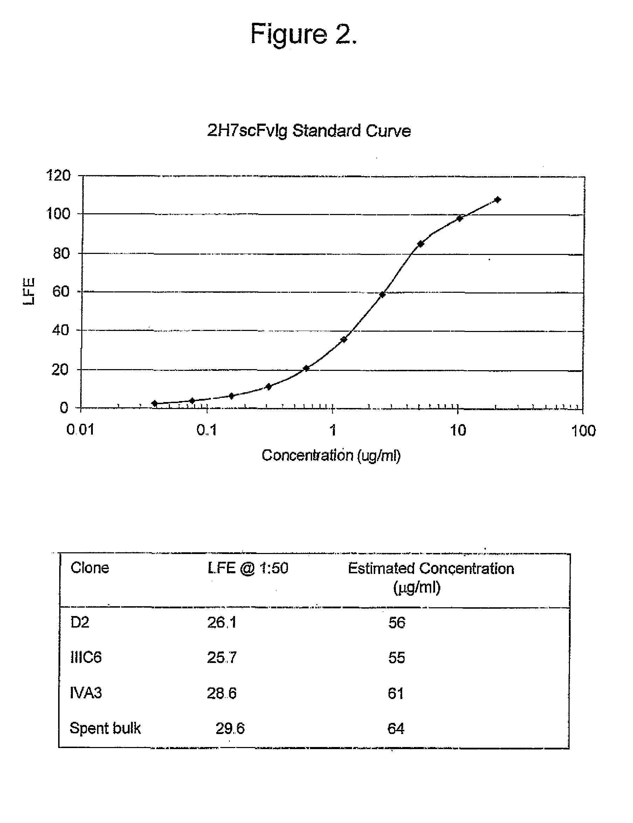

Production levels of 2H7scFv-Ig by transfected, stable CHO cells were analyzed by flow cytometry. Purified 2H7scFv-Ig to CHO cells was allowed to bind to CHO cells that expressed CD20 (CD20 CHO) and analyzed by flow cytometry using a fluorescein-conjugated anti-human IgG second step reagent (Catalog Numbers H10101 and H10501, CalTag, Burlingame, CA). Figure 2 (top) shows a standard curve generated by titration of 2H7scFv-Ig binding to CD20 CHO At each concentration of' 2H7scFv-Ig, the mean brightness of the fluorescein signal in linear units is shown, Supernatants collected from T flasks containing stable CHO cell clones expressing 2H7scFv-Ig were then allowed to bind to CD20 CHO and the binding was analyzed by flow cytometry The fluorescein signal generated by 2H7scFv-Ig contained in the supernatants was measured and the 2H7scFv-Ig concentration in the supernatants was calculated from the standard curve (Figure 2, bottom).

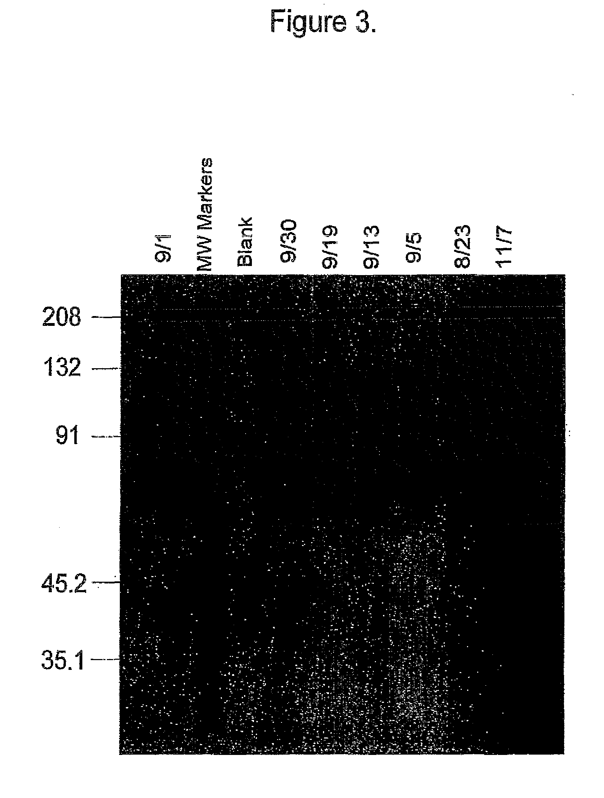

Purified 2H7scFv-Ig (SEQ ID NO:_) was analyzed by electrophoresis on SDS-Polyactylamide gels. Samples of 2H7scFv-Ig, purified by independent Protein A Agarose column runs, were boiled in SDS sample buffer without reduction of disulfide bonds and applied to SDS 10% Tris-BIS gels (Catalog # NP0301, Novex, Carlsbad, CA). Twenty micrograms of each purified batch was loaded on the gels. The proteins were visualized after electrophoresis by Coomassie Blue staining (Pierce Gel Code Blue Stain Reagent, Catalog #24590, Pierce, Rockfotd, IL), and destaining in distilled water Molecular weight markers were included on the same gel (Kaleidoscope Prestained Standards, Catalog # 161-0324, Bio-Rad, Hercules, CA) The results are presented in Figure 3. The number above the lanes designate independent purification batches. The molecular weights in kilodaltons of the size markers are indicated on the left side of the figure. Further experiments with alternative sample preparation conditions indicated that reduction of disulfide bonds by boiling the protein in SDS sample buffer containing DTT or 2-mercaptoethanol caused the 2H7scFv-Ig to aggregate.

Any number of other immunological parameters may be monitored using routine assays that are well known in the art. These may include, for example, antibody dependent cell-mediated cytotoxicity (ADCC) assays, secondary *in vitro* antibody responses, flow immunocytofluorimetric analysis of various peripheral blood or lymphoid mononuclear cell subpopulations using well established marker antigen systems, immunohistochemistry or other relevant assays. These and other assays may be found, for example, in Rose et al (Eds.), *Manual of Clinical Laboratory Immunology, 5^{th} Ed.,* 1997 American Society of Microbiology, Washington, DC.

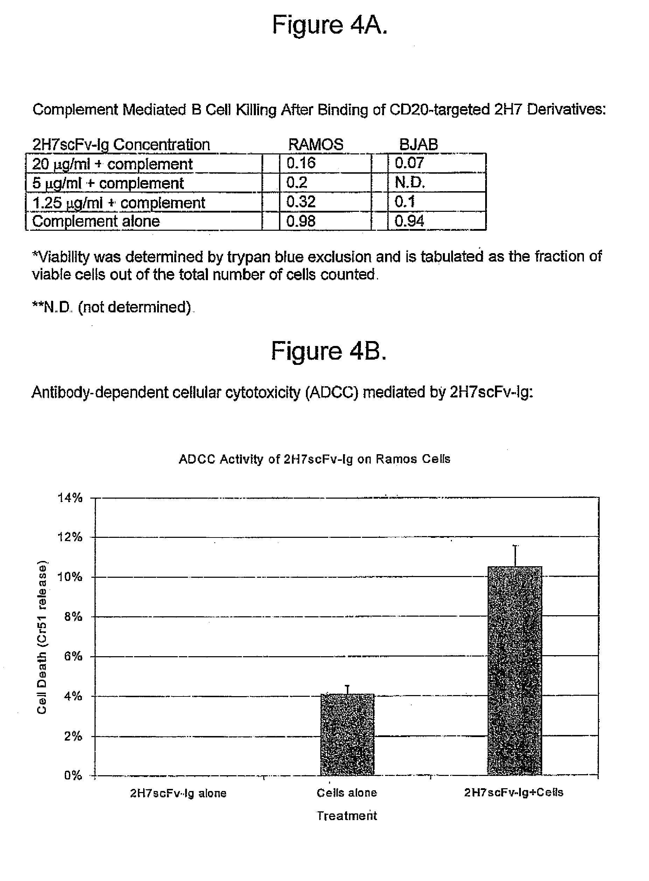

The ability of 2H7scFv-Ig to kill CD20 positive cells in the presence of' complement was tested using B cell lines Ramos and Bjab. Rabbit complement (Pel-Freez, Rogers, AK) was used in the assay at a final dilution of 1/10. Purified 2H7scFv-Ig was incubated with B cells and complement for 45 minutes at 37 °C, followed by counting of live and dead cells by trypan blue exclusion. The results in Figure 4A show that in the presence of rabbit complement, 2H7scFv-Ig lysed B cells expressing CD20

The ability of 2H7scFv-Ig to kill CD20 positive cells in the presence of peripheral blood mononuclear cells (PBMC) was tested by measuring the release of ⁵¹Cᵣ from labeled Bjab cells in a 4-hour assay using a 100:1 ratio of PBMC to Bjab cells. The results shown in Figure 4B indicated that 2H7scFv-Ig can mediate antibody dependent cellular cytotoxicity (ADCC) because the release of ⁵¹Cr was higher in the presence of both PBMC and 2H7scFv-Ig than in the presence of either PBMC or 2H7scFv-Ig alone.

### EXAMPLE 3

### EFFECT OF SIMULTANEOUS LIGATION OF CD20 AND CD40 ON GROWTH OF NORMAL B CELLS, AND ON CD95 EXPRESSION, AND INDUCITION OF APOPIOSIS

This example illustrates the effect of cross-linking of CD20 and CD40 expressed on the cell surface on cell proliferation

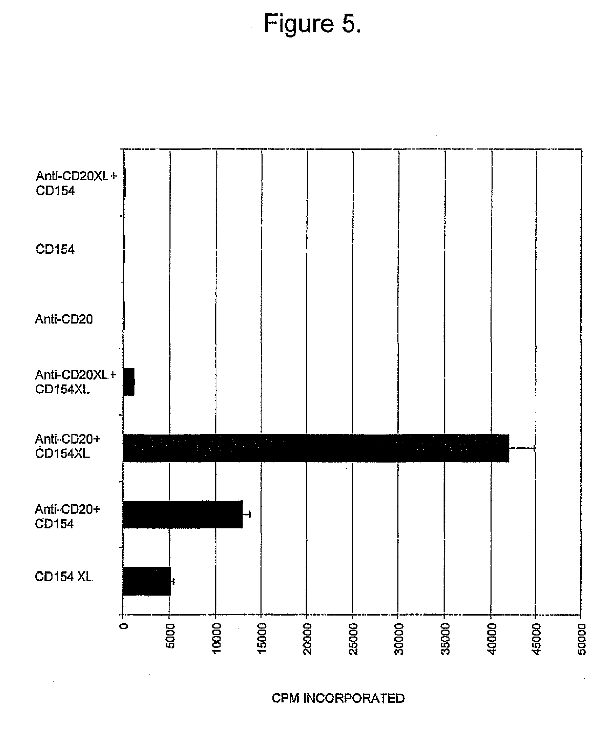

Dense resting B cells were isolated from human tonsil by a Percoll step gradient and T cells were removed by E-rosetting. Proliferation of resting, dense tonsillar B cells was measured by uptake of ³[H]-thymidine during the last 12 hours of a 4-day experiment. Proliferation was measured in quadruplicate cultures with means and standard deviations as shown. Murine anti-human CD20 mAb 1F5 (anti-CD20) was used alone or was cross-linked with anti-mmine κ mAb 187.1 (anti-CD20XL) CD40 activation was accomplished using soluble human CD154 fused with murine CD8 (CD154) (Hollenbaugh et al., E34BO J 11: 4212-21 (1992)), and CD40 cross-linking was accomplished using anti-murine CD8 mAb 53-6 (CD154XL). This procedure allowed simultaneous cross-linking of CD20 and CD40 on the cell surface. The results are presented in Figure 5.

The effect of CD20 and CD40 cross-linking on Ramos cells, a B lymphoma cell line, was examined. Ramos cells were analyzed for CD95 (Fas) expression and percent apoptosis eighteen hours after treatment (no goat anti-mousse IgG (GAM)) and/or crosslinking (+GAM) using murine mAbs that specifically bind CD20 (1F5) and CD40 (G28-5). Control cells were treated with a non-binding isotype control (64 1) specific for CD3.

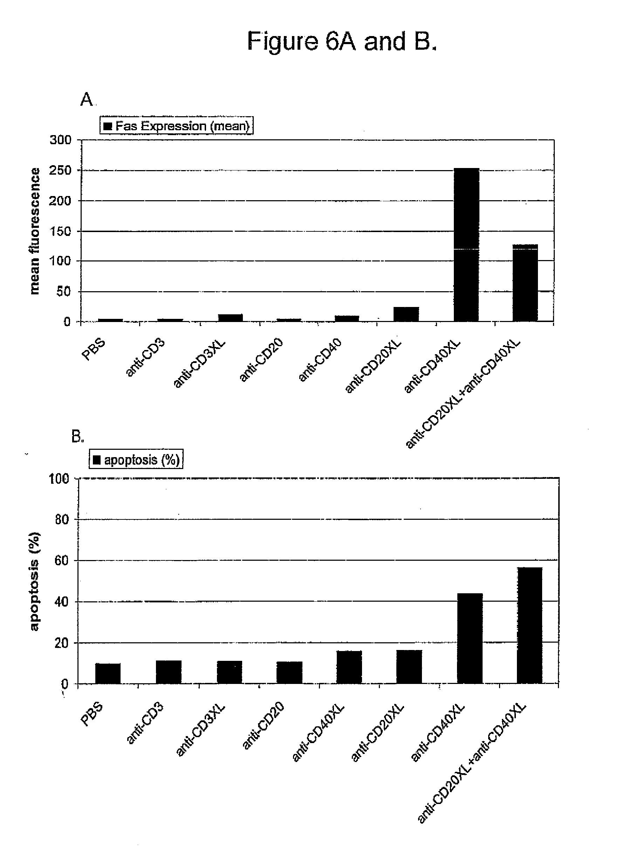

Treated Ramos cells were harvested, incubated with FITC-anti-CD9S, and analyzed by flow cytometry to determine the relative expression level of Fas on the cell surface after CD20 or CD40 cross-linking. Data is plotted as mean fluorescence of cells after treatment with the stimuli indicated (Figure 6A).

Treated Ramos cells from the same experiment were harvested and binding of annexin V was measured to indicate the percentage apoptosis in the treated cultures. Apoptosis was measured by binding of Annexin V 18 hours after cross-linking of CD20 and CD40 using 1F5 and G28-5 followed by cross-linking with GAM. Binding of' Annexin V was measured using a FITC-Annexin V kit (Catalog # PN-IM2376, Immunotech, Marseille, France,). Annexin V binding is known to be an early event in progression of' cells into apoptosis Apoptosis, or programmed cell death, is a process characterized by a cascade of' catabolic reactions leading to cell death by suicide. In the early phase of apoptosis, before cells change morphology and hydrolyze DNA, the integrity of the cell membrane is maintained but cells lose the asymmetry of their membrane phospholipids, exposing negatively charged phospholipids, such as phosphatidylserine, at the cell surface. Annexin V, a calcium and phopholipid binding protein, binds preferentially and with high affinity to phosphatidylserine, Results demonstrating the effect of cross-linking both CD20 and CD40 on expression of the FAS receptor (CD95) are presented in Figure 6B. The effect of cross-linking of both CD20 and CD40 on Annexin V binding to cells is shown in Figure 6B.

### EXAMPLE 4

### CONSTRUCTION AND CHARACTERIZATION OF 2H7 ScFv-CD154 FUSION PROTEINS

To construct a molecule capable of binding to both CD20 and CD40, cDNA encoding the 2H7 scFv was fused with cDNA encoding CD 154, the CD40 ligand. The 2H7 scFv cDNA encoded on the HindIII-BclI fragment was removed from the 2H7 scFvIg construct, and inserted into a pD18 vector along with a BarnHI-XbaI cDNA fragment encoding the extracellular domain of human CD154. The extracellular domain is encoded at the carboxy terminus of CD154, similar to other type II membrane proteins.

The extracellular domain of human CD154 was PCR amplified using cDNA generated with random primers and RNA from human T lymphocytes activated with PHA (phytohemagglutinin). The primer sets included two different 5' or sense primers that created fusion junctions at two different positions within the extracellular domain of CD154 Two different fusion junctions were designed that resulted in a short or truncated form (form S4) including amino acids 108 (Glu)-261 (Leu) + (Glu), and a long or complete form (form L2) including amino acids 48 (Arg) -261 (Leu) + (Glu), of the extracellular domain of CD154, both constructed as BamHI-XbaI fragments. The sense primer which fuses the two different truncated extracellular domains to the 2H7scFv includes a BamHI site for cloning. The sense primer for the S4 form of the CD154 cDNA is designated SEQUENCE ID NO: 11 or CD154BAM108 and encodes a 34 mer with the following sequences 5'-gtt gtc gga tcc aga aaa cag ctt tga aat gca a-3' , while the antisense primer is designated SEQUENCE ID NO: 12 or CD154XBA and encodes a 44 mer with the following sequence: 5'-gtt gtt tct aga tta tea etc gag ttt gag taa gcc aaa gga cg-3'.

The oligonucleotide primers used in amplifying the long form (L2) of the CD154 extracellular domain encoding amino acids 48 (Arg)-261 (Leu) + (Glu), were as follows: The sense primer designated CD154 BAM48 (SEQUENCE ID NO:13) encoded a 35-mer with the following sequence: 5'-gtt gtc gga tcc aag aag gtt gga caa gat aga ag-3'. The antisense primer designated or CD 154XBA (SEQUENCE ID NO:_) encoded the 44-mer: 5'-gtt gtt tct aga tta tca ctc gag ttt gag taa gcc aaa gga cg-3', Other PCR reaction conditions were identical to those used for amplifying the 2H7 scFv (see Example 1), PCR fragments were purified by PCR quick kits (QIAGEN, San Diego, CA), eluted in 30 □l ddH₂O, and digested with BamHI and XbaI (Roche) restriction endonucleases in a 40 □l reaction volume at 37 °C for 3 hours. Fragments were gel purified, purified using QIAEX kits according to the manufactures instructions (QIAGEN), and ligated along with the 2H7 HindIII.-BcII fragment into the pD18 expression vector digested with HindIII-XbaI. Ligation reactions were transformed into DH5-alpha chemically competent bacteria and plated onto LB plates containing 100 □g/ml ampicillin. Transformants were grown overnight at 37 °C, and isolated colonies used to inoculate 3 ml liquid cultures in Luria Broth containing 100 □g/ml ampicillin. Clones were screened after mini-plasmid preparations (QIAGEN) for insertion of both the 2H7 scFv and the CD154 extracellular domain fragments.

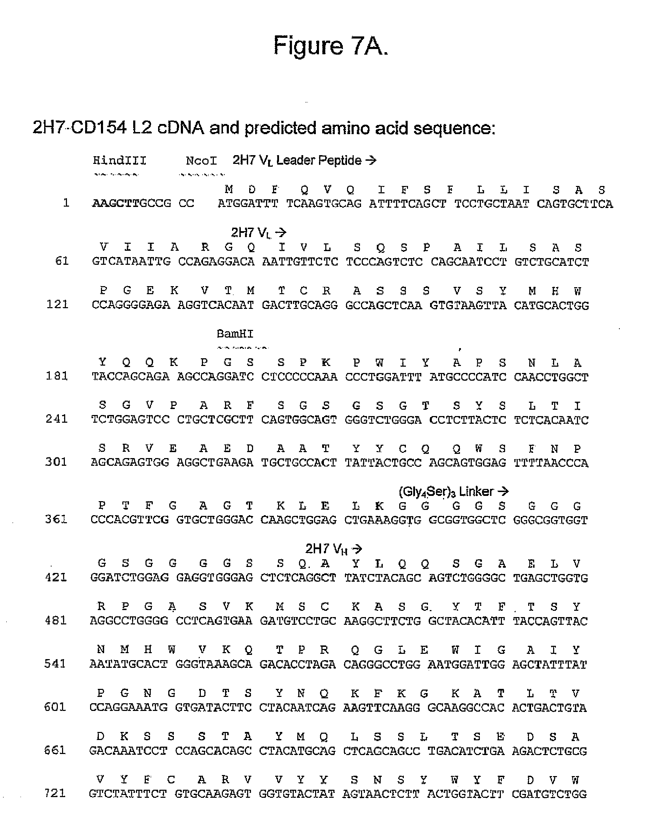

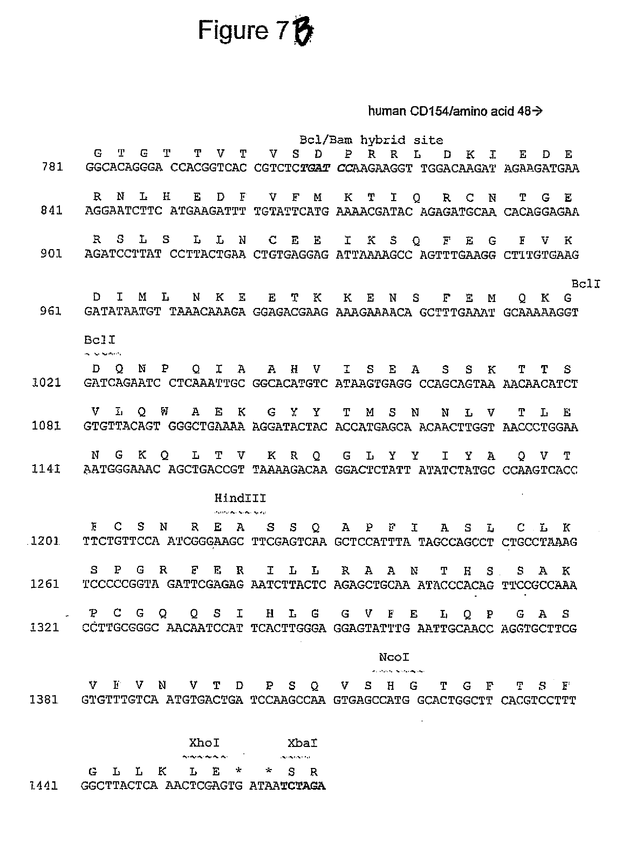

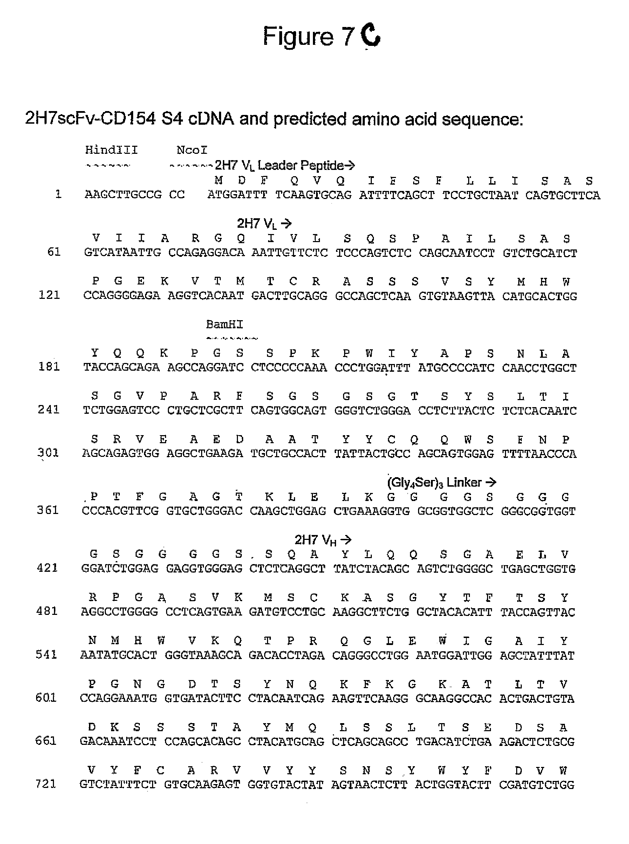

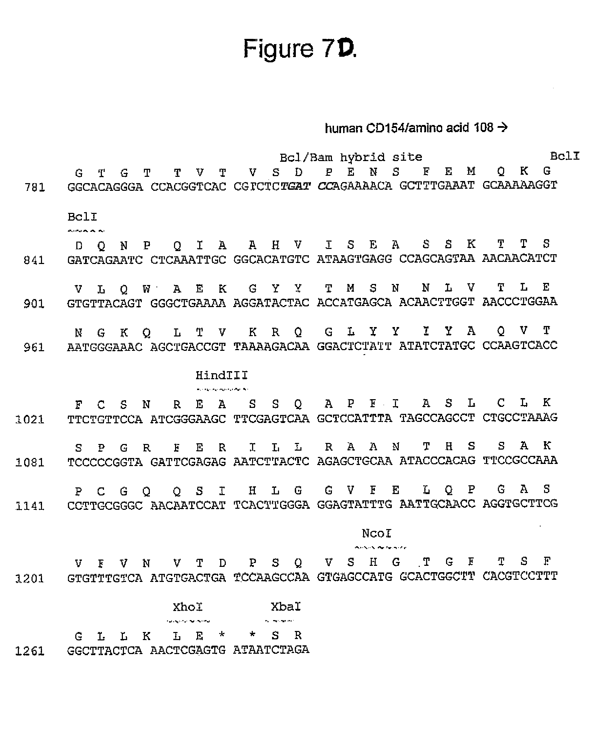

The 2H7scFv-CD154 construct cDNAs were subjected to cycle sequencing on a PE 9700 thermocycler using a 25-cycle program that included denaturating at 96 °C, 10 seconds, annealing at 50 °C for 5 seconds, and extension at 60°C, for 4 minutes. The sequencing primers used were pD18 forward (SEQ ID NO:__: 5'-gtctatataagcagagctctggc-3') and pD18 reverse (SEQ ID NO:_: 5'-cgaggctgatcagcgagctctagca-3') primers. In addition, an internal primer was used that had homology to the human CD154 sequence (SEQ ID NO:_: 5'-ccgcaatttgaggattctgatcacc-3').. Sequencing reactions included primers at 3 2 pmol, approximately 200 ng DNA template, and 8 □l sequencing mix. Sequencing reactions were performed using the Big Dye Terminator Ready Sequencing Mix (PE-Applied Biosystems, Foster City, CA) according to the manufacturer's instructions. Samples were subsequently purified using Centrisep columns (Princeton Separations, Adelphia, NJ). The eluates were dried in a Savant speed-vacuum dryer, denatured in 20 □l template Suppression Reagent (ABI) at 95°C for 2 minutes, and analyzed on an ABI 310 Genetic Analyzer (PE-Applied Biosystems), The sequence was edited, translated, and analyzed using Vector Nti version 6.0 (Informax, North Bethesda, MD) The 2H7scFv-CD154 L2 cDNA sequence and predicted amino acid sequence is presented in Figure 7A, and 2H7scFv-CD154 S4 cDNA sequence and predicted amino acid sequence is presented in Figure 7B.

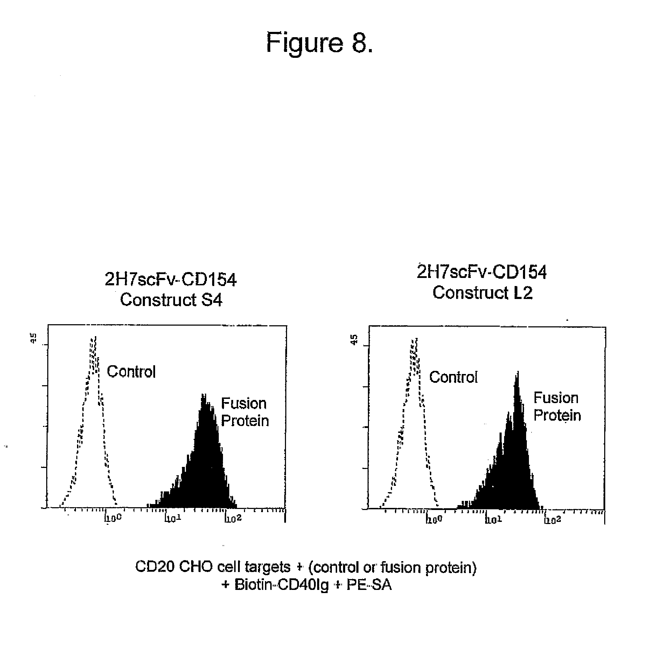

The binding activity of the 2H7 scFv-CD154 fusion proteins (SEQ. ID NO:_ and _) to CD20 and CD40 simultaneously was determined by flow cytometry. The assay used CHO cell targets that express CD20. After a 45-minute incubation of CD20 CHO cells with supernatants from cells transfected with the 2H7 scFv-CD154 expression plasmid, the CD20 CHO cells were washed twice and incubated with biotin-conjugated CD40-Ig fusion protein in PBS/2% FBS. After 45 min, cells were washed twice and incubated with phycoerythrin (PE)-labeled strepavidin at 1:100 in PBS/2% FBS (Molecular Probes, Eugene OR). After an additional 30 min incubation, cells were washed 2X and were analyzed by flow cytometry. The results show that the 2H7 scFv-CD154 molecule was able to bind to CD20 on the cell surface and to capture biotin-conjugated CD40 from solution (Figure 8)

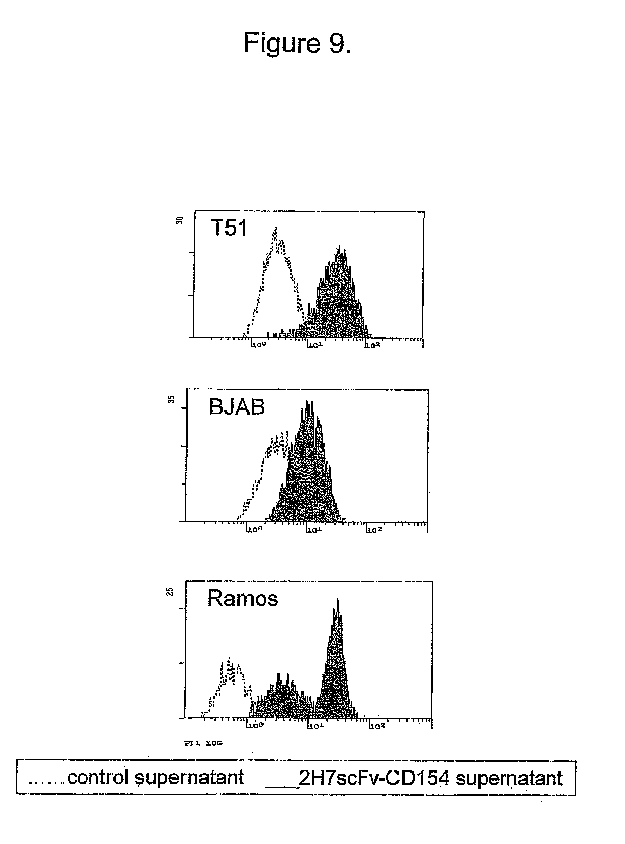

To determine the effect of the 2H7scFv-CD154 on growth and viability of B lymphoma and lymphoblastoid cell lines, cells were incubated with 2H7scFv-CD154 L2 (SEQ. ID NO: _) for 12 hours and then examined for binding of Annexin V. Binding of Annexin V was measured using a FITC-Annexin. V kit (Immunotech, Marseille, France, Catalog # PN-IM2376). B cell lines were incubated in 1 ml cultures with dilutions of concentrated, dialyzed supernatants from cells expressing secreted forms of the 2H7scFv-CD154 fusion proteins. The results are presented in Figure 9

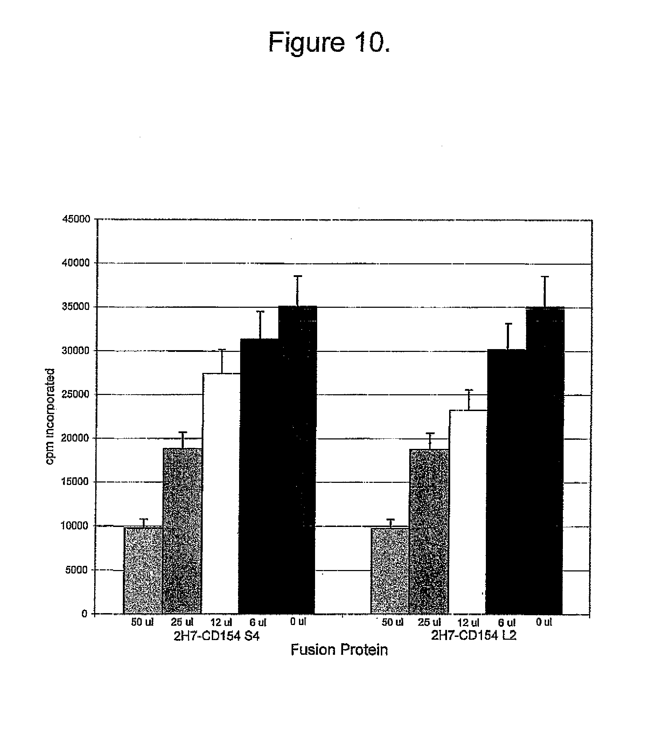

The growth rate of the Ramos B lymphoma cell line in the presence of 2H7scFv-CD154 was examined by uptake of ³H-thymidine for the last 6 hours of a 24-hour culture. The effect of 2H7scFv-CD154 on cell proliferation is shown in Figure 10.

### EXAMPLE 5

### CONSTRUCTION AND CHARACTERIZATION OF CYTOXB ANTIBODY DERIVATIVES

CytoxB antibodies were derived from the 2H7 scFv-IgG polypeptide. The 2H7 scFv (see Example 1) was linked to the human IgG4 Fc domain via an altered hinge domain (see Figure 11). Cysteine residues in the hinge region were substituted with serine residues by site-directed mutagenesis and other methods known in the art. The mutant hinge was fused either to a wild-type Fc domain to create one construct, designated CytoB-MHWTG1C, or was fused to a mutated Fc domain (CytoxB-MHMG1C) that had additional mutations introduced into the CH2 domain. Amino acid residues in CH2 that are implicated in effector function are illustrated in Figure 11 Mutations of' one or more of these residues may reduce FcR binding and mediation of' effector functions. In this example, the leucine residue 234 known in the art to be important to Fc receptor binding, was mutated in the 2H7 scFv fusion protein, CytoxB-[MG1H/MG1C]. In another construct, the human IgG1 hinge region was substituted with a portion of the human IgA hinge, which was fused to wild-type human Fc domain (CytoxB-IgAHW7HG1C). (See Figure 11). This mutated hinge region allows expression of' a mixture of monomeric and dimeric molecules that retain functional properties of the human IgG1 CH2 and CH3 domains Synthetic, recombinant cDNA expression cassettes for these molecules were constructed and polypeptides were expressed in CHODC44 cells according to methods described in Example 2.

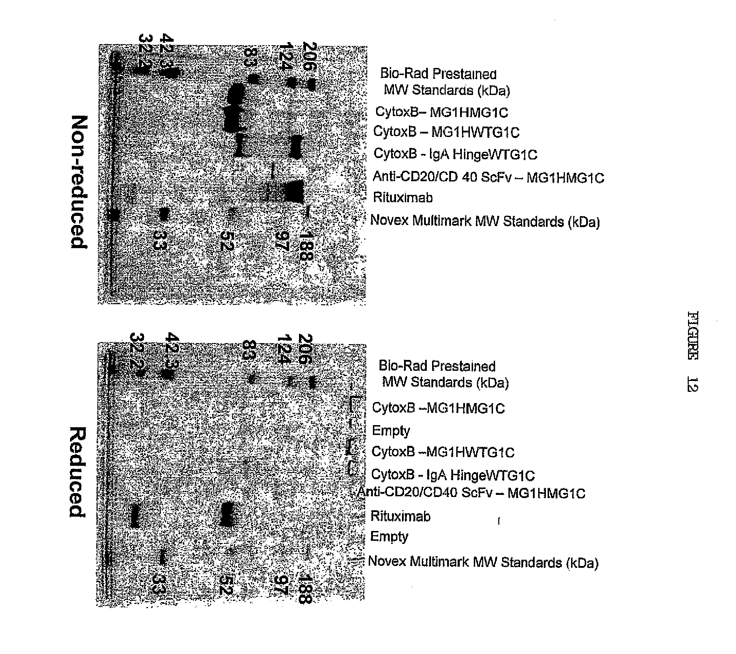

Purified fusion protein derivatives of CytoxB-scFvIg molecules were analyzed by SDS-PAGE according to the methods described in Example 2 Polyacrylamide gels were run under non-reducing and reducing conditions. Two different molecule weight marker sets, BioRad prestained markers, (BioRad, Hercules, CA) and Novex Multimark molecular weight markers were loaded onto each gel. The migration patterns of the different constructs and of Rituximab™ are presented in Figure 12.

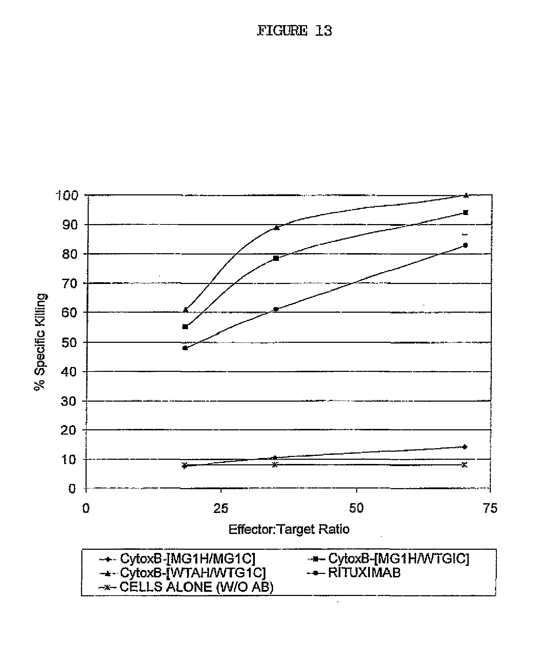

The ability of the different derivatives of CytoxB-scFvlg molecules to mediated ADCC was measured using the Bjab B lymphoma cells as the target and freshly prepared human PBMCs as effector cells. (See Example 2). Effector to target ratios were varied as follows: 70:1, 35:1, and 18:1, with the number of Bjab cells per well remaining constant but the number of PBMCs were varied. Bjab cells were labeled for 2 hours with ⁵¹Cr and aliquoted at a cell density of 5 x 10⁴ cells/well to each well of flat-bottom 96 well plates. Purified fusion proteins or rituximab were added at a concentration of 10 mg/ml to the various dilutions of PBMCs, Spontaneous release was measured without addition of PBMC or fusion protein, and maximal release was measured by the addition of detergent (1% NP-40) to the appropriate wells. Reactions were incubated for 4 hours, and 100 ul of culture supernatant was harvested to a Lumaplate (Packard Instruments) and allowed to dry overnight prior to counting cpm released. The results are presented in Figure 13.

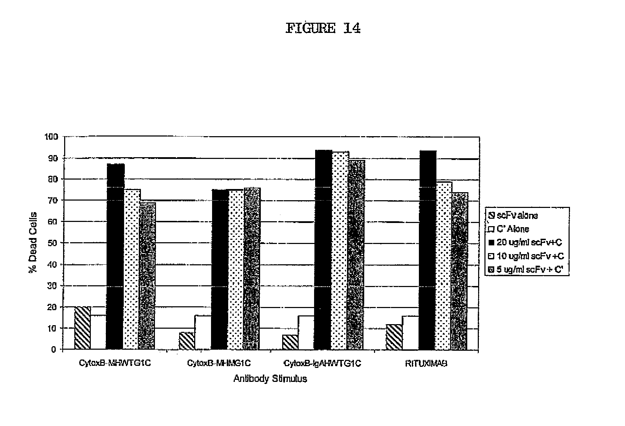

Complement dependent cytotoxicity (CDC) activity of the CytoxB derivatives was also measured. Reactions were performed essentially as described in Example 2. The results are presented in Figure 14 as percent of dead cells to total cells for each concentration of fusion protein.

### EXAMPLE 6

### IN VIVO STUDIES IN MACAQUES

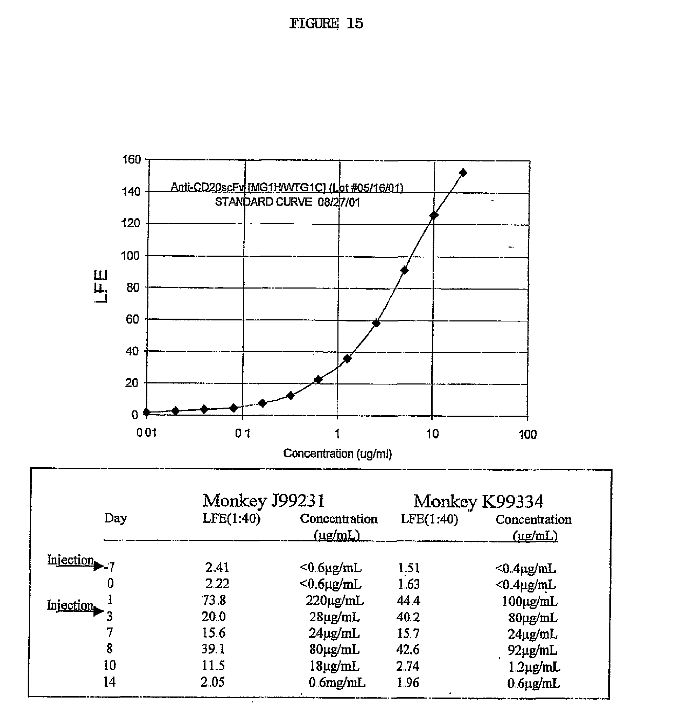

Initial *in vivo* studies with CytoxB derivatives have been performed in nonhuman primates Figure 15 shows data characterizing the serum half-life of CytoxB in monkeys. Measurements were performed on serum samples obtained from two different macaques (199231 and K99334) after doses of 6 mg/kg were administered to each monkey on the days indicated by arrows. For each sample, the level of 2H7scFvIg present was estimated by comparison to a standard curve generated by binding of' purified GytoxB-(MHWTG1C)-Ig fusion protein to CD20 CHO cells (see Example 2). The data are tabulated in the bottom panel of the Figure 15.

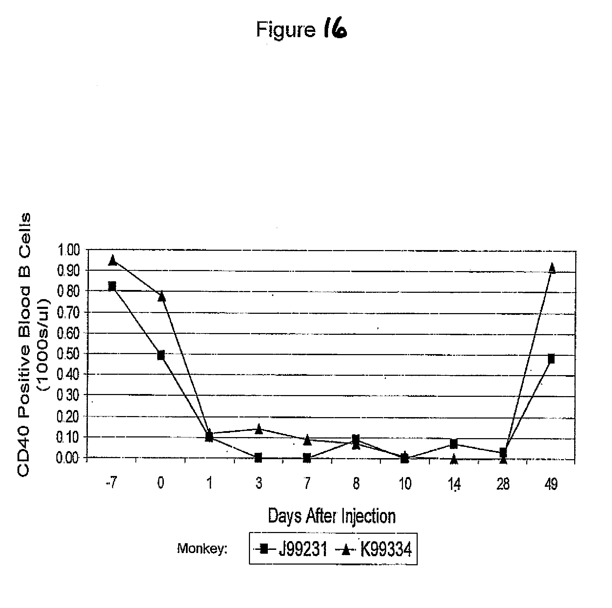

The effect of CytoxB-(MHWTG1)Ig fusion protein on levels of circulating CD40+ cells in macaques was investigated Complete blood counts were performed at each of the days indicated in Figure 16. In addition, FACS (fluorescence activated cell sorter) assays were performed on peripheral blood lymphocytes using a CD40-specific fluorescein conjugated antibody to detect B cells among the cell population. The percentage of positive cells was then used to calculate the number of B cells in the original samples. The data are graphed as thousands of B cells per microliter of blood measured at the days indicated after injection (Figure 16).

### EXAMPLE 7

### CONSTRUCTION AND EXPRESSION OF AN ANTI-CD19 SCFv-IG FUSION PROTEIN

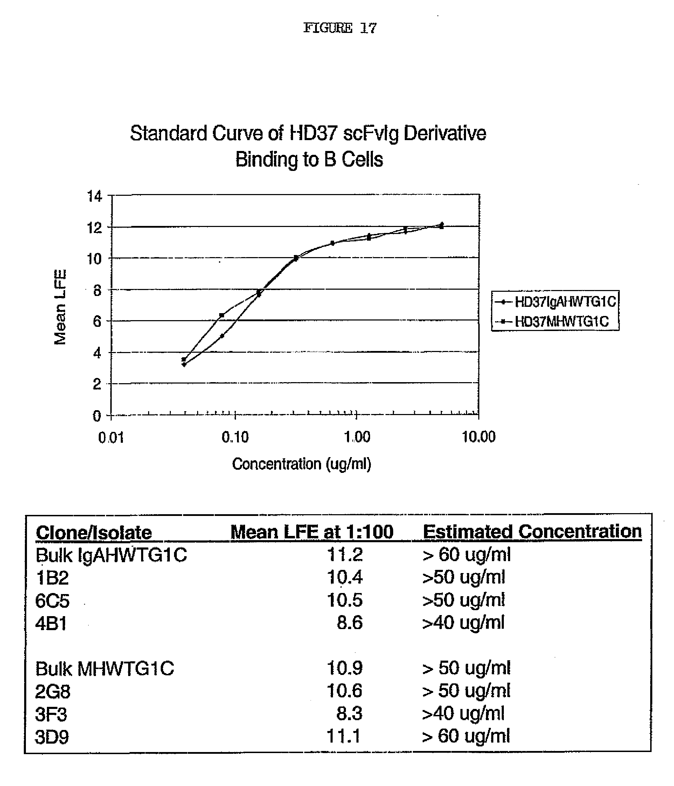

An anti-CD19 scFv-Ig fusion protein was constructed, transfected into eukaryotic cells, and expressed according to methods presented in Examples 1, 2, and 5 and standard in the art The variable heavy chain regions and variable light chain regions were cloned from RNA isolated from hybridoma cells producing antibody HD37, which specifically binds to CD19, Expression levels of' a HD37scFv-IgAHWTG1C and a HD37scFv-IgMHWTG1C were measured and compared to a standard curve generated using purified HD37 scFvIg. The results are presented in Figure 17.

### EXAMPLES 8

### CONSTRUCTION AND EXPRESSION OF AN ANTI-L6 SCFV-IG FUSION PROTEIN

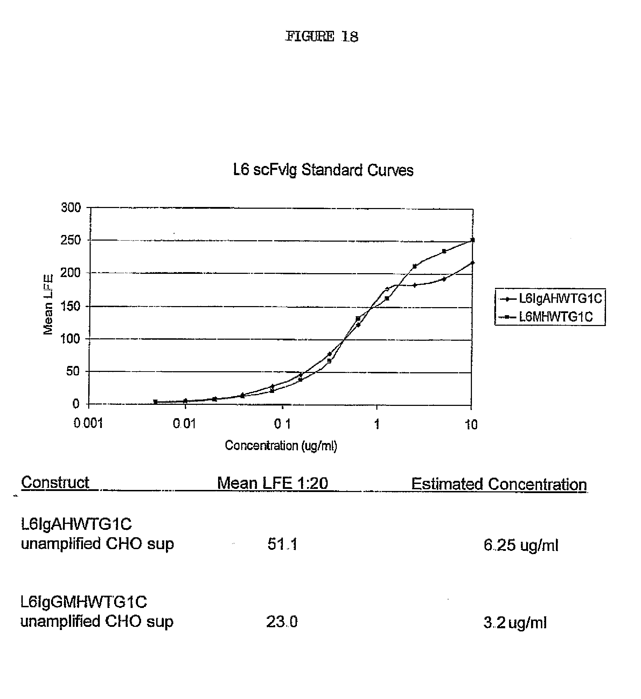

An scFv-Ig fusion protein was constructed using variable regions derived from an anti-carcinoma mAb, L6. The fusion protein was constructed, transfected into eukaryotic cells, and expressed according to methods presented in Examples 1, 2, and 5 and standard in the art. Expression levels of L6scFv-IgAHWTG1C and L6scFv-IgMHWTG1C were measured and compared to a standard curve generated using purified HD37 scFvIg. The results are presented in Figure 18.

### EXAMPLE 9

### CHARACTERIZATION OF VARIOUS scFv-IG FUSION PROTEINS

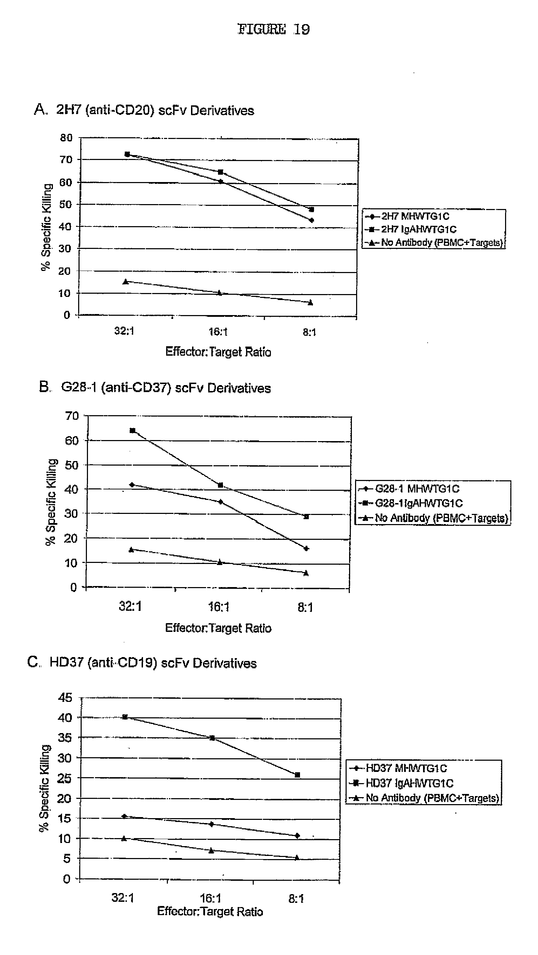

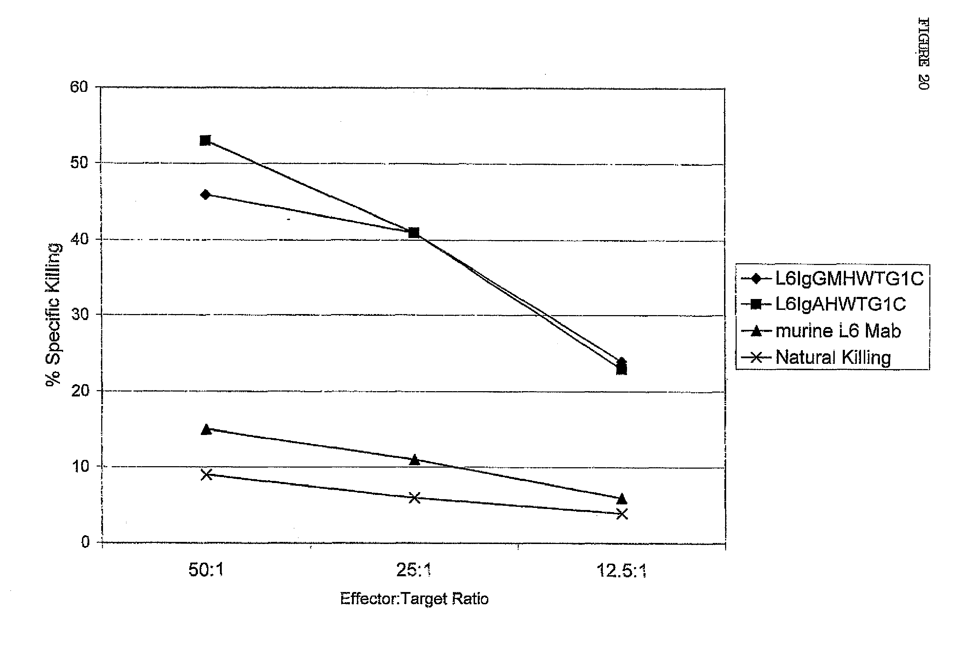

In addition to the scFv-Ig fusion protein already described, G28-1 (anti-CD37) scFv-Ig fusion proteins were prepared essentially as described in Examples 1 and 5. The variable regions of the heavy and light chains were cloned according to methods known in the art. ADCC activity of 2H7-MHWTG1C, 2H7-IgAHWTG1C, G28-1- MHWTG1C, G28-1 IgAHWTG1C, HD37- MHWTG1C, and HD37-IgAHWTG1C was determined according to methods described above (see Example 2). Results are presented in Figure 19. ADCC activity of L6scFv-IgAHWTG1C and L6scFv-IgMHWIG1C was measured using the 2981 human lung carcinoma cell line. The results are presented in Figure 20. The murine L6 monoclonal antibody is known not to exhibit ADCC activity.

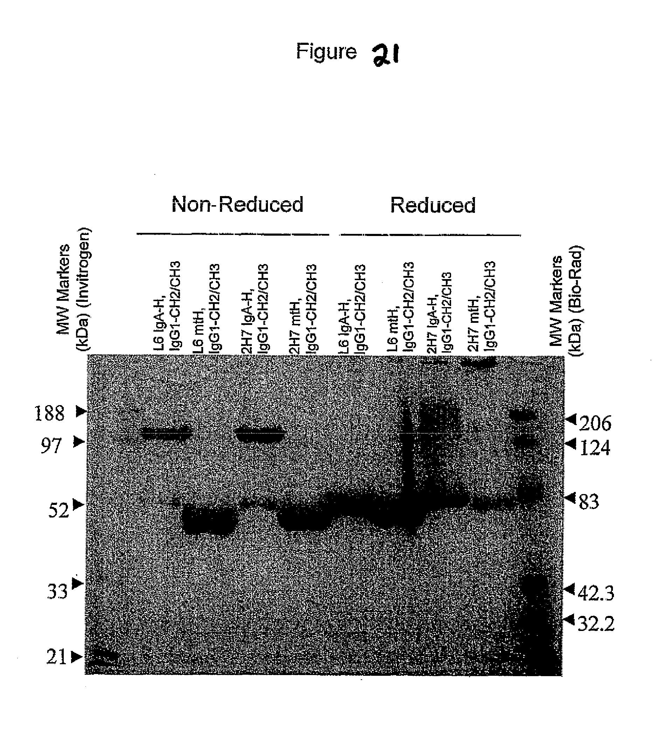

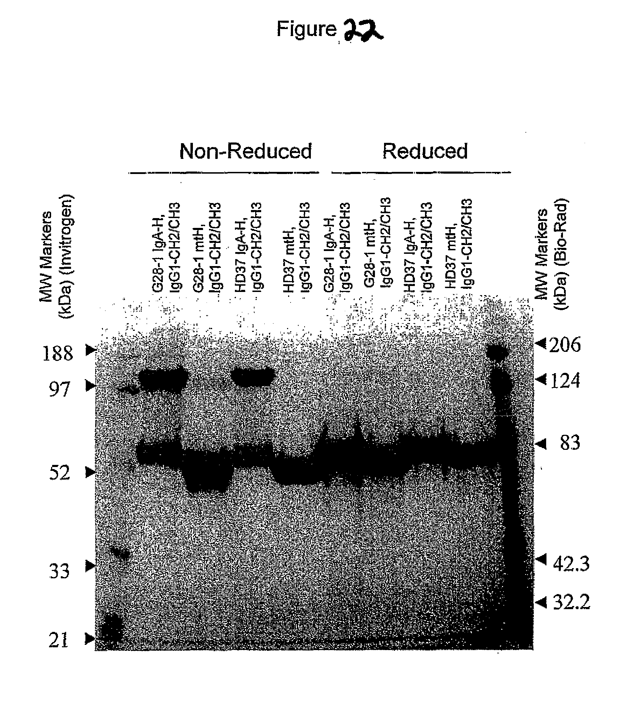

The purified proteins were analyzed by SDS-PAGE under reducing and non-reducing conditions. Samples were prepared and gels run essentially as described in Examples 2 and 5. The results for the L6 and 2H7 scFv-Ig fusion proteins are presented in Figure 21 and the results for the G28-1 and HD37 scFv-Ig fusion proteins are presented in Figure 22.

From the foregoing, it will be appreciated that, although specific embodiments of the invention have been described herein for the purpose of illustration, various modifications may be made without deviating from the spirit and scope of the invention. Accordingly, the present invention is not limited except as by the appended claims.

The present invention is now described with reference to the following clauses:
1. A binding domain-immunoglobulin fusion protein, comprising:
   (a) a binding domain polypeptide that is fused to an immunoglobulin hinge region polypeptide, wherein said hinge region polypeptide is selected from the group consisting of' (i) a mutated hinge region polypeptide that contains no cysteine residues and that is derived from a wild-type immunoglobulin hinge region polypeptide having one or more cysteine residues, (ii) a mutated hinge region polypeptide that contains one cysteine residue and that is derived from a wild-type immunoglobulin hinge region polypeptide having two or more cysteine residues, (iii) a wild-type human IgA hinge region polypeptide, (iv) a mutated human IgA hinge region polypeptide that contains no cysteine residues and that is derived from a wild-type human IgA region polypeptide, and (v) a mutated human IgA hinge region polypeptide that contains one cysteine residue and that is derived from a wild type human IgA region polypeptide;
   (b) an immunoglobulin heavy chain CH2 constant region polypeptide that is fused to the hinge region polypeptide; and
   (c) an immunoglobulin heavy chain CH3 constant region polypeptide that is fused to the CH2 constant region polypeptide,
   wherein:
   (1) the binding domain-immunoglobulin fusion protein is capable of at least one immunological activity selected from the group consisting of antibody dependent cell-mediated cytotoxicity and complement fixation, and
   (2) the binding domain polypeptide is capable of specifically binding to an antigen.
2. The binding domain-immunoglobulin fusion protein of clause 1 wherein the immunoglobulin hinge region polypeptide is a mutated hinge region polypeptide and exhibits a reduced ability to dimerize, relative to a wild-type human immunoglobulin G hinge region polypeptide.
3. The binding domain-immunoglobulin fusion protein of clause 1 wherein the binding domain polypeptide comprise at least one immunoglobulin variable region polypeptide that is selected from the group consisting of an immunoglobulin light chain variable region polypeptide and an immunoglobulin heavy chain variable region polypeptide.
4. The binding domain -immunoglobulin fusion protein of clause 3 wherein the immunoglobulin variable region polypeptide is derived from a human immunoglobulin
5. The binding domain Fv-immunoglobulin fusion protein of cause 1 wherein the binding domain polypeptide comprises:
   (a) at least one immunoglobulin light chain variable region polypeptide;
   (b) at least one immunoglobulin heavy chain variable region polypeptide; and
   (c) at least one linker peptide that is fused to the polypeptide of (a) and to the polypeptide of (b).
6. The binding domain-immunoglobulin fusion protein of clause 5 wherein the immunoglobulin light chain variable region and heavy chain variable region polypeptides are derived from human immunoglobulins.
7. The binding domain-inmiunoglobulin fusion protein of clause 1 wherein at least one of the immunoglobulin heavy chain CH2 constant region polypeptide and the immunoglobulin heavy chain CH3 constant region polypeptide is derived from a human immunoglobulin heavy chain.
8. The binding domain-immunoglobulin fusion protein of clause 1 wherein the immunoglobulin heavy chain constant region CH2 and CH3 polypeptides are of an isotype selected from the group consisting of human IgG and human IgA.
9. The binding domain-immunoglobulin fusion protein of clause 1 wherein the antigen is selected from the group consisting of CD19, CD20, CD37, CD40 and L6.
10. The binding domain-immunoglobulin fusion protein of clause 5 wherein the linker polypeptide comprises at least one polypeptide having as an amino acid sequence Gly-Gly-Gly-Gly-Ser [SEQ ID NO:21].
11. The binding domain-immunoglobulin fusion protein of clause 5 wherein the linker polypeptide comprises at least three repeats of a polypeptide having as an amino acid sequence Gly-Gly-Gly-Gly-Ser [SEQ ID NO:21].
12. The binding domain-immunoglobulin fusion protein of clause 1 wherein the immunoglobulin hinge region polypeptide comprises a human IgA hinge region polypeptide.
13. The binding domain-immunoglobulin fusion protein of clause 1 wherein the binding domain polypeptide comprises a CD154 extracellular domain.
14. The binding domain-immunoglobulin fusion protein of clause 1 wherein the binding domain polypeptide comprises a CD154 extracellular domain and at least one immunoglobulin variable region polypeptide.
15. An isolated polynucleotide encoding a binding domain-immunoglobulin fusion protein, said protein comprising:
   (a) a binding domain polypeptide that is fused to an immunoglobulin hinge region polypeptide, wherein said hinge region polypeptide is selected from the group consisting of (i) a mutated hinge region polypeptide that contains no cysteine residues and that is derived from a wild-type immunoglobulin hinge region polypeptide having one or more cysteine residues, (ii) a mutated hinge region polypeptide that contains one cysteine residue and that is derived from a wild-type immunoglobulin hinge region polypeptide having two or more cysteine residues, (iii) a wild-type human IgA hinge region polypeptide, (iv) a mutated human IgA hinge region polypeptide that contains no cysteine residues and that is derived from a wild-type human IgA region polypeptide, and (v) a mutated human IgA hinge region polypeptide that contains one cysteine residue and that is derived from a wild-type human IgA region polypeptide;
   (b) an immunoglobulin heavy chain CH2 constant region polypeptide that is fused to the hinge region polypeptide; and
   (c) an immunoglobulin heavy chain CH3 constant region polypeptide that is fused to the CH2 constant region polypeptide,
   wherein:
   (1) the binding domain-immunoglobulin fusion protein is capable of at least one immunological activity selected from the group consisting of' antibody dependent cell-mediated cytotoxicity and complement fixation, and
   (2) the binding domain polypeptide is capable of specifically binding to an antigen.
16. A recombinant expression construct comprising a polynucleotide according to clause 15 that is operably linked to a promoter.
17. A host cell transformed or transfected with a recombinant expression construct according to clause 16.
18. A method of producing a binding domain-immunoglobulin fusion protein, comprising the steps of:
   (a) culturing a host cell according to clause 17 under conditions that permit expression of the binding domain-immunoglobulin fusion protein; and
   (b) isolating the binding domain-immunoglobulin fusion protein from the host cell culture
19. A pharmaceutical composition comprising a binding domain-immunoglobulin fusion protein according to clause 1 in combination with a physiologically acceptable carrier.
20. A method of treating a subject having or suspected of having a malignant condition or a B-cell disorder, comprising administering to a patient a therapeutically effective amount of a binding domain-immunoglobulin fusion protein according to clause 1.
21. The method of clause 20 wherein the malignant condition or B-cell disorder is selected from the group consisting of a B-cell lymphoma and a disease characterized by autoantibody production.
22. The method of clause 20 wherein the malignant condition or B-cell disorder is selected from the group consisting of rheumatoid arthritis, myasthenia gravis. Grave's disease, type I diabetes mellitus, multiple sclerosis and an autoimmune disease

## Claims

1. A single chain protein, comprising:
(a) a binding domain polypeptide that binds to a target biological molecule, the binding domain polypeptide being joined to
(b) an immunoglobulin hinge peptide, the hinge peptide being joined to
(c) an immunoglobulin heavy chain CH2 constant region polypeptide, the CH2 constant region polypeptide being joined to
(d) an immunoglobulin heavy chain CH3 constant region polypeptide,
wherein the hinge peptide is an IgG or IgA hinge peptide in which the number of cysteine residues is one, or the number of cysteine residues is two and the first cysteine of the hinge that is responsible for forming a disulfide bond with a light chain constant region in a naturally occurring IgG antibody is not deleted or substituted, and
wherein the single chain protein (i) binds to the target biological molecule, and (ii) promotes antibody dependent cell-mediated cytotoxicity or complement fixation or both.

2. The single chain protein of claim 1, wherein the binding domain polypeptide is a single chain Fv polypeptide.

3. The single chain protein of claim 3, wherein the polypeptide linker comprises an amino acid sequence of Gly-Gly-Gly-Gly-Ser (SEQ ID NO: 21) or at least three repeats of amino acid sequence Gly-Gly-Gly-Gly-Ser (SEQ ID NO: 21).

4. A single chain protein of any one of the preceding claims, wherein the target is selected from CD20, CD37, CD19, CD22, CD30 ligand, CD54, CD106, interleukin-12,CD2, CD5, CD10, CD27, CD28, CD40, CTLA-4, 4-1BB, 4-1BB ligand, interferon-γ, interleukin-4, interleukin-17, interleukin-17 receptor, CD59, CD48, CD72, CD70, CD86/B7.2, CD40 ligand, CD43, VLA-4 (α₄β₇), CD83, DEC-205, HER1, HER2, HER3, HER4, epidermal growth factor receptor, vascular endothelial growth factor, vascular endothelial growth factor receptor, insulin-like growth factor-I, insulin-like growth factor-II, transferrin receptor, estrogen receptor, progesterone receptor, follicle stimulating hormone receptor, retinoic acid receptor, MUC-1, NY-ESO-1, NA 17-A, Melan-A/MART-1, tyrosinase, Gp-100, MAGE, BAGE, GAGE, CTA class receptors, the HOM-MEL-40 antigen encoded by the SSX2 gene, carcinoembryonic antigen, or PyLT.

5. The single chain protein of any one of the preceding claims, wherein the immunoglobulin hinge region is selected from an IgG1, IgG2, IgG3, or IgG4 hinge region.

6. The single chain protein of any one of the preceding claims, wherein the immunoglobulin heavy chain CH2 and CH3 constant region polypeptides are human CH2 and CH3 constant region polypeptides or human IgG1 CH2 and CH3 constant region polypeptides.

7. The single chain protein of any one of the preceding claims, wherein the heavy chain constant region comprises a human CH2 constant region polypeptide in which a leucine has been replaced with a serine at position 234.

8. A composition comprising a single chain protein of any one of the preceding claims and a physiologically acceptable carrier.

9. An isolated polynucleotide encoding a single chain protein of any one of the preceding claims.

10. A vector comprising a polynucleotide of claim 9.

11. A host cell comprising a vector of claim 10.

12. A single chain protein or composition of any one of the preceding claims for use in the treatment of a malignant condition or an immune system disorder.

13. The single chain protein of claim 12, wherein the malignant condition or immune system disorder is chronic lymphocytic leukemia, non-Hodgkin's lymphoma, rheumatoid arthritis, myasthenia gravis, Grave's disease, type I diabetes mellitus, multiple sclerosis, or an autoimmune disease.
